# FASCICULE DE BREVET EUROPEEN

(11) **EP 1 406 920 B1**
(45) Date de publication et mention de la délivrance du brevet: **15.11.2006**
(21) Numéro de dépôt: 02762539.1
(22) Date de dépôt: 05.07.2002
(51) Int. Cl.: C07K 5/06, G01N 33/68

(54) **NOUVEAUX SUBSTRATS CHROMOGENES ET LEUR UTILISATION POUR LE DOSAGE DE L'ACTIVITE DE CARBOXYPEPTIDASES**
NEUE CHROMOGENEN SUBSTRATE UND DEREN VERWENDUNG ZUM NACHWEIS DER WIRKUNG VON CARBOXYPEPTIDASEN
NOVEL CHROMOGENIC SUBSTANCES AND USE THEREOF FOR THE DETERMINATION OF CARBOXYPEPTIDASE ACTIVITIES

(30) Priorité: 06.07.2001 FR 0109030
(43) Date de publication de la demande: 14.04.2004
(73) Titulaire: Diagnostica Stago, 92600 Asnières (FR)
(72) Inventeur: QUENTIN, Gérard, F-28160 Yèvres (FR)
(74) Mandataire: Desaix, Anne
(86) Numéro de dépôt international: PCT/FR2002/002376
(87) Numéro de publication internationale: WO 2003/004516

(56) Documents cités:
- W L MOCK ET AL.: "Catalytic activity of carboxypeptidase B and of carboxypeptidase Y with anisylazoformyl substrates" BIOORGANIC & MEDICINAL CHEMISTRY LETTERS, vol. 9, 1999, pages 187-192, XP004152597 OXFORD, GB ISSN: 0960-894X cité dans la demande
- W L MOCK ET AL.: "Arazoformyl peptide surrogates as spectrophotometric kinetic assay substrates for carboxypeptidase A" ANALYTICAL BIOCHEMISTRY., vol. 239, 1996, pages 218-222, XP002196534 ACADEMIC PRESS INC. NEW YORK., US ISSN: 0003-2697 cité dans la demande
- W L MOCK & D J STANFORD: "Arazoformyl dipeptide substrates for thermolysin. Confirmation of a reverse protonation catalytic mechanism" BIOCHEMISTRY., vol. 35, no. 23, 1996, pages 7369-7377, XP002196535 AMERICAN CHEMICAL SOCIETY. EASTON, PA., US ISSN: 0006-2960 cité dans la demande
- K A SCHATTEMAN ET AL.: "Assay of procarboxypeptidase U, a novel determinant of fibrinolytic cascade, in human plasma" CLINICAL CHEMISTRY., vol. 45, no. 6, juin 1999 (1999-06), pages 807-813, XP002196536 AMERICAN ASSOCIATION FOR CLINICAL CHEMISTRY. WINSTON., US ISSN: 0009-9147

## Description

La présente invention est relative à des composés chromogènes et leur utilisation pour le dosage d'enzymes de la famille des carboxypeptidases U. Elle vise plus spécifiquement l'utilisation desdits composés pour le dosage de l'activité du TAFI (Thrombin Activatable Fibrinolysis Inhibitor) dans un échantillon sanguin et la méthode de dosage correspondante.

Les carboxypeptidases (CP) constituent un groupe d'enzymes au sein de la famille des exopeptidases. Ce sont des enzymes qui coupent les liaisons amide des chaînes polypeptidiques au niveau du dernier acide aminé COOH-terminal. Elles comprennent les sérine carboxypeptidases, les cystéine carboxypeptidases et les métallocarboxypeptidases.

De nombreuses carboxypeptidases ont été isolées et séquencées, chez les bactéries, les levures et les végétaux. Ces enzymes sont également présentes dans un grand nombre de tissus chez les mammifères.

Des carboxypeptidases ont notamment été isolées au niveau du pancréas et dans des mastocytes. D'autres carboxypeptidases circulantes dans le plasma ont également été clonées, isolées et séquencées.

Toutes ces enzymes ont un rôle IN VIVO dépendant à la fois de leur localisation et de leurs propriétés physiques. Ainsi les carboxypeptidases se classent différemment selon leur spécificité de substrat. Les carboxypeptidases A ont un spectre relativement large : elles hydrolysent la liaison peptidique C-terminale du dernier acide aminé préférentiellement s'il est hydrophobe (Phe, Leu, Val, Ala). Elles hydrolysent plus lentement les acides aminés dicarboxyliques (Asp, Glu) ainsi que la glycine (Gly), et n'hydrolysent pas du tout les acides aminés basiques tels que l'arginine (Arg), la lysine (Lys), l'histidine (His) ou l'ornithine (Orn), un homologue inférieur de la lysine, ni les acides aminés secondaires tels que la proline (Pro) ou l'hydroxyproline (Hyp).

Les carboxypeptidases B hydrolysent spécifiquement les derniers acides aminés basiques C-terminaux, tels que Arg, Lys. Cette classe d'enzymes se subdivise en sous-classes constituées par les carboxypeptidases H (également dénommée encéphaline convertase ou carboxypeptidase E), M, N et U.

La carboxypeptidase E a été localisée dans les granules sécrétoires des îlots pancréatiques, les glandes surrénales et pituitaires, et le cerveau. La carboxypeptidase M est une enzyme membranaire présente dans de nombreux tissus et cellules en culture.

La carboxypeptidase N (désignée parfois ci-après CPN) est une enzyme circulante dans le plasma. Elle protège l'organisme contre l'effet vasoactif et inflammatoire de peptides contenant un acide aminé basique C-terminal (de préférence une lysine), libérés dans la circulation. Cette enzyme existe sous sa forme active dans le sang. Elle est également dénommée kininase du fait de ses substrats naturels qui sont la bradykinine, les kinines, les anaphylatoxines.

Enfin, les carboxypeptidases U (unstable) (désignés parfois ci-après CPU) sont des enzymes qui hydrolysent également les acides aminés basiques C-terminaux, avec une préférence pour les arginines, mais qui, contrairement aux carboxypeptidases N, sont très instables lorsqu'elles se trouvent sous leur forme activée.

Une revue générale récente sur les enzymes de la famille des carboxypeptidases a été réalisée par BOUMA et al. (1).

Le TAFI est une métallocarboxypeptidase à zinc basique (1-4). C'est plus précisément une carboxypeptidase U, car son activité est instable à 37° C. Cette enzyme, dont l'ADNc et la séquence d'acides aminés correspondante chez l'homme sont par exemple décrits dans le brevet US 5,206,161, est une protéine plasmatique dont le rôle dans la régulation de la fibrinolyse a été initialement envisagé à partir de l'observation IN VITRO d'un retard de la lyse du caillot en présence de TAFI activé.

Le TAFI activé clive les résidus arginine et lysine exposés en position COOH-terminale sur la fibrine. Cette hydrolyse conduit à une diminution du nombre de sites de fixation du plasminogène et du tPA à la surface du caillot de fibrine, et donc une diminution de la transformation du plasminogène en plasmine par le tPA.

Le TAFI a été successivement identifié sous le nom de procarboxypeptidase B (pro-PCPB) puis de procarboxypeptidase instable (proCPU : Unstable ProCarboxypeptidase) et procarboxypeptidase R (CPR), du fait d'une activité carboxypeptidasique sur les résidus arginine. La forme zymogène est une glycoprotéine à chaîne unique de 60 KDa synthétisée dans le foie et circulante dans le plasma.

Le clivage du zymogène est réalisé majoritairement par la thrombine au niveau du site arg 92. Cette protéolyse libère un peptide N-terminal de 92 acides aminés contenant plusieurs sites de glycosylation. L'action catalytique de la thrombine sur le TAFI est considérablement augmentée par la thrombomoduline en présence d'ions divalents. La vitesse d'activation du TAFI catalysée par la thrombine est ainsi accrue plus de 1000 fois du fait de la formation d'un complexe ternaire avec la thrombomoduline.

Le TAFI activé (TAFIa) est constitué par le domaine catalytique C-terminal du zymogène et comporte 309 acides aminés.

Du fait de son rôle clef dans les mécanismes de régulation du processus de la fibrinolyse, il s'est rapidement avéré utile de pouvoir mesurer la quantité de TAFI activé constitutionnel et de TAFI activable présent dans le plasma dans différents états pathologiques.

Plusieurs méthodes de dosage de l'activité de diverses carboxypeptidases utilisant des substrats spécifiques ont été décrites dans l'état de l'art.

Ainsi, WOLFF et al. (5) ont comparé les performances des substrats synthétiques Hip-Arg, Hip-Lys, Hip-Om sur les carboxypeptidases B pancréatiques purifiées par chromatographie en mesurant les différences d'absorption en UV.

En 1970, S. SUZUKI et al. (6) ont amélioré la détection du produit d'hydrolyse en dérivatisant la benzoylglycine libérée par le réactif TT (2, 4, 6 Trichloro-5-Triazin). Cette dérivation opérait spécifiquement sur le CH₂ de la glycine en α d'une fonction acide libre uniquement. Ce dérivé devient jaune vif (longueur d'onde maximale à 382 nm).

Cette méthode de développement de coloration jaune sur les restes Hippuryl a largement été utilisée par de nombreux auteurs par la suite.

En 1972, K. LORENTZ et al. (7) ont utilisé une réaction de coloration de l'arginine libérée cette fois par action des carboxypeptidases B sur un substrat Hip-Arg en utilisant la p-benzoquinone comme réactif. De cette manière, le dérivé coloré à une longueur d'onde maximale à 480 nm.

En 1980, Th. H. Plummer et al. (8) ont utilisé un substrat synthétique : le Furylacryloyl-Ala-Lys pour quantifier les carboxypeptidases N, la lecture se faisant à 324 nm (proche UV mais non visible).

En 1985, G.H. Fischer et al. (9) ont utilisé un substrat fluorescent par dansylation N-terminale d'un peptide spécifique de la carboxypeptidase à doser. Ce dosage nécessitait une étape d'extraction des produits d'hydrolyse pour une bonne séparation des espèces avant lecture.

En 1986, H. SARUTA et al. ont déterminé par une méthode colorimétrique, l'activité de la carboxypeptidase A (10). Ceux-ci ont utilisé une enzyme spécifique : "l'Hippuricase" pour hydrolyser le dérivé hippuricyl lui-même obtenu par hydrolyse du substrat hydroxy-hippuricyl-Phg par la CPA. Cette réaction était révélée finalement par le 4-Aminoantipyrine pour donner un colorant quinoneimide absorbant à 505 nm.

En 1998, NAM JOO HONG et al. ont utilisé un substrat synthétique avec un analogue de l'arginine, le thiaarginine pour doser les CPB (11).
Toutefois, les méthodes colorimétriques décrites ci-dessus soit n'ont jamais été utilisées pour doser spécifiquement l'activité enzymatique de CPN ou CPU, notamment le TAFI, soit présentent certains inconvénients incompatibles avec un test de dosage simple et efficace de ce type d'enzyme par colorimétrie.

En effet, selon les cas considérés :
- soit la réaction de dérivatisation aboutissant à un produit coloré n'est pas spécifique des espèces libérées lors de l'hydrolyse,
- soit les réactifs de dérivatisation sont très toxiques et ne peuvent pas être utilisés à l'échelle industrielle,
- soit la méthode de révélation colorée est trop longue et contraignante et ne peut être automatisée aisément.

En 1996, W.L. Mock et al. ont décrit une méthode de dosage d'une endoprotéase à zinc extracellulaire d'origine bactérienne, la thermolysine, à l'aide de substrats synthétiques se décolorant sous l'action de l'hydrolyse enzymatique (12). Pour ce faire, les auteurs ont synthétisé des composés dipeptidiques constitués d'un résidu leucine portant un groupement N-(4-methoxyphenylazoformyl) greffé au niveau de la fonction amine, et d'un autre acide aminé neutre (Leu, Ala, Phe ou Gly).

L'incorporation du groupement arazoformyl donne lieu à un composé fortement coloré. En présence de la thermolysine, la molécule de colorant se réarrange pendant la réaction chimique d'hydrolyse selon un mécanisme bien décrit dans (12 - 13), donnant naissance à des produits non colorés (fragment anizole, N₂, CO₂ et acide aminé). Il est donc aisé de suivre la vélocité de l'enzyme par la vitesse de décoloration du milieu.

Les mêmes auteurs ont également décrit une méthode cinétique de dosage par spectrophotométrie basée sur le même principe que la précédente pour la carboxypeptidase A (13) et la carboxypeptidase B porcine pancréatique (14).

Dans ce cas, les composés utilisés étaient constitués d'un seul acide aminé connu pour être clivé par la carboxypeptidase A (Phé, Leu) ou la carboxypeptidase B (Lys), portant un groupement N-arazoformyl. Le clivage enzymatique de ces composés en produits non colorés, était suivi par mesure de la décroissance de coloration du milieu.

Partant de ces enseignements, les auteurs de la présente invention ont synthétisé de nouveaux composés colorés pour doser par méthode colorimétrique l'activité de carboxypeptidases U, et plus particulièrement, l'activité du TAFI.

Selon un premier aspect, la présente invention a donc pour objet de nouveaux composés, constituant des substrats de carboxypeptidase U, et plus particulièrement des substrats du TAFI activé. Ces composés sont des substrats chromogènes.

Les composés de l'invention sont caractérisés en ce qu'il s'agit des composés arazoformyl de formule générale (I) suivante : 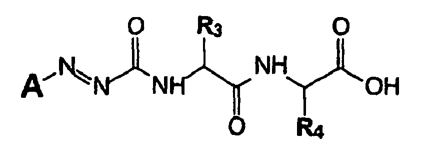 dans laquelle : 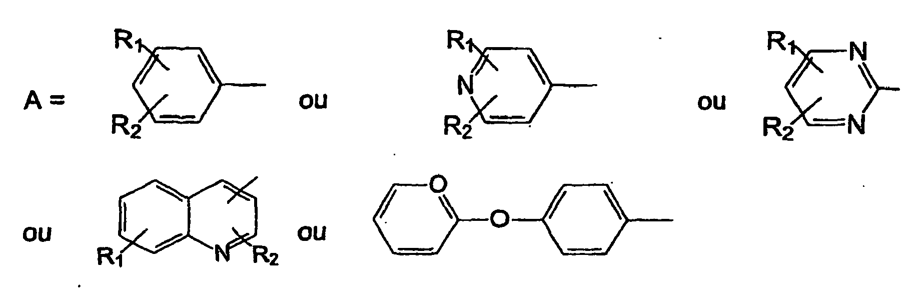
- R1, R2 = H, -CH₃, -CH(CH₃)_{2,} -OCH₃, -Cl, -CF₃, -OCF₃, -SCH₃
- R₃ = la chaîne latérale d'un acide aminé hydrolysable (ou un radical d'acide aminé hydrolysable) par une carboxypeptidase A.
- R₄ = un radical d'acide aminé basique.

Plus particulièrement, R₃ est choisi parmi les radicaux d'acides aminé: hydrophobes tels que :
- la tyrosine : 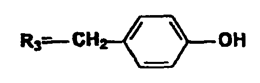
- la phenylalanine : 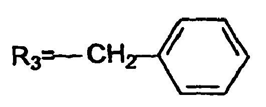
- l'alanine : R₃ = -CH₃
- la valine : R₃ = -CH-(CH₃)₂
- la leucine : R₃ = -CH₂-CH-(CH₃)₂
- l'isoleucine : 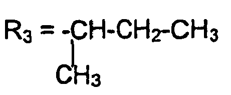
- la phenylglycine 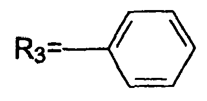
R4 est plus précisément choisi parmi les radicaux d'acides aminés tels que :
- l'arginine 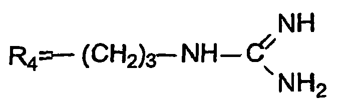
- la lysine (R4 = -(CH₂)₄-NH₂)
- l'omithine R₄=-(CH₂)₃-NH₂

De préférence, R₃ représente un reste acide aminé aromatique et en particulier la phenylalanine ou la tyrosine, et R4 l'arginine ou la lysine.

R₁ est de préférence choisi parmi :
- H et -CH₃, et,
R₂ de préférence choisi parmi :
- CH₃, -O-CH₃ et -S-CH₃

Une famille préférée de composés selon l'invention est représentée par la formule générale : 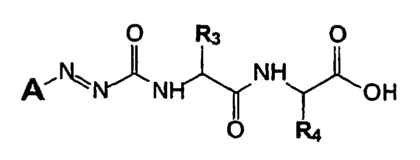 dans laquelle : 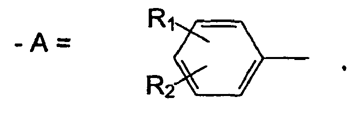 et.
- R₁, R₂ = -H, -CH₃, (CH₃)₂ CH-, CH₃-O-, Cl-, -CF₃
- R₃ = un radical d'acide aminé hydrolysable par une carboxypeptidase A.
- R₄ = un radical d'acide aminé basique.
R₃ représente préférentiellement la phenylalanine, ou la tyrosine et,
R₄ représente préférentiellement l'arginine ou la lysine.
R₁ est de préférence choisi parmi :
- H et -CH₃, et,
R₂ de préférence choisi parmi :
- CH₃, -O-CH₃ et -S-CH₃

Un composé particulièrement préféré de la famille de la présente invention est un composé de formule (I) dans laquelle : 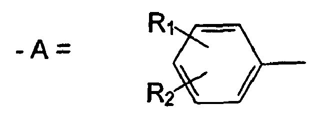 ledit composé étant choisi au sein du groupe constitué par les composés suivants, dans lesquels : 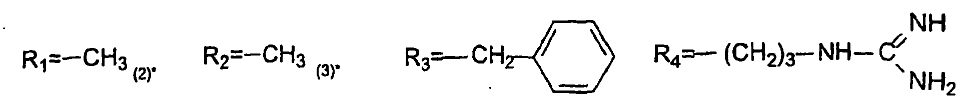 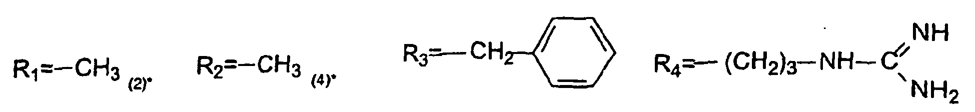 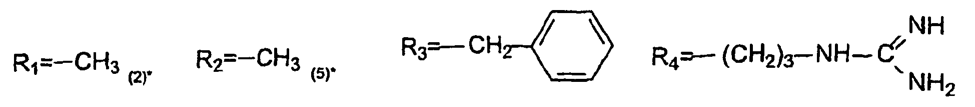 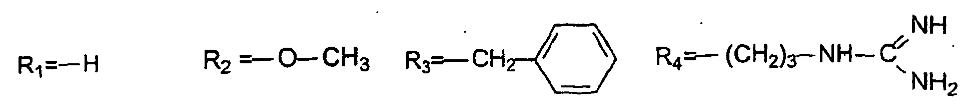 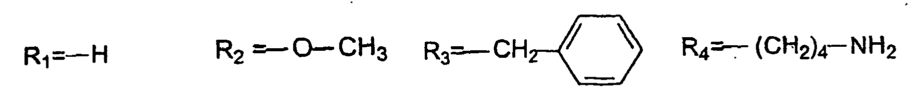 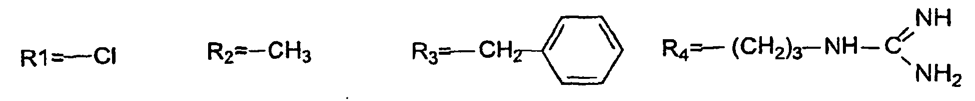 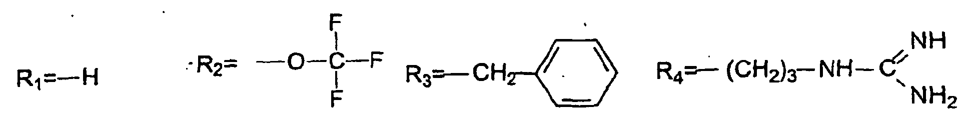 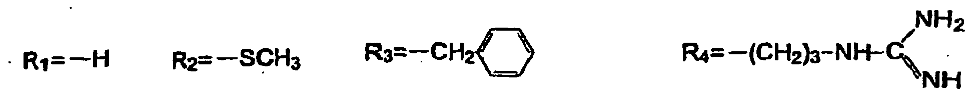 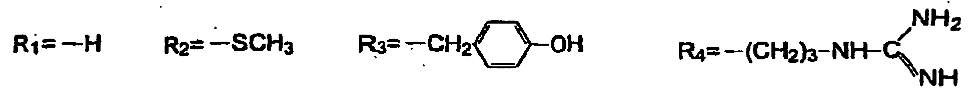 * les chiffres entre parenthèses déterminant la position des groupements methyl sur le radical phényl.
L'invention concerne en particulier, dans le cadre des définitions données ci-dessus, chaque composé répondant à la formule (I) et résultant des combinaisons possibles des groupements A, R1, R2, R3 et R4 dont il a été donné une définition préférée ci-dessus.

Dans ce qui suit, les composés ainsi définis seront indifféremment dénommés "composés de formule (I)" ou "substrats de formule (I)".

Selon un deuxième aspect, la présente invention a pour objet une méthode de dosage colorimétrique de l'activité d'une carboxypeptidase CPU, dans un échantillon biologique dans laquelle:
- on met en contact ledit échantillon avec un composé chromogène de formule (I) tel que défini ci-dessus, et une enzyme de la famille des carboxypeptidases A, dans des conditions permettant l'hydrolyse de l'échantillon et,
- on détermine l'hydrolyse dudit composé de formule (I) par la CPU de l'échantillon par la mesure de la décroissance de sa coloration (correspondant à une diminution de l'absorption) à une longueur d'onde sélectionnée à partir du spectre d'absorption dudit composé.

La diminution de la coloration résulte de la double hydrolyse du substrat de formule (I) par les carboxypeptidases U de l'échantillon d'une part, par la carboxypeptidase A d'autre part.

La méthode selon la présente invention est de préférence réalisée à l'aide d'un composé de formule (I) dans lequel l'acide aminé hydrophobe est ta phénylalanine ou de préférence la tyrosine, et l'acide aminé basique est l'arginine ou la lysine.

La méthode de l'invention est préférentiellement réalisée à l'aide d'un composé de formule (I) dans laquelle R₁ est choisi parmi -H et -CH₃ et R₂ parmi -CH₃, -O-CH₃ et -S-CH₃. Avantageusement, lorsque dans le composé de formule (I), R₁ est H, R₂ est S-CH₃.

La méthode de la présente invention est avantageusement réalisée avec la famille de composés de formule (I) dans laquelle :

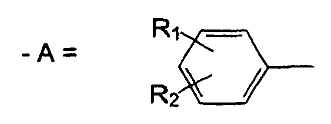

R₁ et R₂ ayant l'une quelconque des significations donnée ci-dessus et R2 étant de préférence -S-CH₃.
Plus particulièrement le composé de formule (I) utilisé est un composé phenylazoformyl dans lequel : 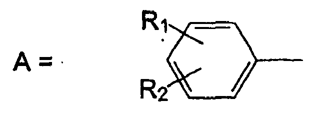 ledit composé étant choisi au sein du groupe constitué par les composés suivants, dans lesquels :

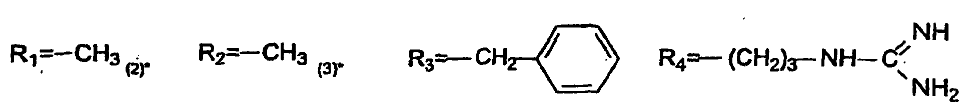

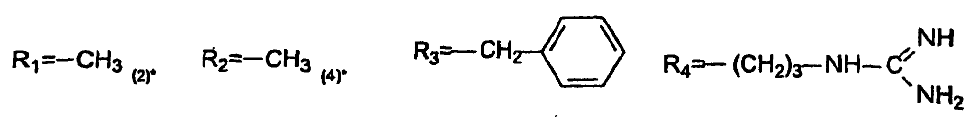

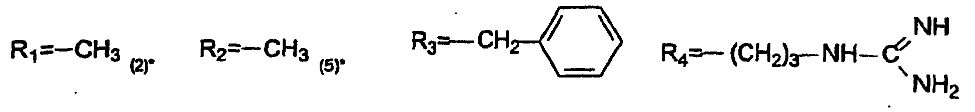

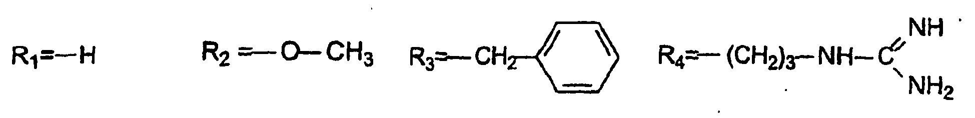

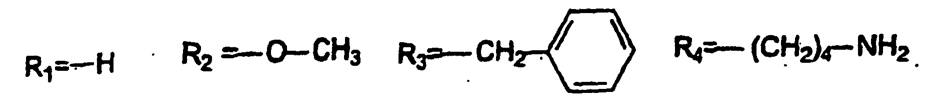

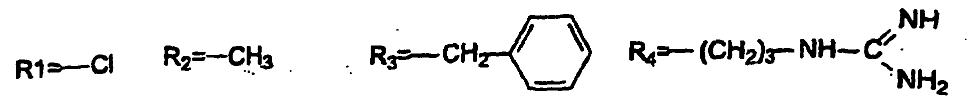

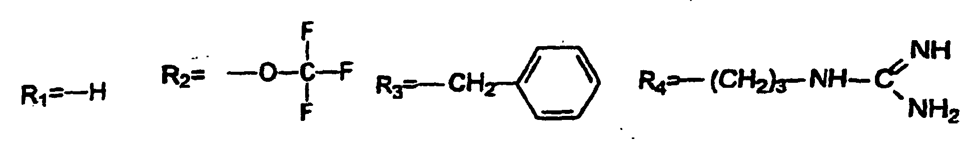

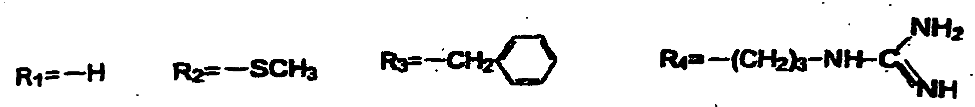

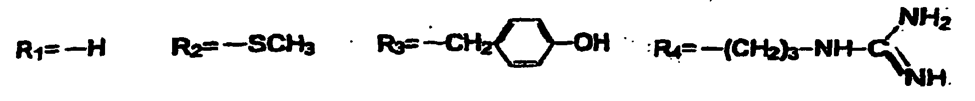

Les chiffres entre parenthèses déterminant la position des groupements méthyl sur le radical phényl.

La carboxypeptidase A utilisée peut provenir de différentes sources, telles que, par exemple, le pancréas ou des cellules mastocytaires. Elle peut être d'origine humaine ou animale.

On utilise préférentiellement dans le cadre de l'invention de la carboxypeptidase A pancréatique d'origine bovine.

Le principe de la méthode de l'invention est basé sur une hydrolyse d'un substrat coloré de formule (I) tel que décrit ci-dessus, par l'action de la carboxypeptidase U éventuellement présente dans l'échantillon analysé, associée à celle de la carboxypeptidase A ajoutée dans le milieu. Cette hydrolyse spécifique conduit à une extinction de la coloration du composé de départ qui peut être suivie à l'aide d'un spectrophotomètre.

Plus précisément, compte tenu des spécificités d'hydrolyse des carboxypeptidases N ou U et A exposées précédemment, et des caractéristiques structurales des composés de formule (I) décrits ci-dessus, la mise en contact d'une carboxypeptidase A et d'une carboxypeptidase U activée avec un tel composé va générer une coupure au niveau de la liaison entre le premier et le second acide aminé dudit composé du fait de faction de la carboxypeptidase U, suivie quasi simultanément d'une coupure au niveau de la liaison entre le groupement azoformyl et le premier acide aminé, du fait de l'action de la carboxypeptidase A.

La résultante de ces deux réactions sera une décoloration du milieu de départ. Il sera alors aisé de suivre en fonction du temps cette décroissance de coloration en se plaçant à une longueur d'onde d'absorption du colorant [composé formule (I)], de préférence à la longueur d'onde correspondant à son maximum (pic) d'absorption.

Cette décroissance est représentative de la cinétique d'hydrolyse. La quantité de carboxypeptidase U active présente initialement dans l'échantillon est calculée en comparant la densité optique mesurée à celle d'une courbe d'étalonnage qui peut par exemple être établie à partir d'une gamme de concentrations de carboxypeptidase U purifiée active en solution.

Inversement, si l'échantillon ne contient pas de carboxypeptidase U ou si celle-ci est inactive, il n'y aura pas de coupure entre le premier et second acide aminé du composé de formule (I), et donc, la carboxypeptidase A ne pourra pas agir au niveau de la liaison du groupement coloré sur le premier acide aminé. De ce fait, on n'observera pas de diminution de la coloration de la solution de départ.

L'invention vise plus particulièrement une méthode de dosage de l'activité d'une CPU dans un échantillon biologique, notamment un échantillon sanguin tel que du sang total ou du plasma pur ou dilué, et, préférentiellement, une méthode de dosage de l'activité du TAFI dans un échantillon sanguin, notamment du plasma pur ou dilué.

Dans ce cas, l'échantillon à tester est d'abord mis en présence d'une solution tampon avec, si nécessaire, un activateur de la carboxypeptidase dont on souhaite mesurer l'activité (solution nommée tampon activateur ci-après), et laissé en incubation, le temps nécessaire pour obtenir l'activation de la carboxypeptidase étudiée. L'incubation est de préférence réalisée à température ambiante pendant 5 à 20 minutes,avantageusement 8 à 12 minutes, de préférence 10 minutes.

Selon un mode de réalisation préféré de l'invention, l'activateur de carboxypeptidase peut être un activateur, notamment un facteur de la coagulation, que l'on laisse agir de façon à activer la carboxypeptidase U, en particulier le TAF4.

Dans ce cas, on ajoute ensuite dans le mélange un inhibiteur des sérine protéases pour aboutir à un blocage du processus de coagulation. L'inhibiteur de sérine protéases est par exemple le PPACK (H-D-Phe-Pro-Arg-chloromethylkétone - Bachem - Réf. N.1065) utilisé dans une gamme de concentration finale de 1 à 50 µM, de préférence de 30 µM, correspondant à des concentrations initiales de 10 à 250 µM et de préférence 150 µM. D'autres composés, tels que le Péfabloc [4-(2-aminoethyl)-benzènesulfonylfluoridehypochloride - Penthapharm - Réf. 399.01] utilisé préféren-tiellement à une concentration de 0,1 mM conviennent également

Simultanément ou immédiatement après l'addition de l'inhibiteur, on ajoute l'un des composés de formule (I) selon l'invention en solution aqueuse. Ce composé est en général utilisé dans une gamme de concentrations finales comprises entre 0,25 et 10 mM, de préférence entre 0,25 et 2,5 mM, avantageusement entre 0,25 et 1mM. Il est de préférence utilisé à 0,4 mM.

Après une nouvelle phase d'incubation, de préférence à température ambiante, durant laquelle le substrat de formule (I) aura été coupé au niveau de son second acide aminé par la CPU de l'échantillon dont on recherche l'activité, l'hydrolyse peut être arrêtée par addition d'HCl, suivie immédiatement par l'ajout d'une base pour réajuster le pH à une valeur comprise entre 7 et 8.

La densité optique du mélange ainsi obtenu est alors mesurée une première fois sans addition de CPA dans le milieu. La CPA est ensuite ajoutée dans le milieu, et la DO est à nouveau mesurée. Sa décroissance peut être suivie au spectrophotomètre. Elle traduit l'hydrolyse du substrat de formule (I) par l'action conjointe de la CPU de l'échantillon et de la CPA.

Les delta DO (Δ DO) mesurés sont comparés avec les valeurs portées sur une courbe d'étalonnage qui peut par exemple être obtenue à partir d'un échantillon déficient en la CPU étudiée, surchargé avec cette enzyme purifiée.

Le protocole décrit ci-dessus constitue un mode de réalisation préféré de l'invention. Toutefois, plusieurs variantes de celui-ci sont envisageables, tant en ce qui concerne les séquences d'addition des différents composés utilisés, que la nature de ces composés. Elles entrent également dans la définition de la présente invention.

Ainsi, Il est par exemple possible d'incorporer le substrat de formule (1) dès le début de la méthode, en même temps que le tampon activateur de la coagulation.

De même, on peut réaliser le dosage à partir de deux aliquots du même milieu réactionnel, dont un seul est traité avec de la CPA. On mesure ensuite le ΔDO entre ces deux aliquots.

Dans le cas où le tampon activateur active la coagulation, l'activation peut être réalisée selon différentes voies connues de l'homme du métier, et utilisées en routine dans les tests de coagulation.

Ces voies d'activation sont notamment :
- la voie d'activation par le complexe thrombine/thrombomoduline. Cette première possibilité constitue un mode de réalisation préféré de la présente invention.
- la voie d'activation par le facteur XI activé, qui dans ce cas remplace la thrombine dans le tampon activateur. Pour accélérer la réaction, il peut être avantageux de rajouter également de la thrombomoduline dans le milieu.
- la voie d'activation par des venins, notamment des venins de serpent, avec également l'ajout éventuel de prothrombine et/ou thrombomoduline dans le milieu. Les venins préférentiellement utilisés sont les venins de la famille des "thrombin-like" tels que le venin d'Arkistrodon rhodostoma ou Bathrops atrox, ainsi que les venins d'activateurs de prothrombine, tels que celui de Notechis scutatus ou d'Echis carinatus (15 - 18).
- la voie d'activation par le facteur tissulaire, en présence de phospholipides et de calcium (activation de la voie exogène selon le principe d'un temps de Quick),
- la voie d'activation de la phase contact,
- la voie d'activation par la plasmine.

Avantageusement, la méthode selon l'invention permet de mesurer pour un même échantillon, d'une part l'activité de la CPU "constitutionnelle'' présente dans celui-ci, i.e. la CPU présente sous une forme active "naturellement" (i.e. sans induction de l'activation par un tampon activateur), et d'autre part, l'activité de la CPU activable, i.e. la CPU présente dans l'échantillon sous une forme inactive, dont l'activité sera induite ou générée durant le processus d'activation lié à l'effet du tampon activateur.

Pour cela, on compare l'activité d'hydrolyse sur un substrat de formule (I) d'un échantillon selon la méthode décrite ci-dessus, en mettant l'échantillon soit en présence de tampon activateur (détection de l'activité totale de la CPU dans l'échantillon), soit en présence d'un tampon physiologique sans activateur (détection de l'activité de la CPU constitutionnelle).

La différence de delta de DO entre les deux mesures permet d'obtenir l'activité de la CPU activable présente dans l'échantillon.

Selon une variante préférée, la présente invention a pour objet une méthode de dosage du TAFI activé dans un échantillon biologique, notamment un échantillon sanguin, ladite méthode utilisant l'un des composés ou substrats de formule (I) décrits précédemment

L'activité du TAFI constitutionnel et/ou activable est mesurée en traitant l'échantillon tel que cela vient d'être décrit, en présence et en absence d'un inhibiteur spécifique du TAFI.

L'inhibiteur qui est de préférence utilisé est le CPI ("Carboxypeptidase Inhibitor from Potato Tubers) dont l'utilisation dans des études *in vitro* et *in vivo* sur la fonction du TAFI a été largement décrite dans la littérature (Réf. 1). Il est aujourd'hui bien connu de l'homme du métier que le CPI inhibe spécifiquement le TAFI sans altérer l'activité d'autres CP plasmatiques. Dans le cadre de la méthode de l'invention, le CPI peut être utilisé dans une gamme de concentrations allant de 0,10 à 0,50 mM (concentrations initiales), ou de 2 à 10 µM exprimés en concentrations finales. Il est de préférence utilisé à 7µM (concentration finale) ou 0,38 mM (concentration initiale).

Selon le principe de cette variante préférée, l'échantillon est activé par le tampon d'activation du TAFI, par exemple un tampon d'activation de la coagulation par le complexe thrombine/thrombomoduline, et traité selon la méthode décrite précédemment soit en présence soit en l'absence d'inhibiteur du TAFI.

La différence de coloration du milieu avec et sans inhibiteur du TAFI permet ainsi de déterminer l'activité enzymatique propre au TAFI activé (TAFIa) dans l'échantillon.

Plus précisément, la méthode de l'invention peut être réalisée selon le protocole suivant : l'échantillon à tester est divisé en 2 aliquotes ou plus si nécessaire, selon que l'on souhaite déterminer l'activité de tout le TAFI contenu dans l'échantillon ou que l'on souhaite faire la distinction entre l'activité du TAFI constitutionnel et celle du TAFI activable.
- Un premier aliquote contenant l'inhibiteur du TAFI est activé et traité selon la méthode décrite précédemment. Celui-ci ne va donc générer qu'une faible décoloration du milieu, résultant d'une hydrolyse résiduelle du substrat de formule (I) par d'autres carboxypeptidases présentes dans l'échantillon.
- Un second aliquote est traité de manière identique au précédent, mais en l'absence d'inhibiteur du TAFI. Il va en résulter une forte décoloration du milieu, liée à l'hydrolyse du substrat de formule (I) par le TAFI activé dans l'échantillon.
- On mesure la différence de coloration (Δ DO) entre le premier et le second aliquote.
Celle-ci est ainsi représentative de l'activité spécifique du TAFI activé dans l'échantillon.

Si l'on souhaite faire la distinction entre l'activité spécifique du TAFI activable et celle du TAFI constitutionnel, on utilise un troisième aliquote du même échantillon dont on mesure également l'activité d'hydrolyse sur le substrat de formule (I), mais dans lequel on n'aura pas déclenché l'activation, en n'ajoutant pas de tampon activateur dans le milieu.

Les Δ DO entre ces différents aliquotes permettent d'obtenir soit l'activité du TAFI total de l'échantillon, soit celle du seul TAFI constitutionnel, et donc d'en déduire celle du TAFI activable.

Selon un troisième aspect, l'invention a pour objet un kit de diagnostic pour le dosage d'une CPU, ledit kit de diagnostic comprenant un substrat de formule (I) tel que défini précédemment.

Préférentiellement, le kit de diagnostic selon la présente invention est un kit pour mesurer l'activité du TAFI dans un échantillon sanguin.

Un tel kit de diagnostic comprend notamment :
- un activateur du TAFI, notamment un tampon activateur du TAFI,
- de la carboxypeptidase A,
- un substrat de formule (I),
- un inhibiteur du TAFI.

Le kit comprend également optionnellement un inhibiteur des serine protéases.

Chacun de ces composants se présentent préférentiellement en poudre ou sous forme lyophilisée.

Les exemples ci-après et les figures illustrent la présente invention.
Figure 1 : Spectre d'absorption des différents composés de formule (1) synthétisés, mesuré avec un spectrophotomètre UV-visible.
Figure 2 : Analyse du AAFFR effectuée par hydrolyse acide.
Figure 3: Courbe d'étalonnage établie à partir d'un plasma déficient en TAFI, surchargé avec du TAFI purifié pour obtenir une gamme de concentration allant de 0 à 26 µg/ml.
Figure 4: courbe d'étalonnage pour une gamme de concentration, obtenue à partir d'un plasma pool. La calibration est réalisée en méthode génération de thrombine. Le dosage colorimétrique est fait avec le substrat chromogène 4-MTPAFYR.

### EXEMPLE N° 1 :

### Synthèse d'un groupe de composés azolformyl selon l'invention.

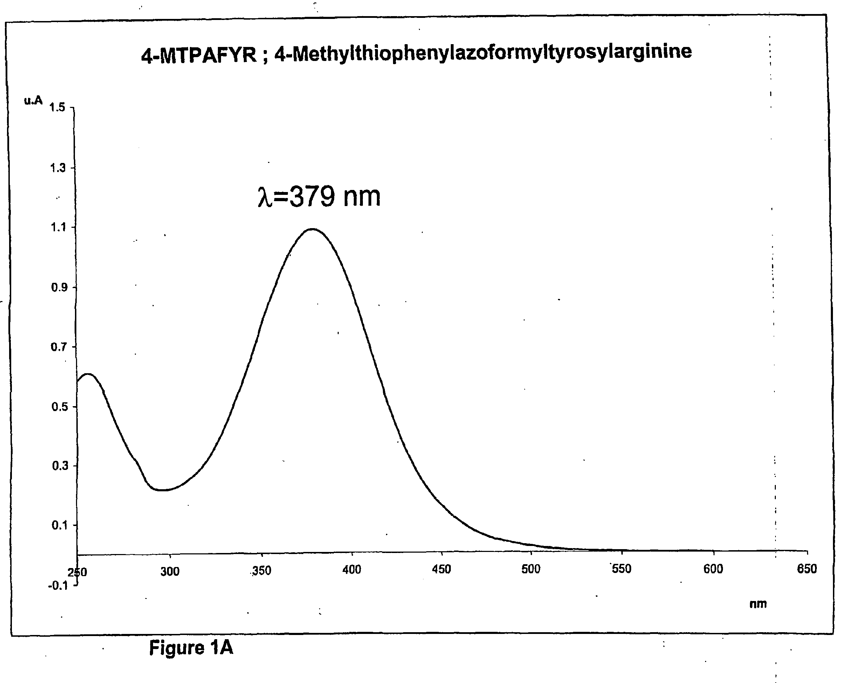

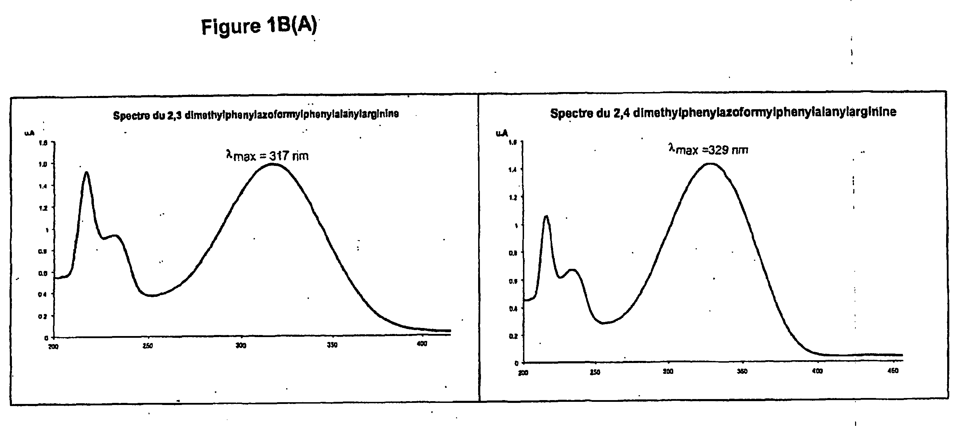

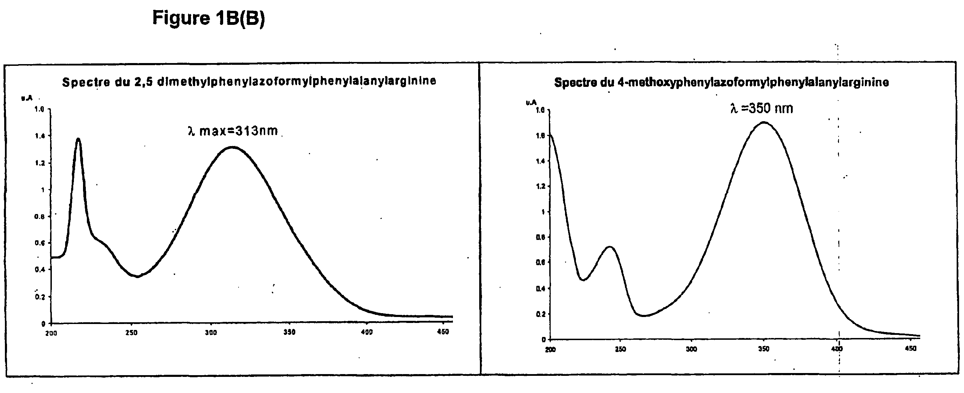

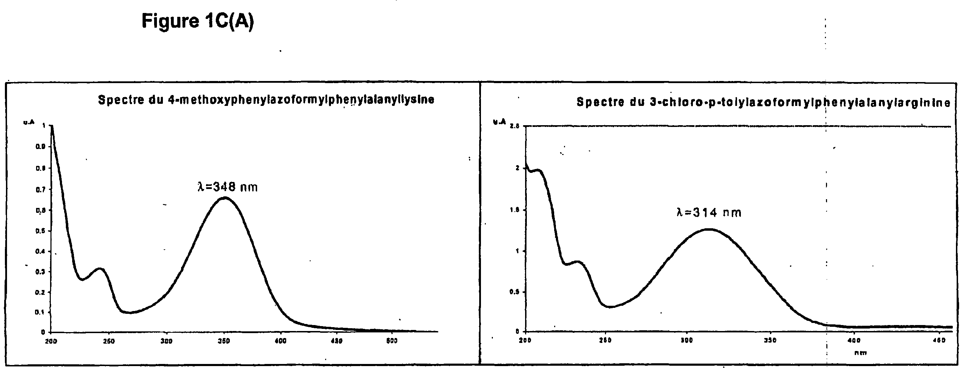

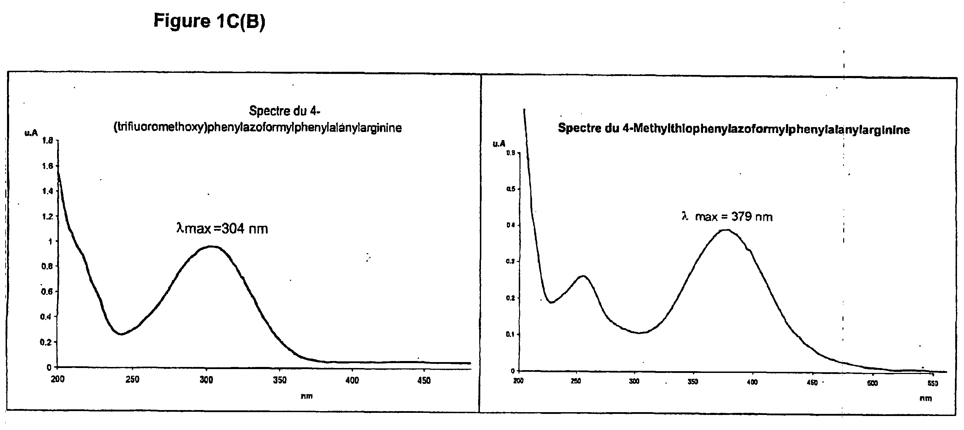

Le tableau I ci-après indique les formules chimiques de 8 des composés préférés parmi les composés de formule (I) selon l'invention. Leurs spectres d'absorption mesurés avec un spectrophotomètre UV-visible (UVIKON-KONTRON) sont reportés sur la figure 1.

On décrit ci-après en détail la synthèse de l'un d'eux, le composé n° 4 (MxPAFFR). Les étapes décrites ci-dessous pour ce composé sont identiques pour chacun des autres, excepté pour le composé n° 5 (MxPAFFK) pour lequel une étape supplémentaire de déprotection de la lysine est nécessaire (voir schéma réactionnel et explications ci-après).

**TABLEAU 1 :**

| **FORMULES** |
|---|
| **Composé 1 : 2,3DMPAFFR** |
| 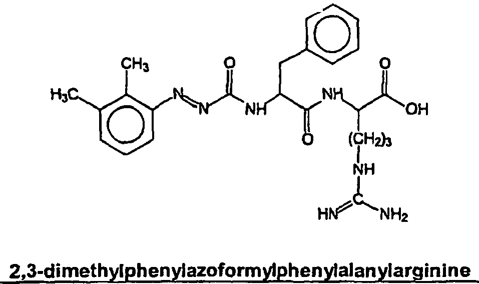 |

| **Composé 2 : 2,4 DMPAFFR** |
|---|
| 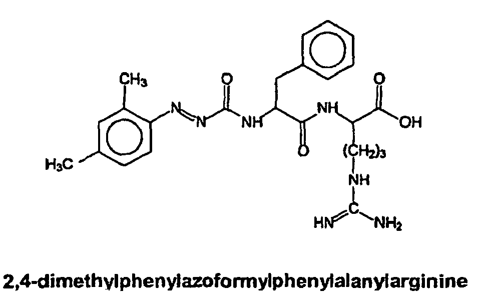 |

| **Composé 3 : 2,5 DMPAFFR** |
|---|
| 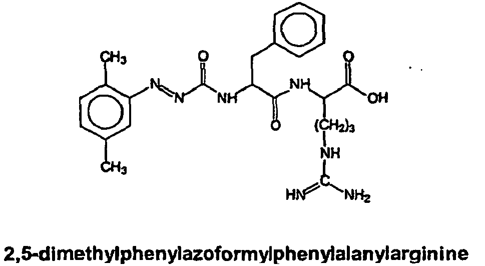 |

| **Composé 4 : MxPAFFR** |
|---|
| 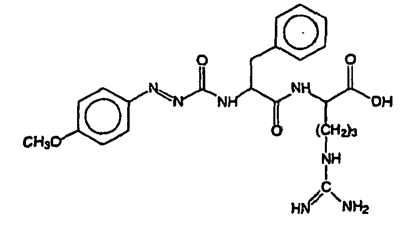 |
| 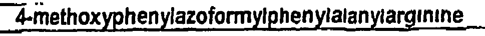 |

| **Composé 5 : MxPAFFK** |
|---|
| 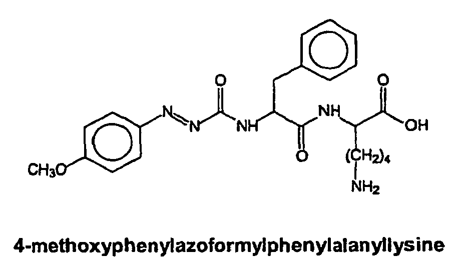 |

| **Composé 6 : CITAFFR** |
|---|
| 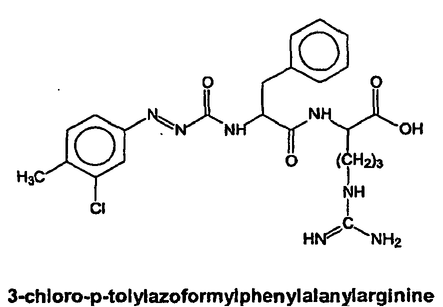 |

| **Composé 7:TFMxPAFFR** |
|---|
| 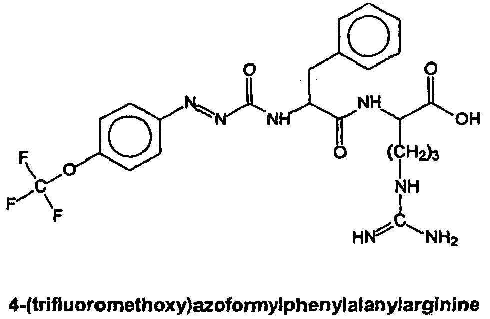 |

| **Composé 8:4-MTPAFFR** |
|---|
| 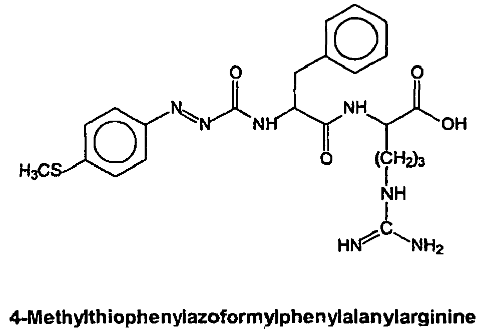 |

| **Composé 9:4-MTPAFYR** |
|---|
| 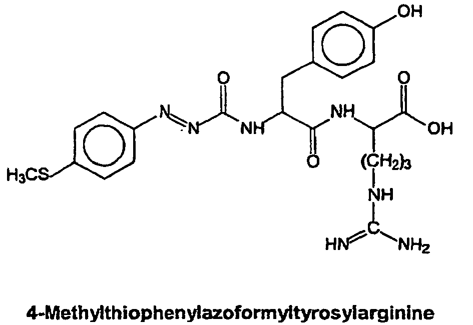 |

### A/ Synthèse du 4-methoxyphenylazoformylphenylalanylarginine (Composé 4) :

**Introduction** : Toutes les étapes décrites ci-dessous sont identiques pour chaque colorant. Chaque étape est suivie par Chromatographie Liquide Haute Performance et par Analyse d'Acides Aminés pour les couplages avec la Phenylalanine et l'Arginine.

### 1^{ère} étape : Réactif de Grignard

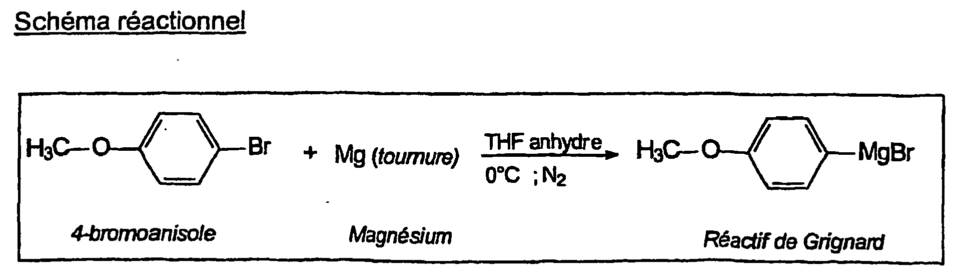

Mode opératoire : Préparer 1 éq. de 4-bromoanisole et 1 éq. de magnésium. Introduire le magnésium et 20 % en masse du 4- bromoanisole dans un tricol, reprendre dans du tétrahydrofuranne anhydre (2mUmmol) et placer le montage sous azote. Initier la réaction en chauffant le tricol en un point avec un décapeur thermique, le milieu réactionnel devient marron. Quand la réaction démarre (effervescence à la surface du magnésium), placer à reflux à 90 °C puis additionner lentement le reste du 4-bromoanisole repris dans du THF anhydre (2mUmmol). Arrêter le reflux après 1h. Le bromure de 4-methoxyphenylmagnesium est utilisé tel quel pour l'étape suivante (couleur marron).

Remarque : La verrerie et le magnésium sont préalablement séchés à l'étuve à 120 °C et les autres produits au dessiccateur.

Temps de rétention : Le réactif de Grignard étant un composé très instable et sensible à l'eau, on obtient donc trois temps de rétention caractéristiques des produits de dégradation formés lors de l'analyse HPLC :
Temps de rétention 1 : 2.68 min
Temps de rétention 2 : 3.32 min
Temps de rétention 3 : 5.38 min

### 2^{ème} étape: Couplage avec le di-t-butylazodicarboxylate

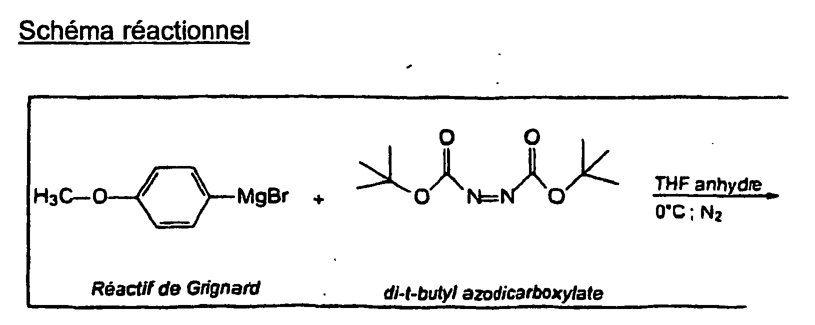

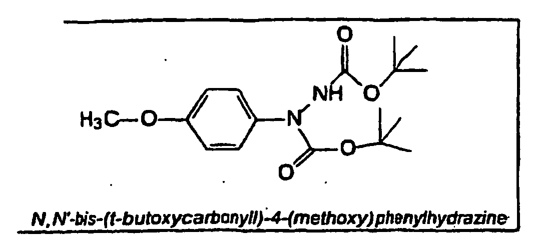

Mode opératoire : Reprendre à part 1 eq. de di-t-butylazodicarboxylate et 1 eq. de 4-methoxyphenylmagnesiumbromide (réactif de Grignard) dans du THF anhydre (2 mL/mmol). Refroidir chacun entre 0-5°C. Additionner le réactif de Grignard sur le di-t-butylazodicarboxylate. Agiter 10 min, ajouter 1.02 eq. d'acide acétique puis laisser revenir à température ambiante. Effectuer une extraction eau/éther. Sécher la phase éthérée, évaporer à sec. On obtient une huile jaune.
Temps de rétention : 7.85 min

### 3^{ème} étape : Déprotection

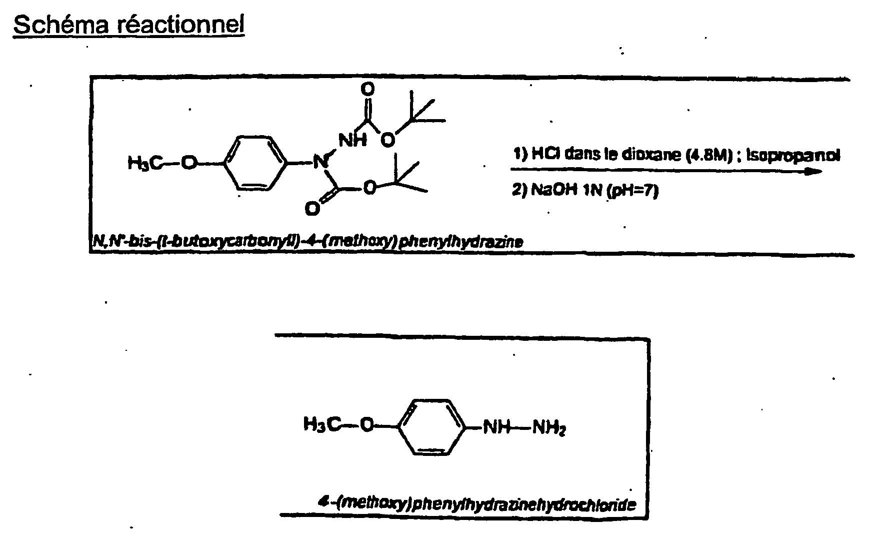

Mode opératoire : Reprendre 1 eq. De N, N'-bis-(t-butoxycarbonyl)-4-(methoxy)phenylhydrazine dans de l'isopropanol (10.2mL/mmol) puis ajouter du HCl dans le dioxane à 4.8 M (2mL/mmol). Porter à reflux (80°C). Après 15 min, refroidir, ajouter de l'ether (5mL/mmol). La 4-methoxyphenylhydrazinehydrochloride précipite. Filtrer et sécher. Reprendre la poudre dans de l'eau puis amener à pH 7. Effectuer une extraction eau / dichloromethane et évaporer la phase dichloromethane à sec. On obtient la 4-methoxyphenylhydrazine (poudre jaune) qui est séchée.
Temps de rétention : 2.20 min

### 4^{ème} étape : Couplage avec le diphenylcarbonate

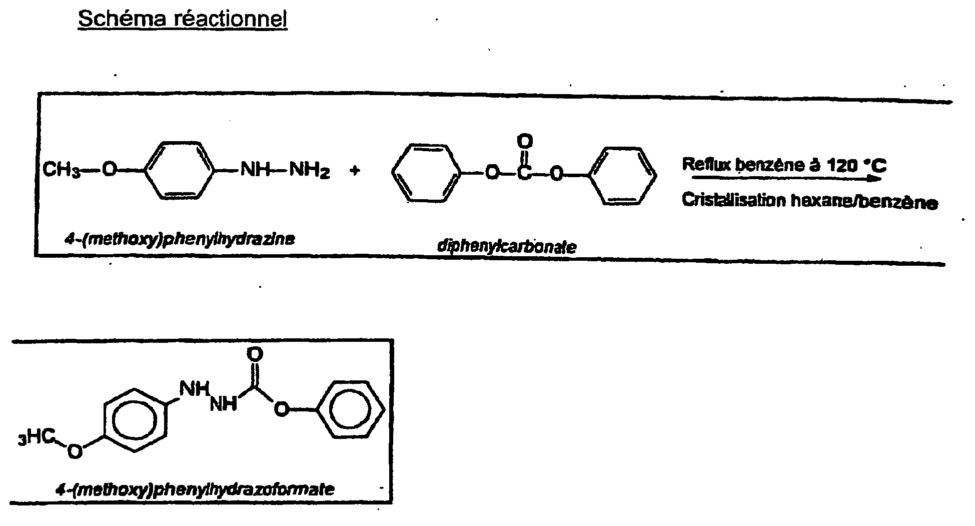

Mode opératoire : Reprendre 1.4 eq. de diphenylcarbonate dans du benzène (0.25 mL/mmol) et porter à reflux à 120 °C. Additionner lentement 1 eq. de 4-methoxyphenylhydrazine repris dans du benzène (0.7 mL/mmol). Arrêter le reflux après 6h. Concentrer le milieu réactionnel puis effectuer une cristallisation hexane/benzène (4/1). Filtrer et sécher les cristaux blanchâtres au dessiccateur.
Temps de rétention : 6.74 min

### 5^{ème} étape: Couplage avec la phénylalanine

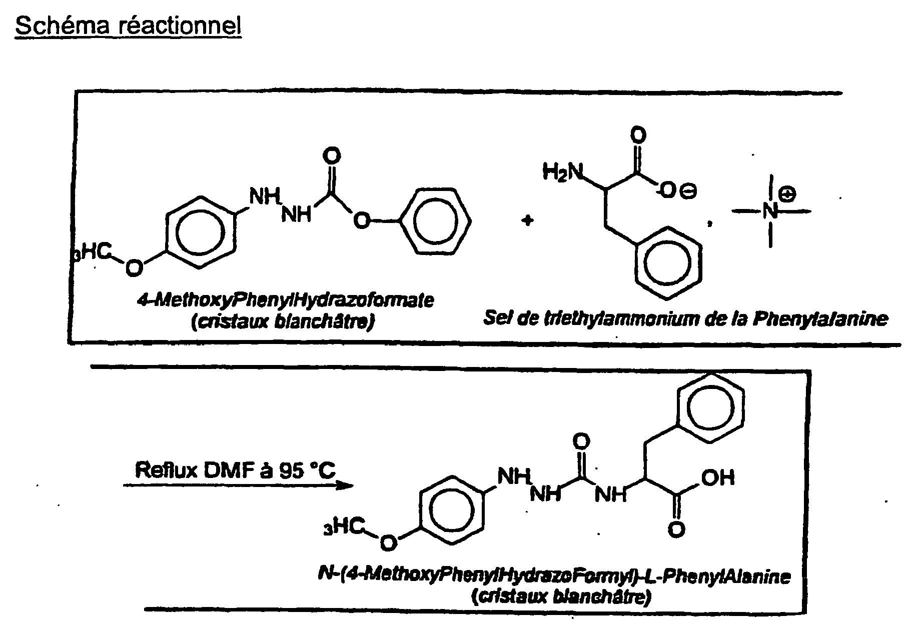

Mode opératoire : Porter à reflux (95°C) 1.2 eq. de sel de triethylammonium de la phenylalanine repris dans de la dimethylformamide (3.33mL/mmol). Additionner lentement 1 éq. de 4-methoxyphenylhydrazoformate repris dans de la dimethylformamide (2mL/mmol). Arrêter le reflux après 1h30, laisser revenir à température ambiante, concentrer. Acidifier (pH =2).Purifier par chromatographie (cristaux blanchâtre).
Temps de rétention : 6.12 min

### 6^{ème} étape: Oxydation ; Passage de la forme "hydrazo" à la forme "azo"

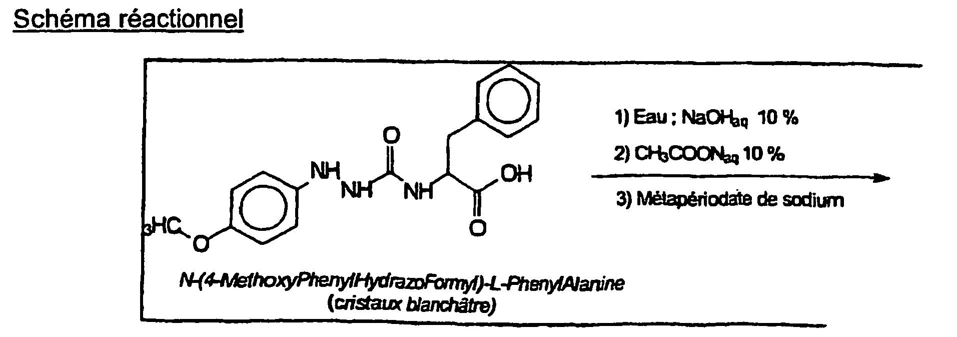

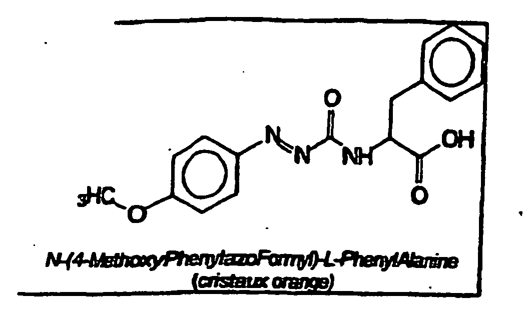

Mode opératoire : Reprendre 1 éq. de N-(4-methoxyphenylhydrazoformyl)-L-Phenylalanine dans de l'eau ultra-pure (20.8 mL/mmol) contenant 1éq. d' hydroxyde de sodium et 1 éq. d'acétate d'ammonium. Additionner 1 éq. de metaperiodate de sodium repris dans de l'eau ultra-pure (4.2 mL/mmol). Après 20 min le milieu réactionnel est acidifié (pH=2) puis purifié par chromatographie (cristaux orange).
Temps de rétention : 6.86 min

### 7^{ème} étape: Couplage avec l'Arginine

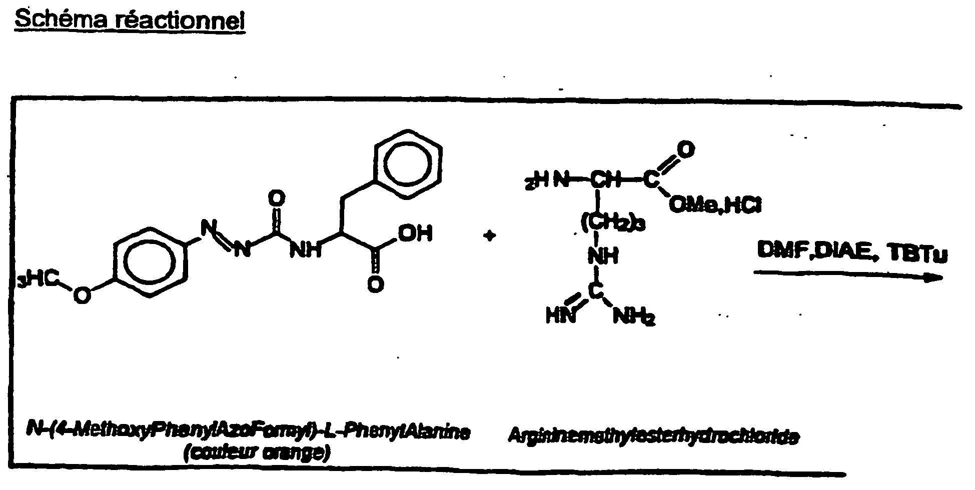

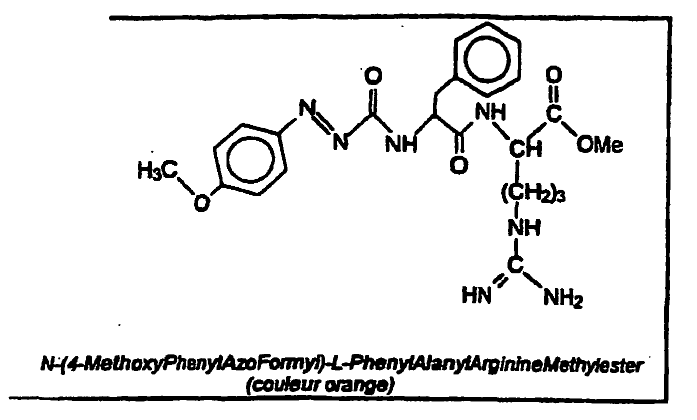

Mode opératoire : Reprendre 1 éq. d'Argininemethylester,hydrochloride dans de la dimethylformamide (3mL/mmol). Ajouter 1.1 éq. de DIAE (Diisopropylethylamine), 1éq. de N-(4-methoxyphenylazoformyl)-L-Phenylalanine puis 1.1 éq. de TBTU (O-(Benzotriazol-1-yl)-N,N,N',N'-tetramethyluronium tetrafluoroborate). Ajouter la quantité nécessaire de DIAE pour avoir un pH à 7-8. Après 5 min, évaporer à sec et purifier par chromatographie (cristaux orange).
Temps de rétention : 6.06 min

### 8^{ème} étape : Saponification

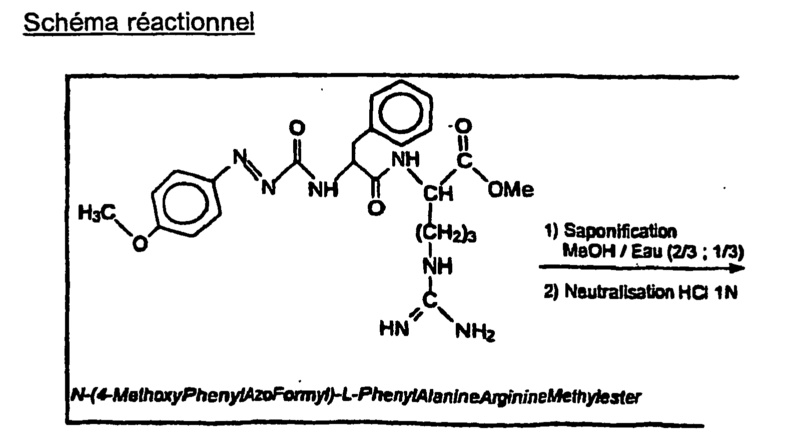

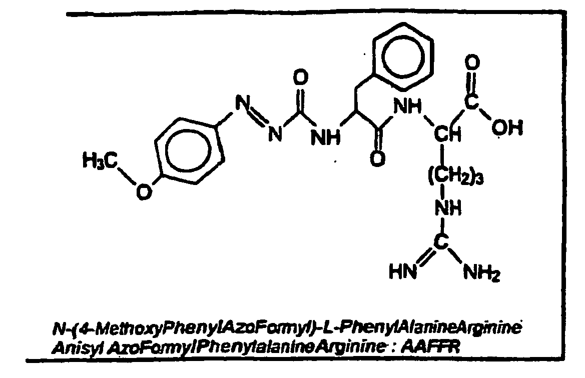

Mode opératoire : reprendre 1éq. de N-(4-methoxyphenylazoformyl)-L-PhenylalanineArgininemethylester dans un mélange eau/methanol (Ratio : 1/2 ; 3 mUmmol). Additionner 3 éq. d'hydroxyde de sodium 1N. Après 1 h, acidifier le MR, concentrer puis effectuer une extraction eau/dichloromethane. La phase dichloromethane est évaporée puis séchée. On obtient le N-(4-methoxyphenylazoformyl)-L-PhenylalanylArginine qui est une poudre orange.
Temps de rétention : 5.5 min

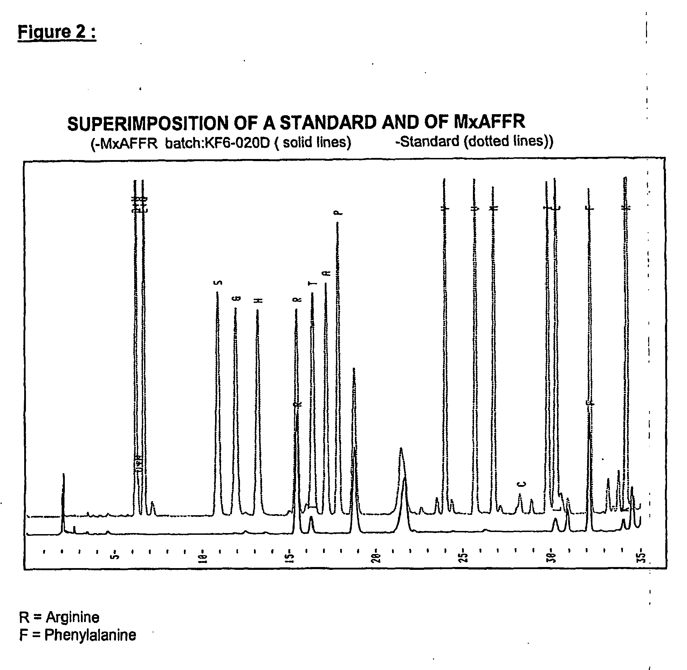

Une analyse d'acide aminé du produit final est effectuée après hydrolyse acide pour contrôler l'homogénéité de la séquence. Les résultats de cette analyse sont reportés sur le chromatogramme de la figure 2.

### Conditions analytiques :

➢ Chaîne HPLC AAA; Dérivatisation précolonne au PITC (phénylisocyanate)
➢ Colonne C18 ; 100A ; 5µ ; 250 x 4.6 mm
➢ Détection à 254 nm ; Débit = 1 mL/min
➢ Eluants :
   A : Tampon acétate pH 5.74
   B: Tampon 70% CH₃CN, 30% AcONa ; 32mM ; pH = 6.10
   C : CH₃CN

Les protocoles de synthèse pour les autres composés sont basés sur les mêmes étapes que celles qui viennent d'être décrites ci-dessus. Ceux-ci sont résumés dans les schémas réactionnels ci-après.

Les temps de rétention obtenus à chaque étape de synthèse de ces composés sont reportés dans le tableau Il ci-dessous :

**TABLEAU II : Temps de rétention obtenus sur HPLC à chaque étape pour les autres colorants synthétisés**

| Étape | 2,3DMPAFFR | 2,4DMPAFFR | 2,5DMPAFFR | MxAPFFR | MxPAFFK | CITAFFR | TFMxPAFFR |
|---|---|---|---|---|---|---|---|
| 1 | / | / | / | / | / | / | / |
| 2 | 9.12 min | 9.18 min | 9.15 min | 7.85 min | 7.85 min | 9.25 min | 9.26 min |
| 3 | 3.59 min | 3.81 min | 3.73 min | 2.20 min | 2.20 min | 3.93 min | 3.94 min |
| 4 | 7.87 min | 7.96 min | 7.82 min | 6.74 min | 6.74 min | 8.06 min | 7.92 min |
| 5 | 7.04 min | 7.13 min | 7.11 min | 6.12 min | 6.12 min | 7.51 min | 7.45 min |
| 6 | 7.82 min | 7.88 min | 7.90 min | 6.86 min | 6.86 min | 7.85 min | 7.79 min |
| 7 | 7.10 min | 7.02 min | 7.10 min | 6.06 min | 9.84 min | 7.13 min | 7.08 min |
| 8 | 6.55 min | 6.54 min | 6.62 min | 5.50 min | 9.23 min | 6.71 min | 6.65 min |

### Conditions analytiques :

➢ Colonne C18 ; 5µ ; 100x4.6mm
➢ Détection à 254 nm ; Débit = 1 mL/min
➢ Gradient : 0' → 10%B 10'→ 90%B
➢ Avec A : Eau à 0.1 % d'acide trifluoroacétique
   B : Acétonitrile à 0.1 % d'acide trifluoroacétique

### B/ Synthèse du 2,3-dimethylphenylazoformylphenylalanylarginine (Composé 1) :

### 1^{ère} étape : Réactif de Grignard

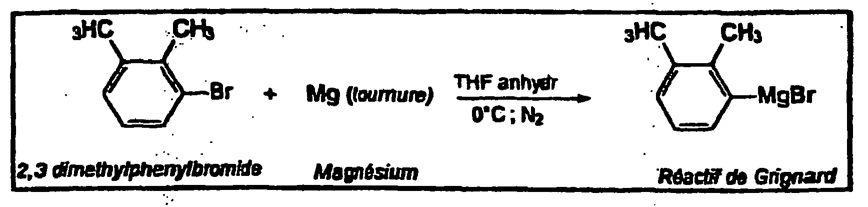

### 2^{ème} étape : Couplage avec le di-t-butylazodicarboxylate

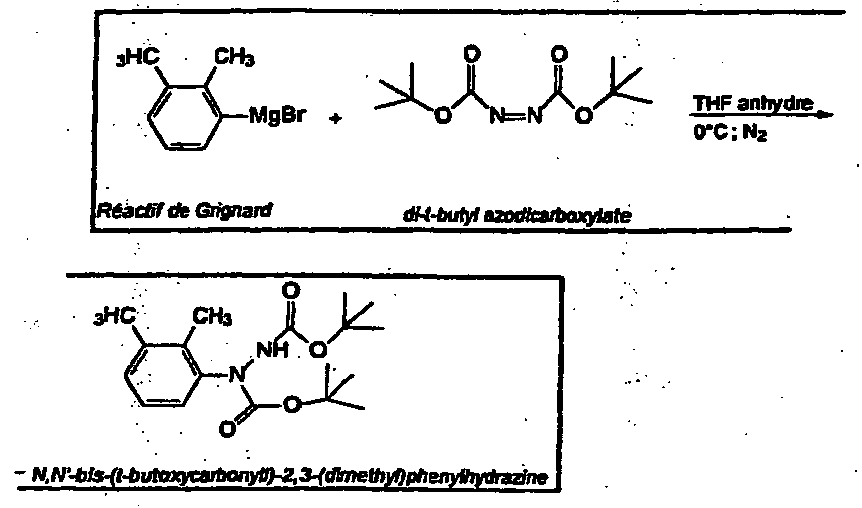

### 3^{ème} étape: Déprotection

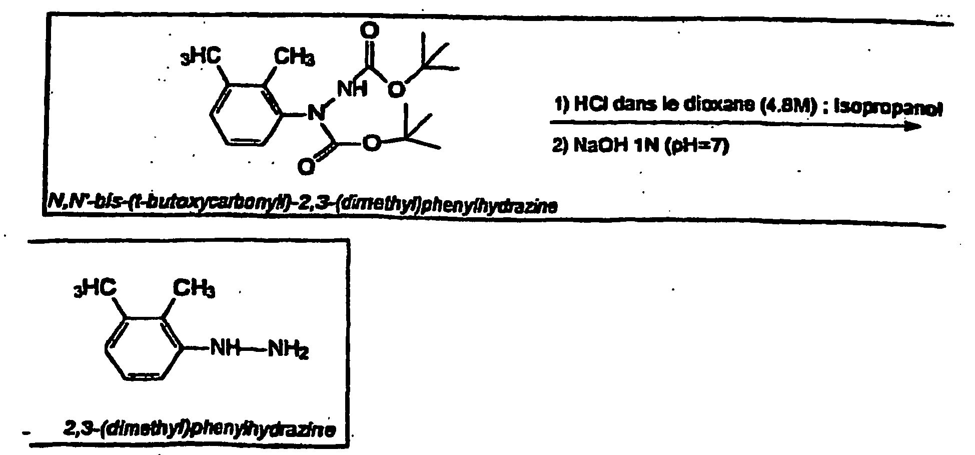

### 4^{ème} étape : Couplage avec le diphenylcarbonate

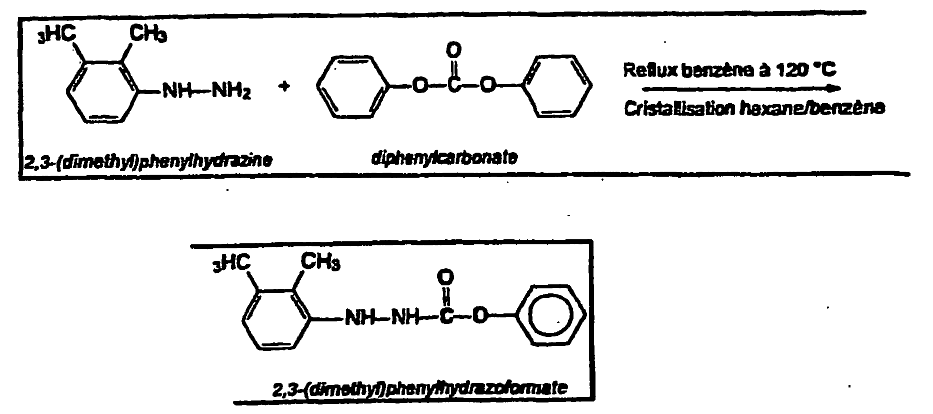

### 5^{ème} étape : Couplage avec la phénylalanine

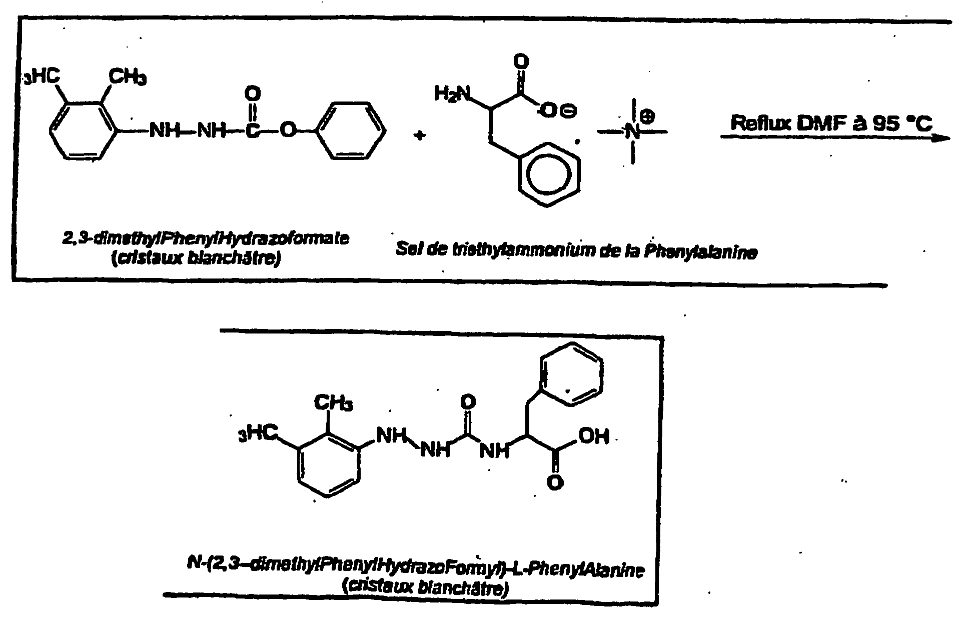

### 6^{ème} étape : Oxydation : Passage de la forme "hydrazo" à la forme "azo"

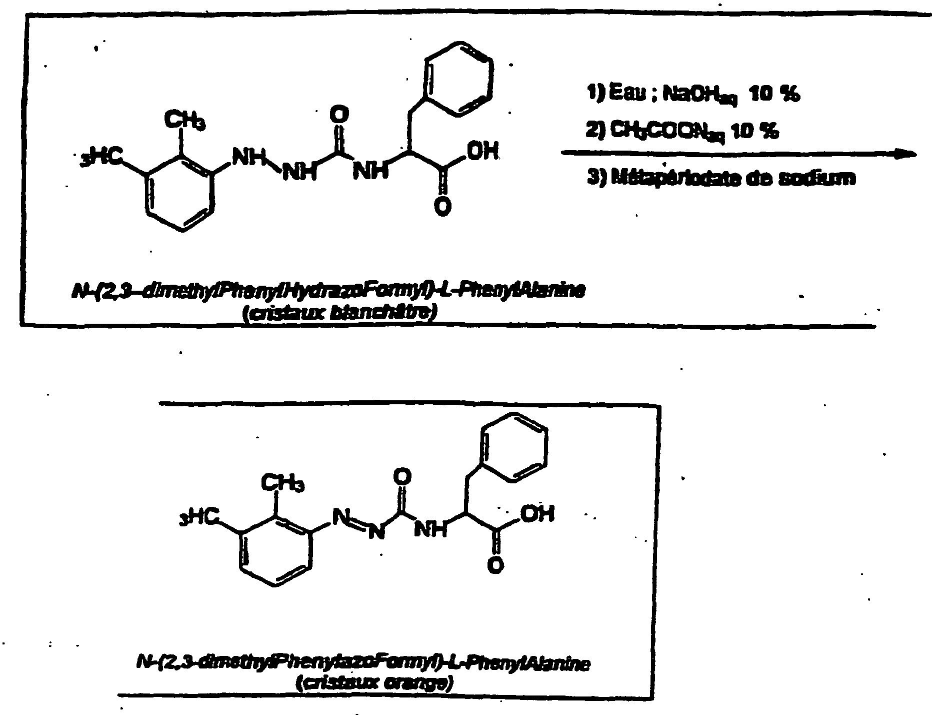

### 7^{ème} étape : Couplage avec l'Arginine

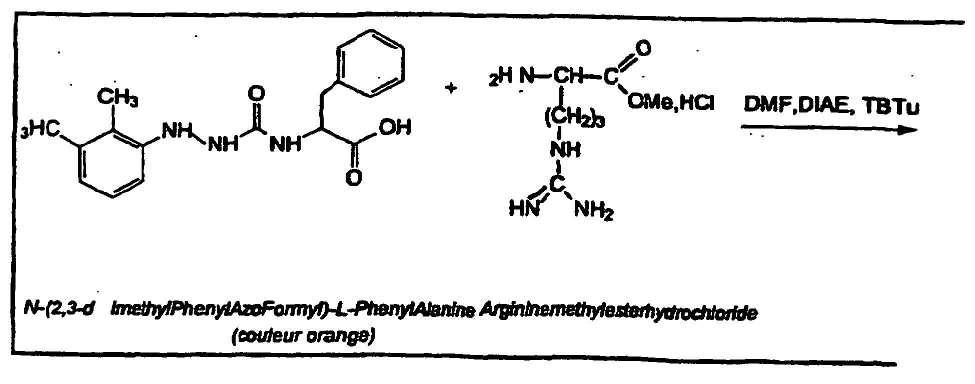

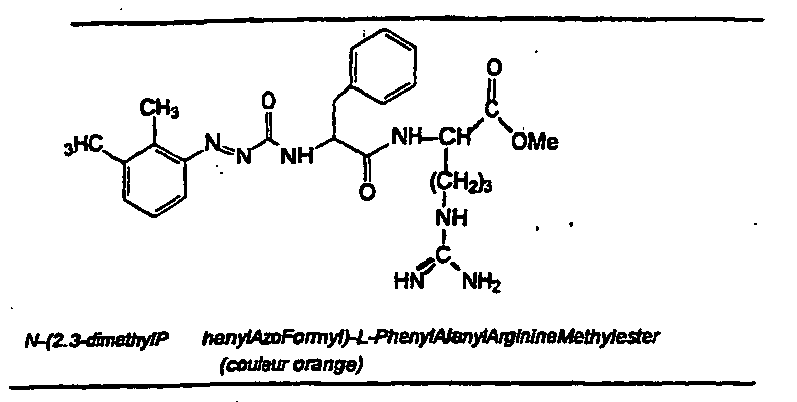

### 8^{ème} étape : Saponification

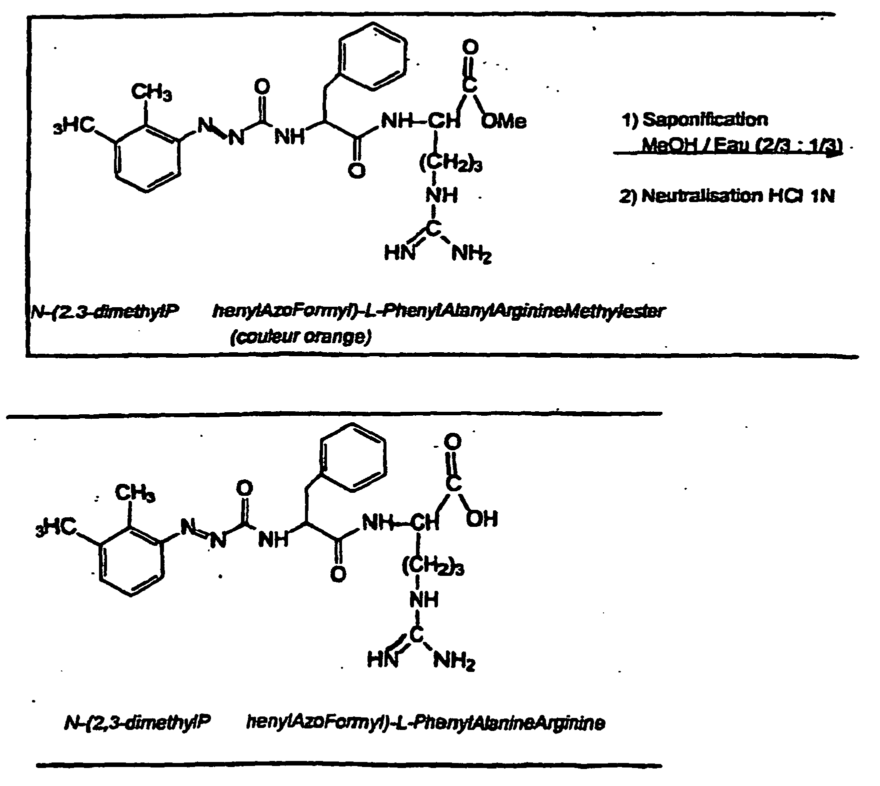

### C/ Synthèse du 2,4-dimethylphenylazoformylphenylalanylarginine (Composé 2):

### 1^{ère} étape : Réactif de Grignard

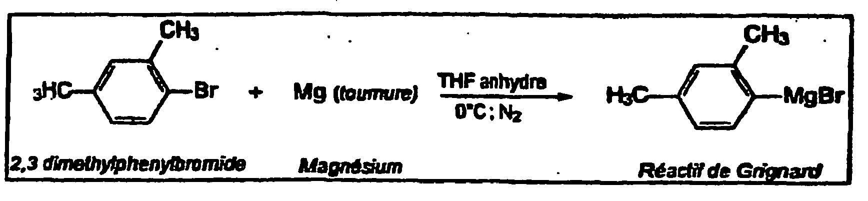

### 2^{ème} étape : Couplage avec le di-t-butylazodicarboxylate

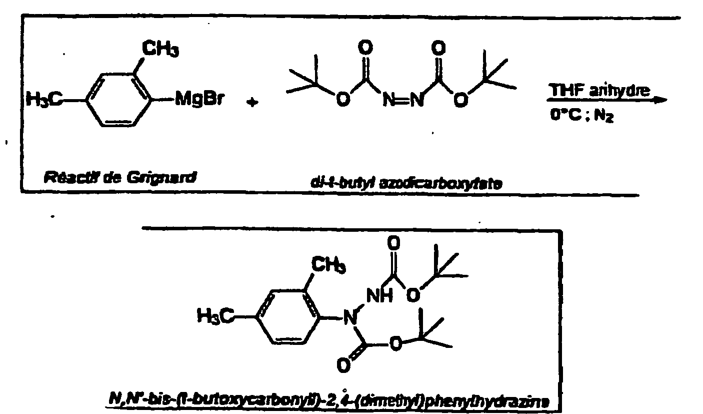

### 3^{ème} étape : Déprotection

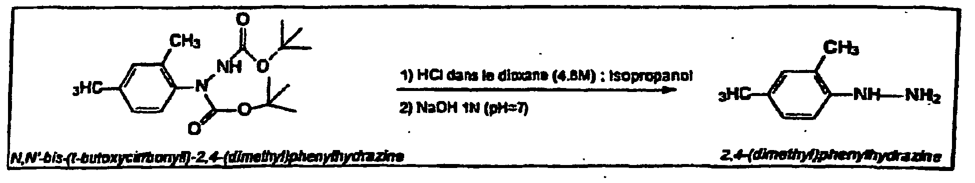

### 4^{ème} étape : Couplage avec le diphenylcarbonate

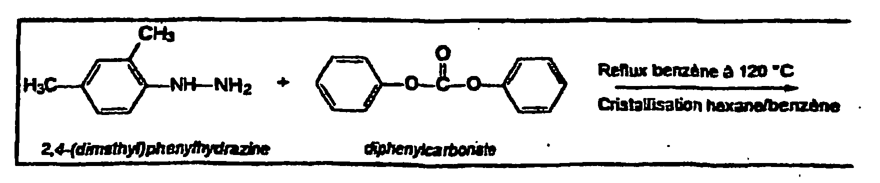

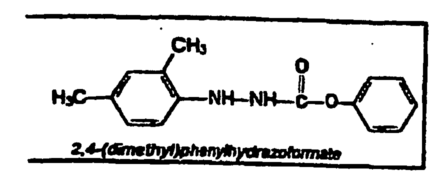

### 5^{ème} étape: Couplage avec la phénylalanine

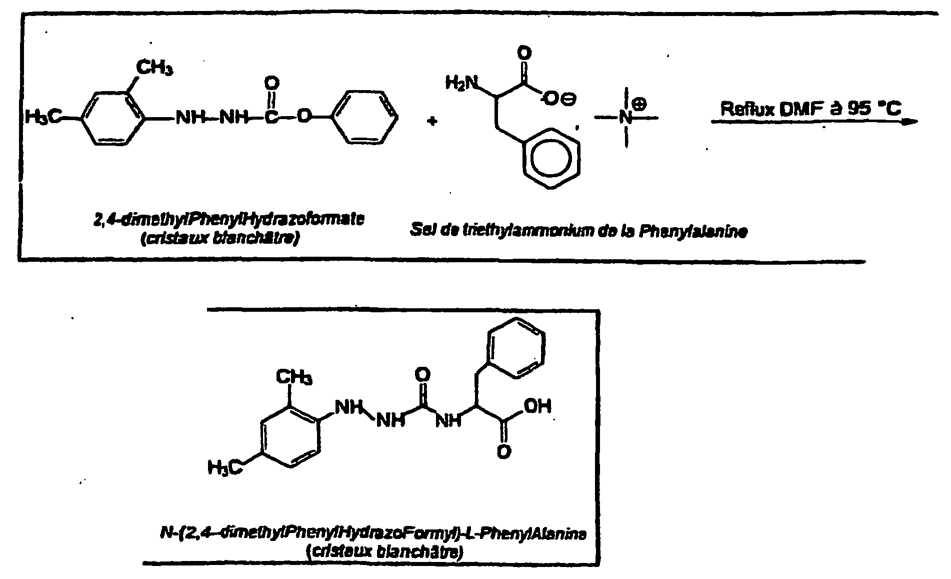

### 6^{è}me étape : Oxydation ; Passage de la forme "hydrazo" à la forme "azo"

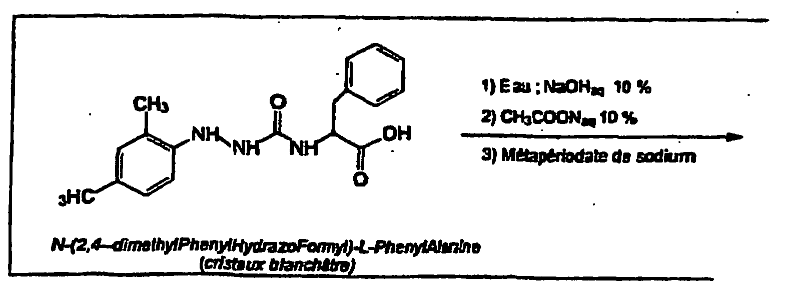

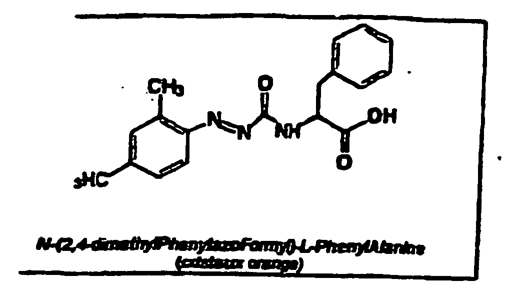

### 7^{ème} étape : Couplage avec l'Arginine

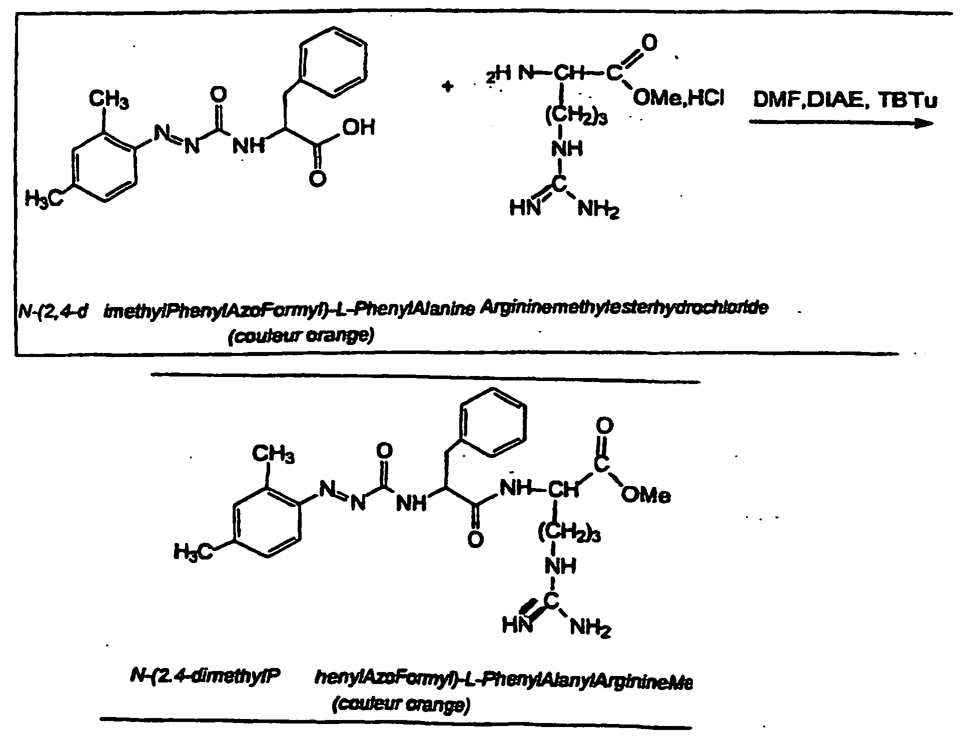

### 8^{ème} étape : Saponification

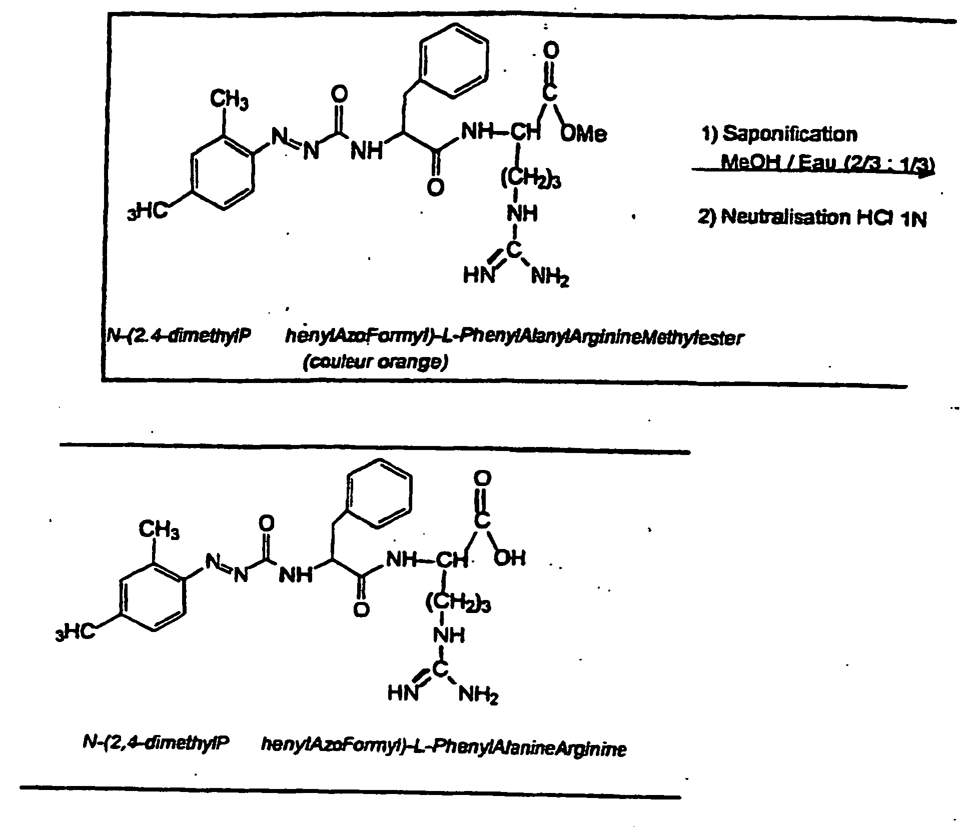

### D/ Synthèse du 2,5-dimethylphenylazoformylphenylalanylarginine (Composé 3):

### 1^{ère} étape : Réactif de Grignard

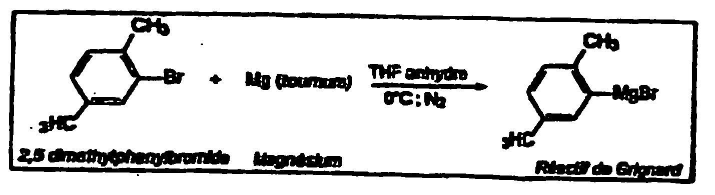

### 2^{ème} étape : Couplage avec le di-t-butylazodicarboxylate

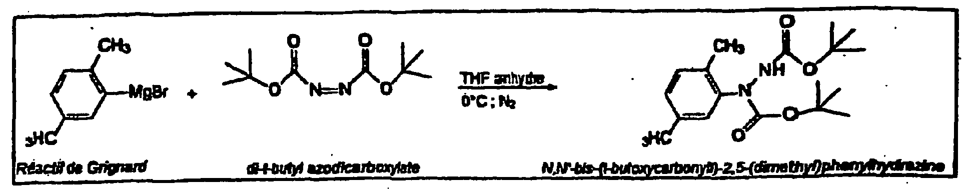

### 3^{ème} étape : Déprotection

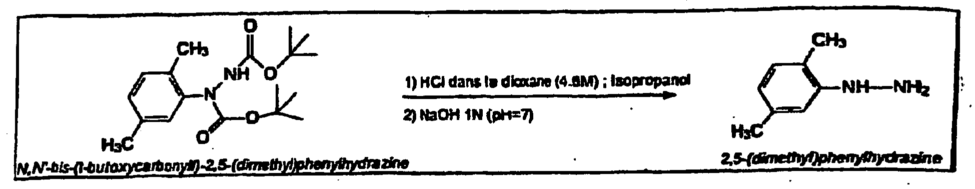

### 4^{ème} étape : Couplage avec le diphenylcarbonate

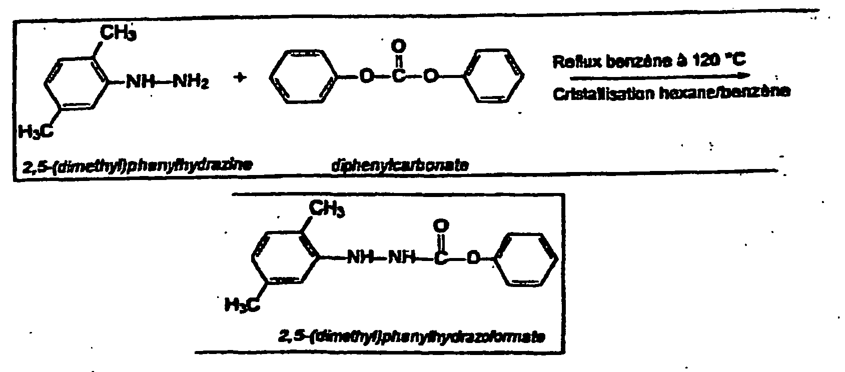

### 5^{ème} étape : Couplage avec la phénylalanine

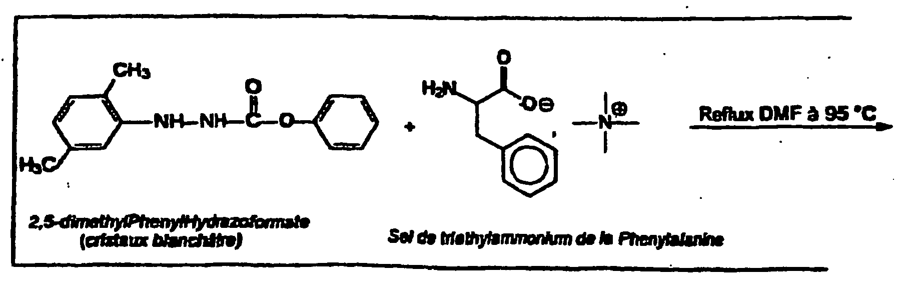

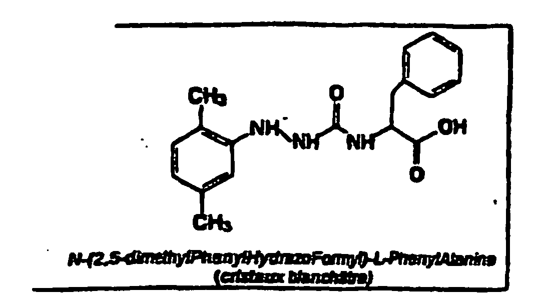

### 6^{ème} étape : Oxydation ; Passage de la forme "hydrazo" à la forme "azo"

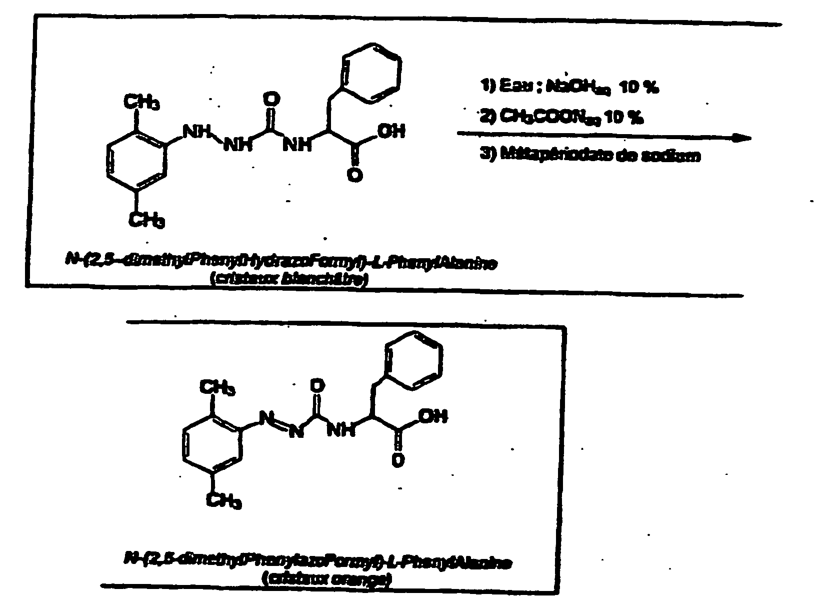

### 7^{ème} étape : Couplage avec l'Arginine

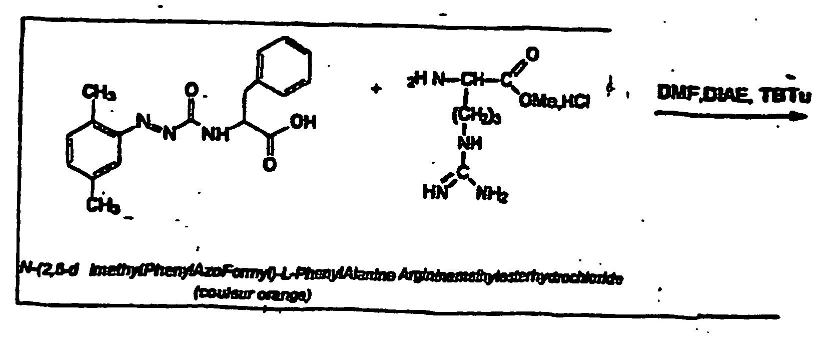

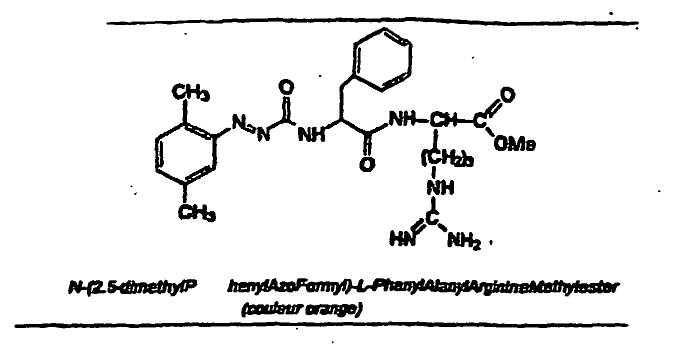

### 8^{ème} étape : Saponification

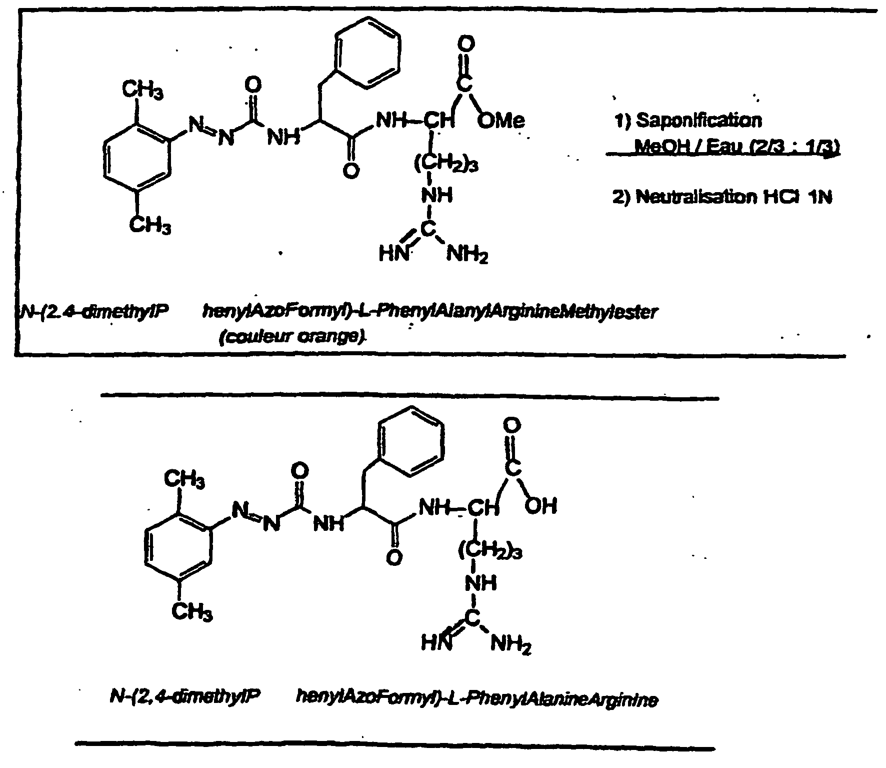

### E/ Synthèse du 4-methoxyphenylazoformylphenylalanyllysine:

### 1^{ère} étape: Réactif de Grignard

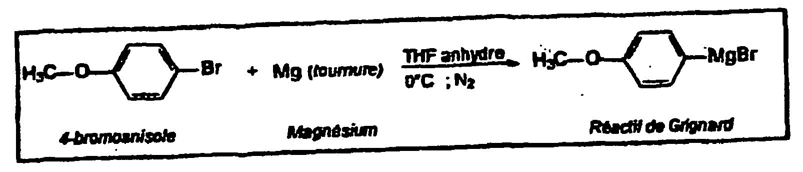

### 2^{ème} étape : Couplage avec le di-t-butylazodicarboxylate

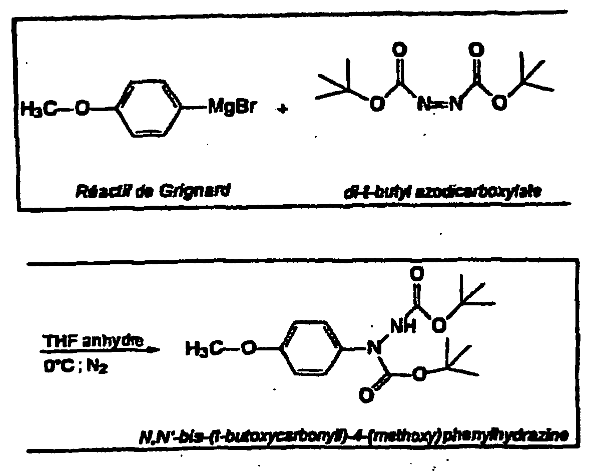

### 3^{ème} étape : Déprotection

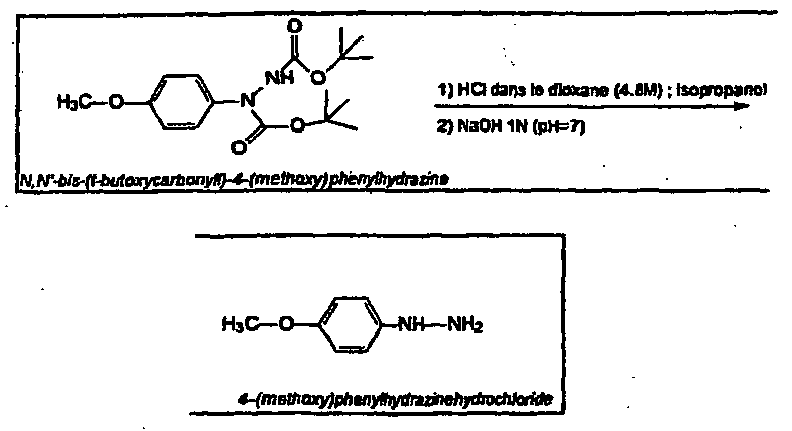

### 4^{ème} étape: Couplage avec le diphenylcarbonate

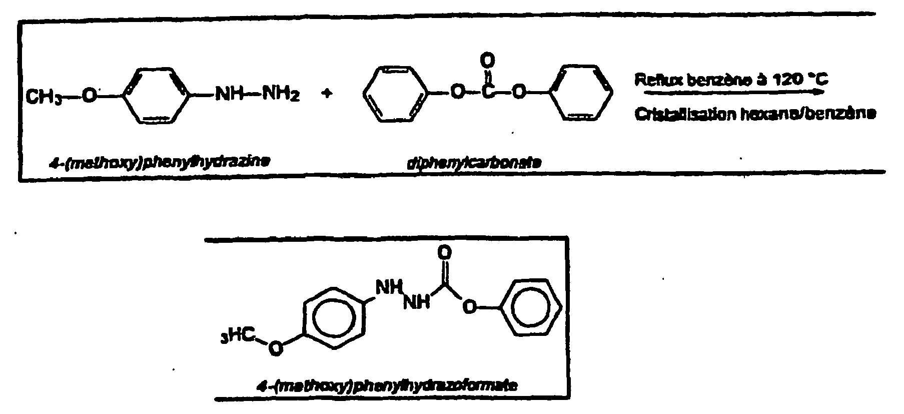

### 5^{ème} étape : Couplage avec la phénylalanine

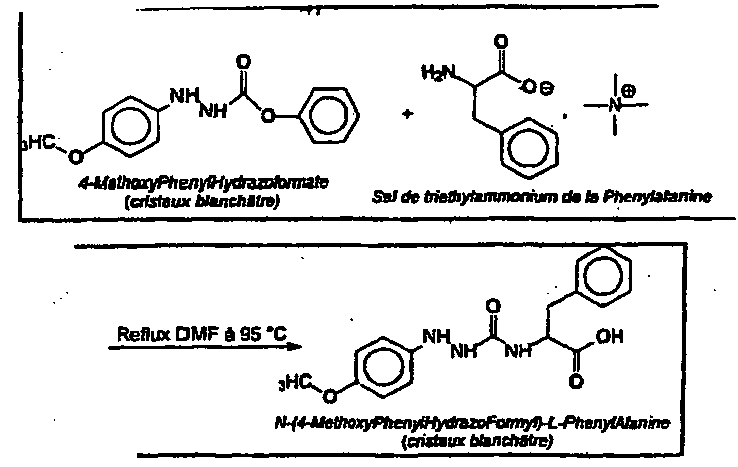

### 6^{ème} étape : Oxydation ; Passage de la forme "hydrazo" à la forme "azo"

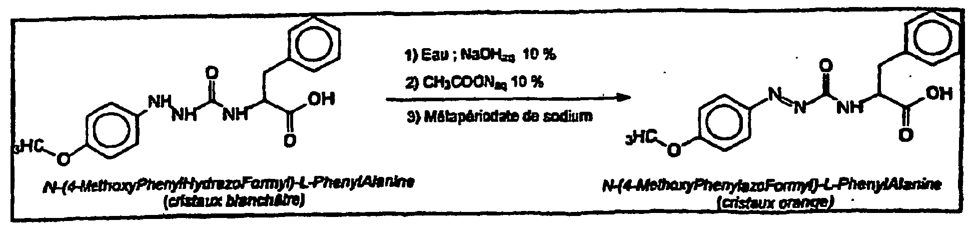

### 7^{ème} étape : Couplage avec la Lysine

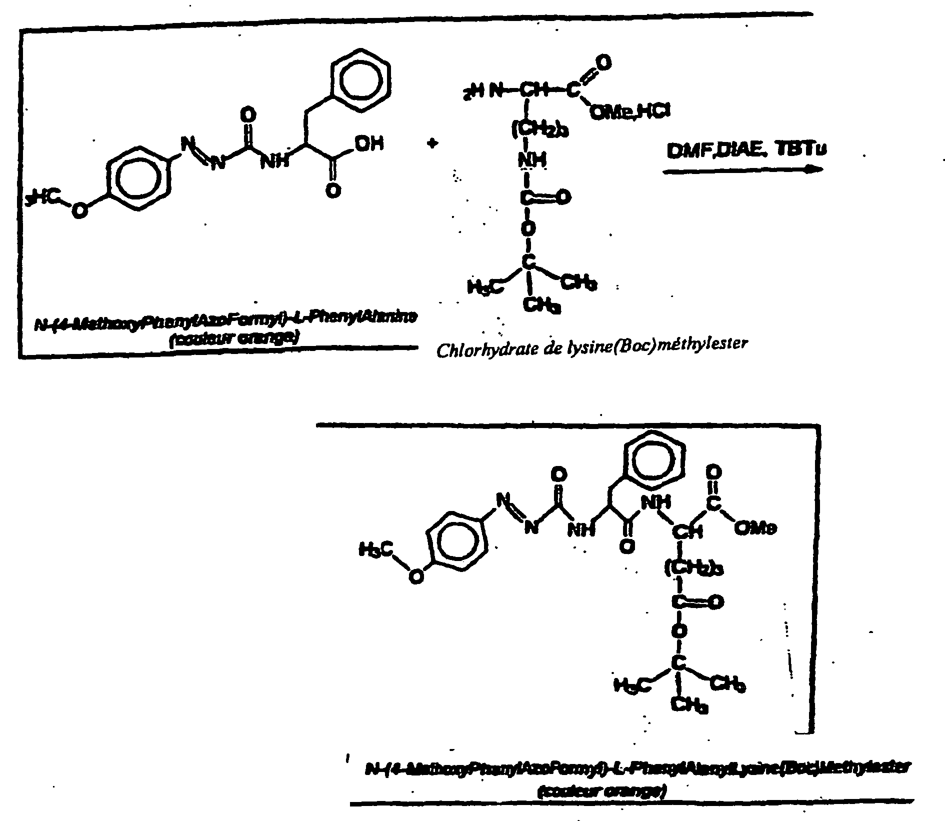

### 8^{ème} étape : Saponification

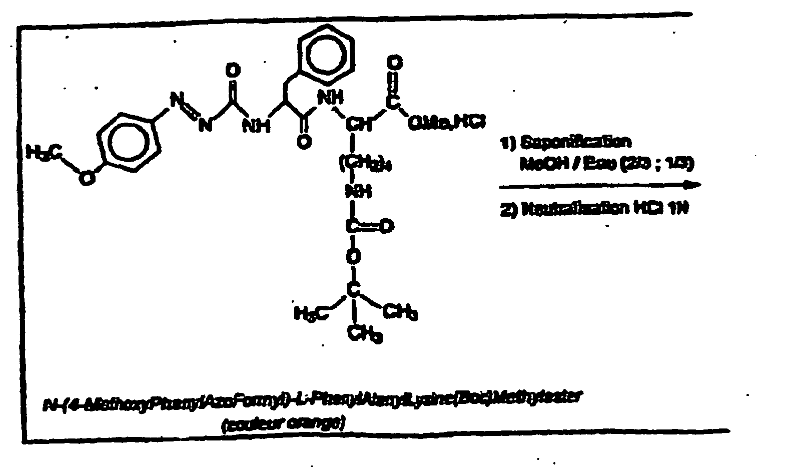

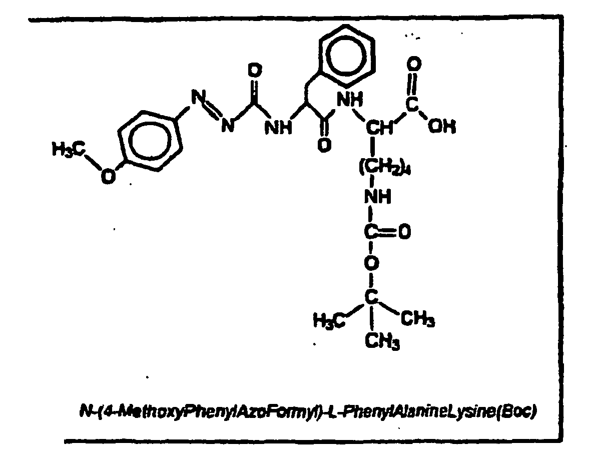

### 9^{ème} étape: Déprotection

Ce composé particulier nécessite une étape supplémentaire pour déprotéger la chaîne latérale de la lysine (ε-Soc :N-ε-tertiobutoxycarbonyl).

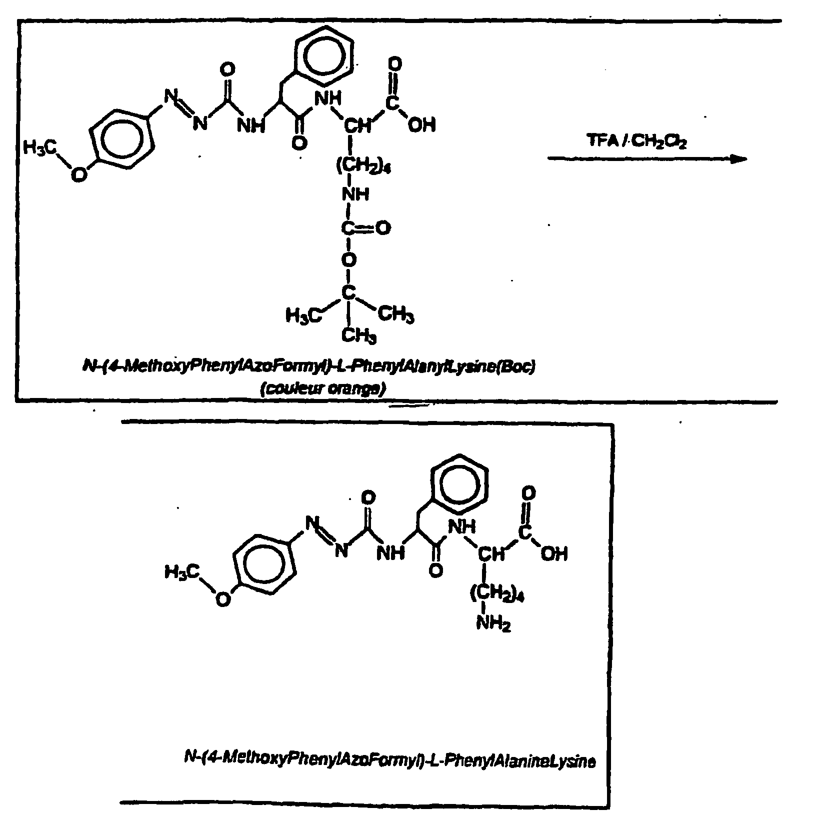

Mode opératoire : Reprendre 1 eq de N-(4-MethoxyPhenylAzoformyl)-L-Phenylalanyl-L-Lysine(ε-Boc) dans du dichloromethane (3mL/mmoL)dans un ballon. Placer sous agitation.Connecter une ampoule à brome contenant de l'acide trifluoroacétique (3mL/mmoL).Additionner lentement (goutte à goutte).Après 15 min, concentrer le milieu réactionnel et le purifier par chromatographie liquide haute performance.On obtient en finale une poudre orangée.

### F/ Synthèse du 3-chloro-p-tolylazoformylphenylalanylarctinine (Composé 6) :

### 1^{ère} étape : Réactif de Grignard

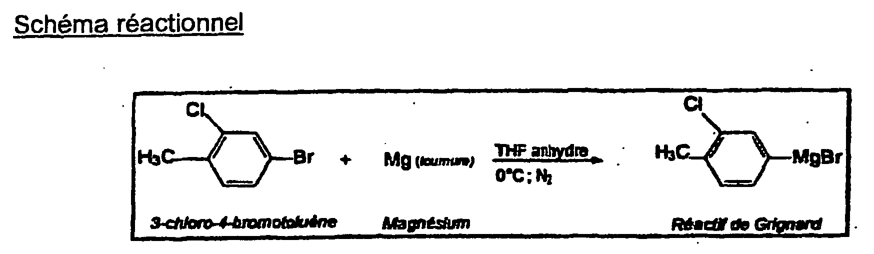

### 2^{ème} étape : Couplage avec le di-t-butylazodicarboxylate

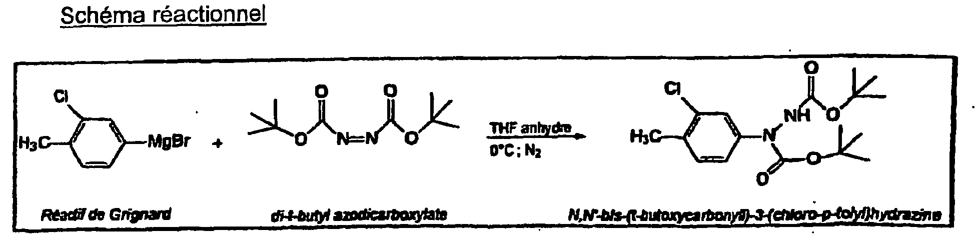

### 3^{ème} étape : Déprotection

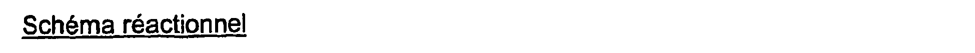

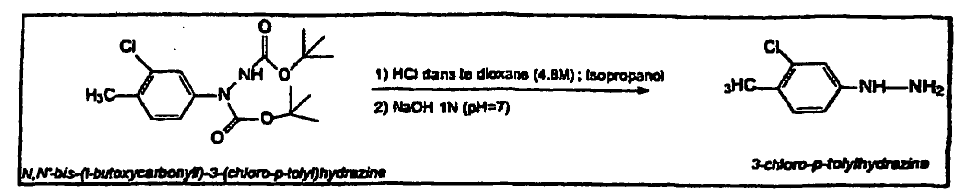

### 4^{ème} étape: Couplage avec le diphenylcarbonate

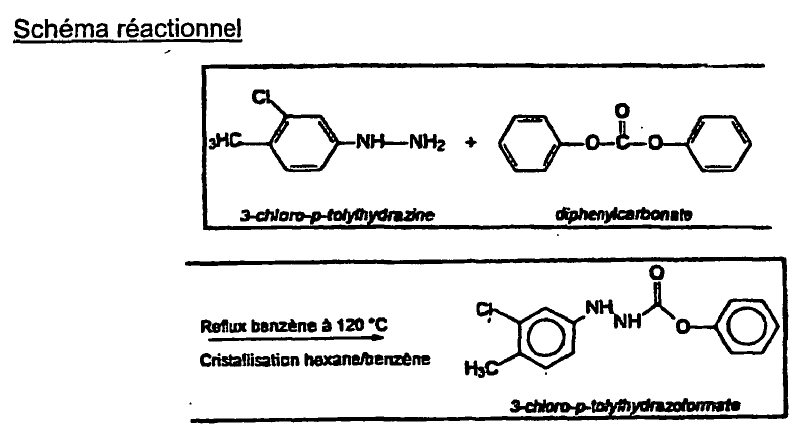

### 5^{ème} étape : Couplage avec la phénylalanine

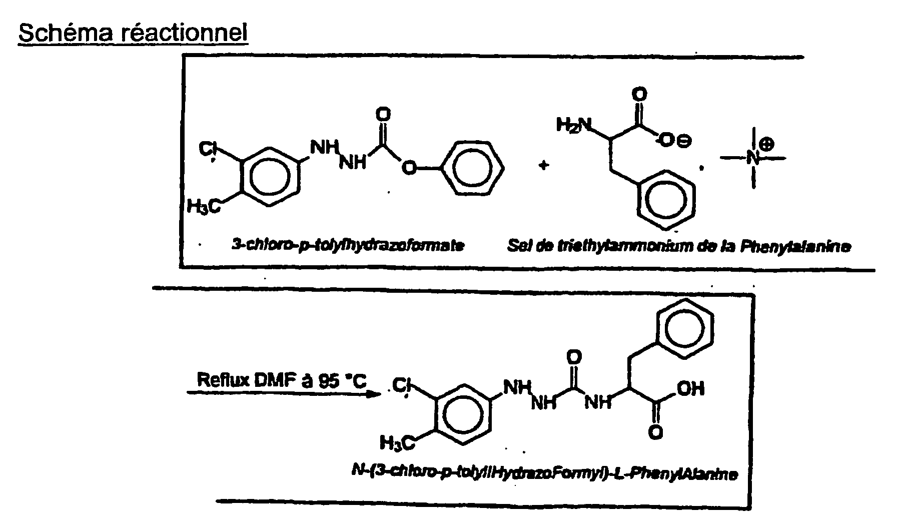

### 6^{ème} étape : Oxydation ; Passage de la forme "hydrazo" à la forme "azo"

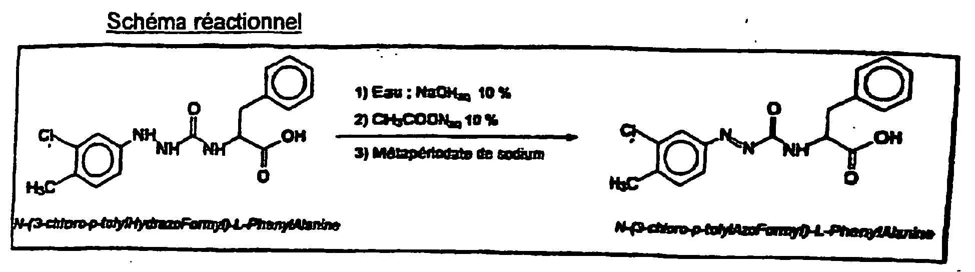

### 7^{ème} étape : Couplage avec l'Arginine

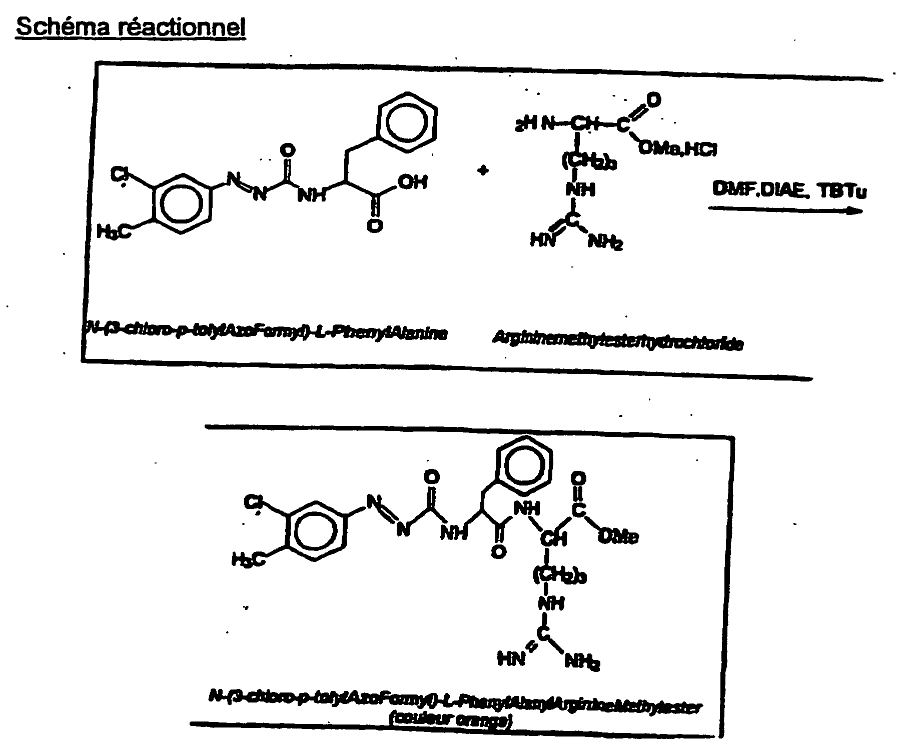

### 8^{ème} étape : Saponification

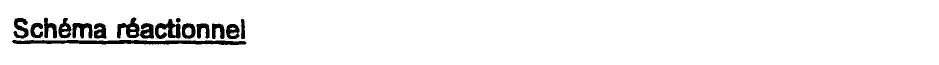

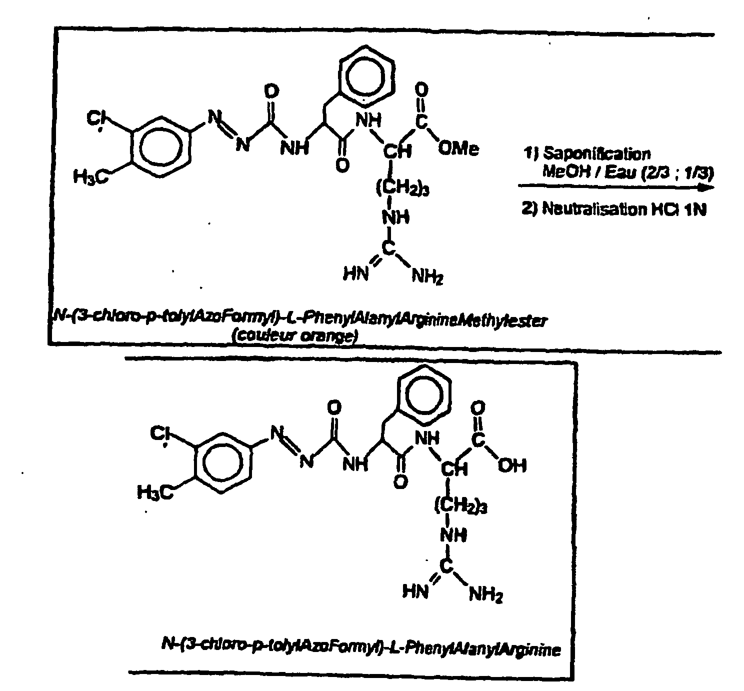

### G/ Synthèse du 3-chloro-p-tolylazoformyphenylalanylarginine (Composé 7) :

### 1^{ère} étape : Réactif de Grignard

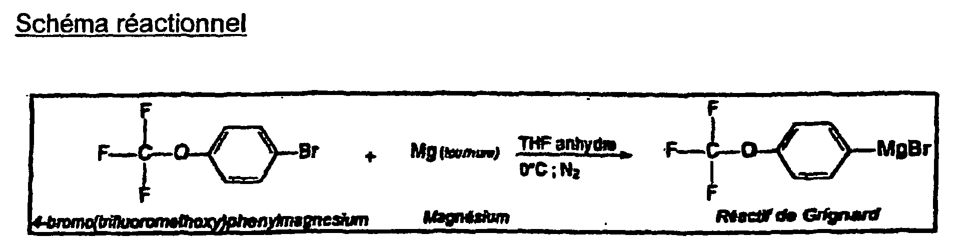

### 2^{ème} étape : Couplage avec le di-t-butylazodicarboxylate

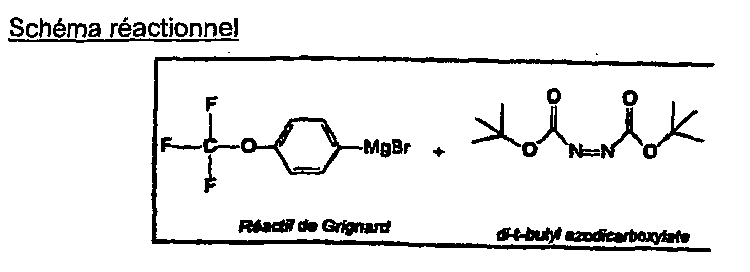

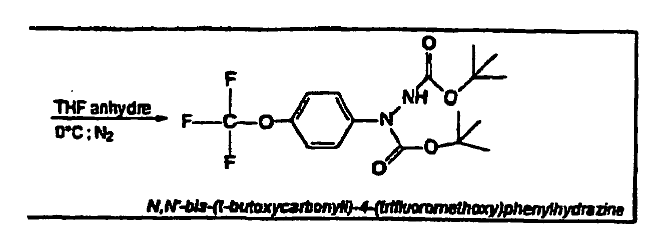

### 3^{ème} étape : Déprotection

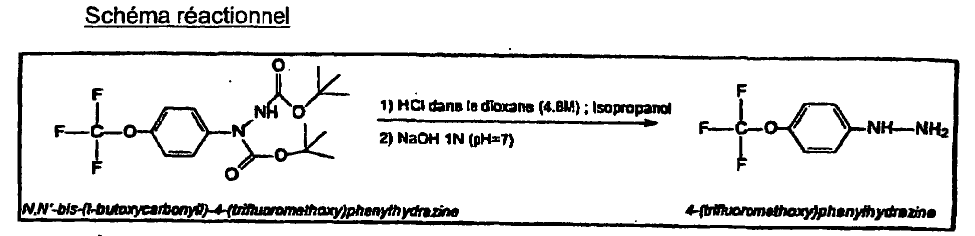

### 4^{ème} étape : Couplage avec le diphenylcarbonate

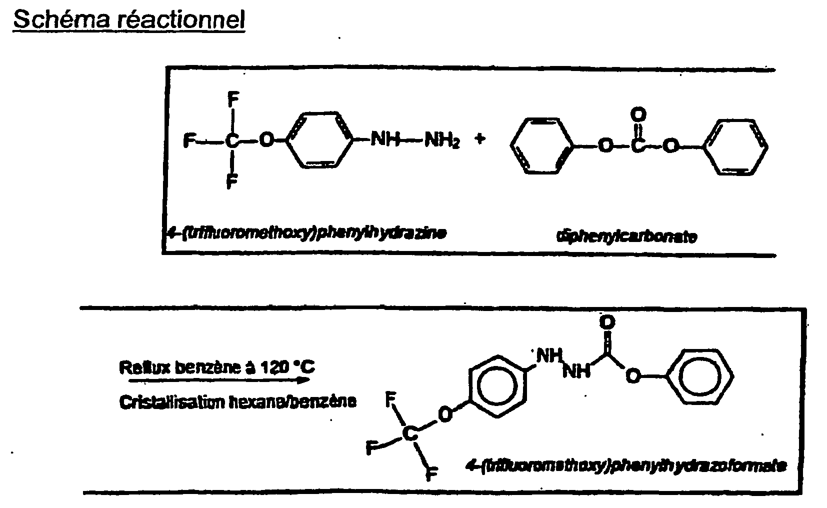

### 5^{ème} étape : Couplage avec la phénylalanine

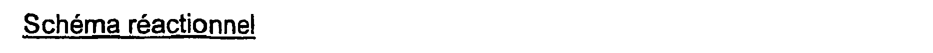

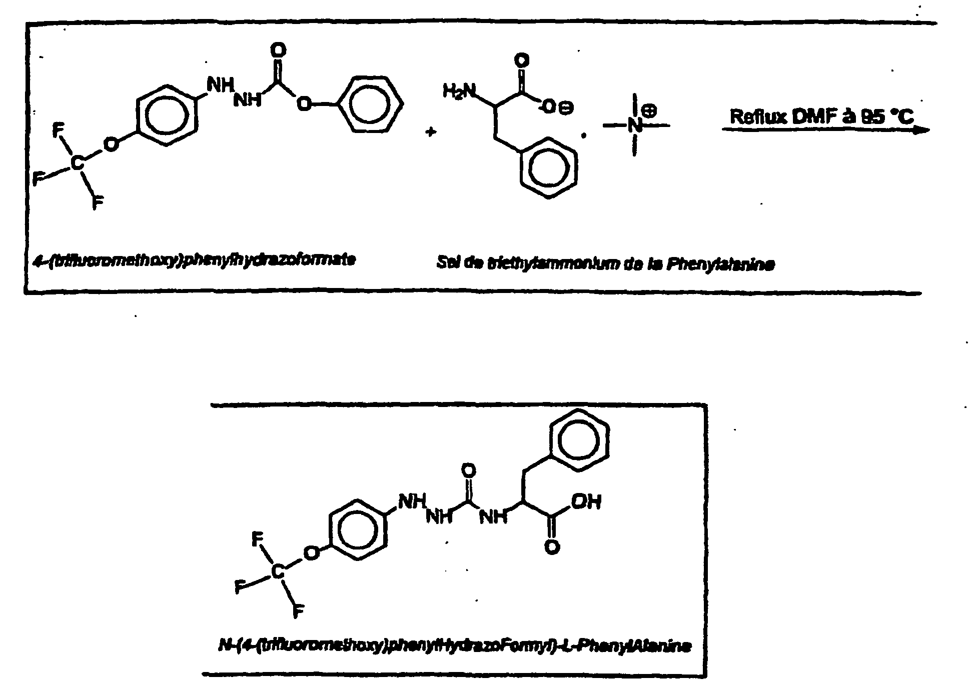

### 6^{ème} étape : Oxydation : Passage de la forme "hydrazo" à la forme "azo"

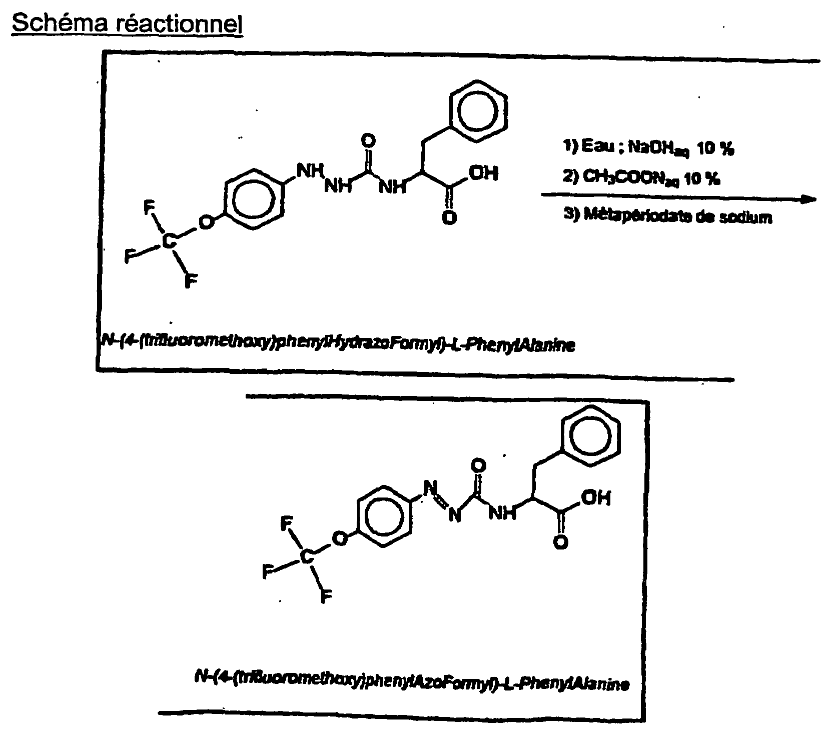

### 7^{ème} étape : Couplage avec l'Arginine

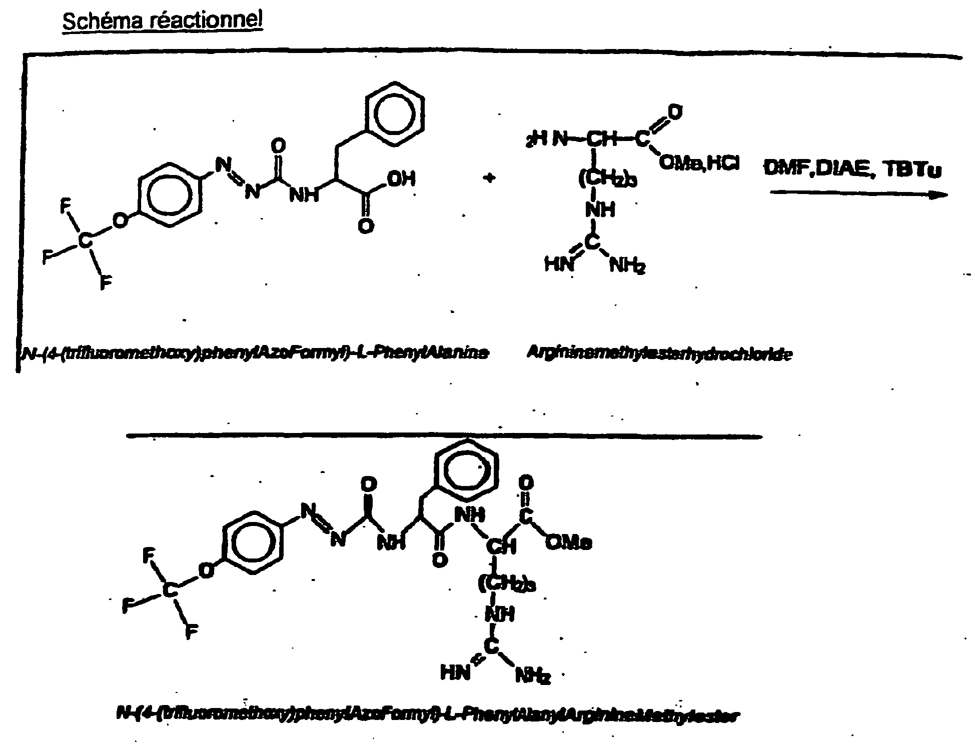

### 8^{ème} étape: Saponification

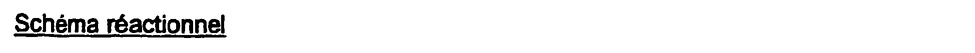

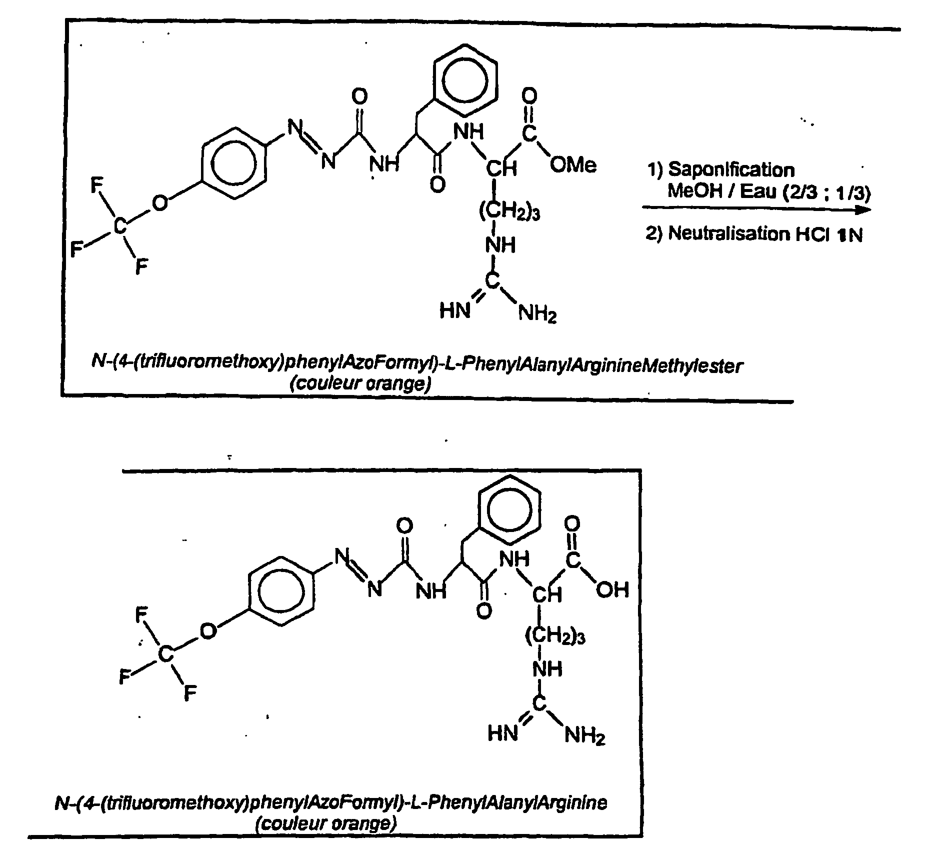

### H/ Synthèse du 4-Methylthiophenylazoformylphenylalanylarginine (Composé 8) :

### 1^{è Rre} étape : Réactif de Grignard

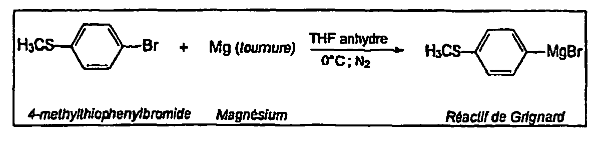

### 2^{ème} étape : Couplage avec le di-t-butylazodicarboxylate

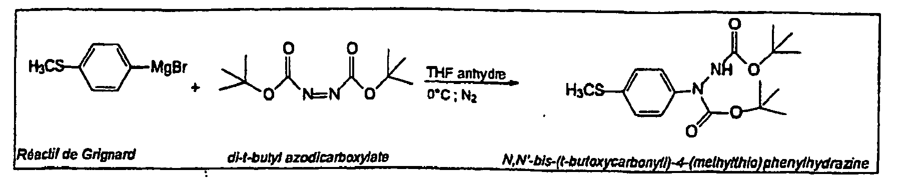

### 3^{ème} étape : Déprotection

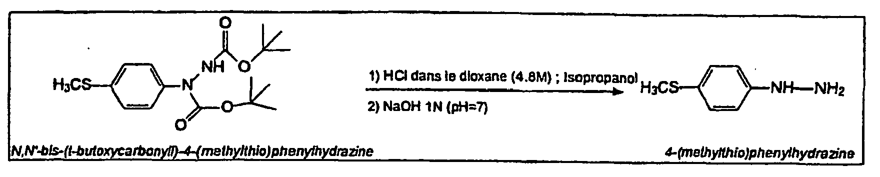

### 4^{ème} étape : Couplage avec le diphenylcarbonate

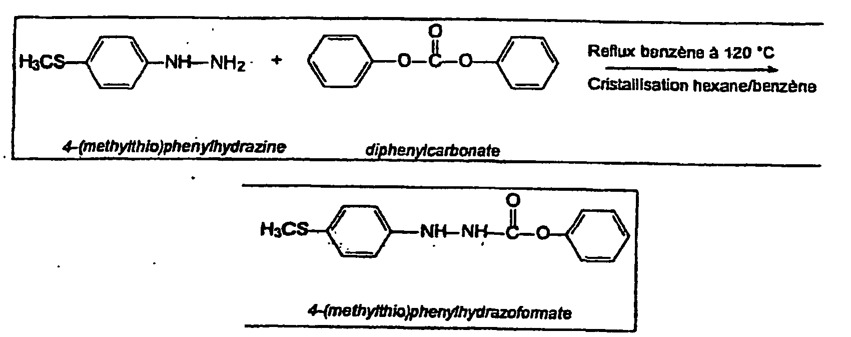

### 5^{ème} étape : Couplage avec la phénylalanine

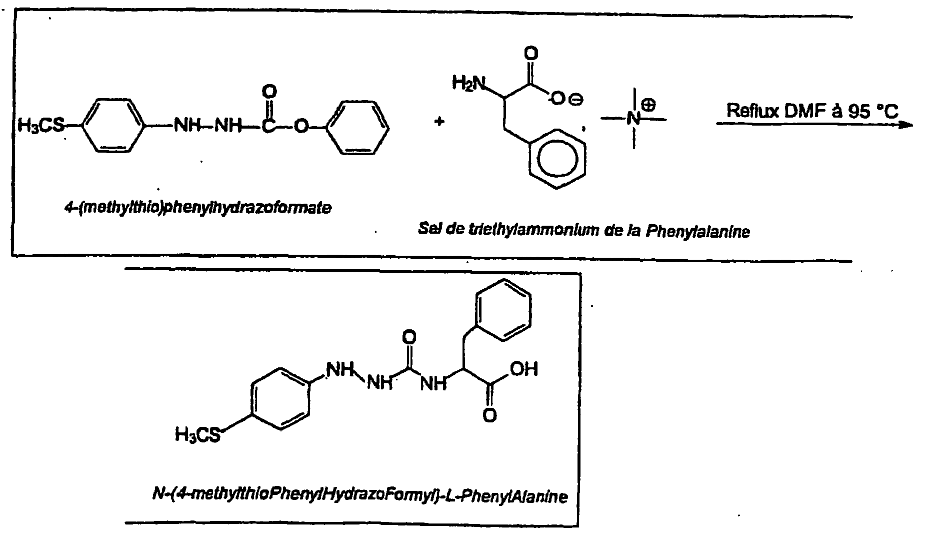

### 6^{éme} étape : Oxydation ; Passage de la forme "hydrazo" à la forme "azo"

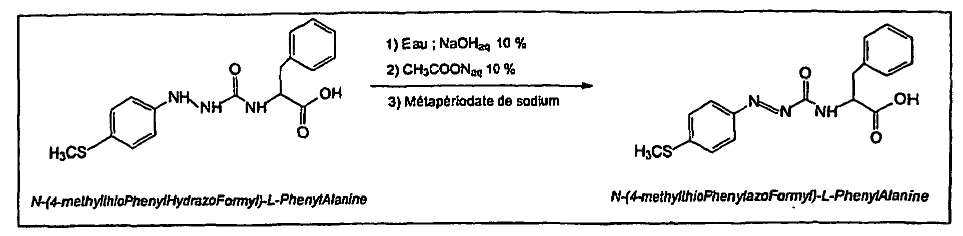

### 7^{ème} étape : Couplage avec l'Arginine

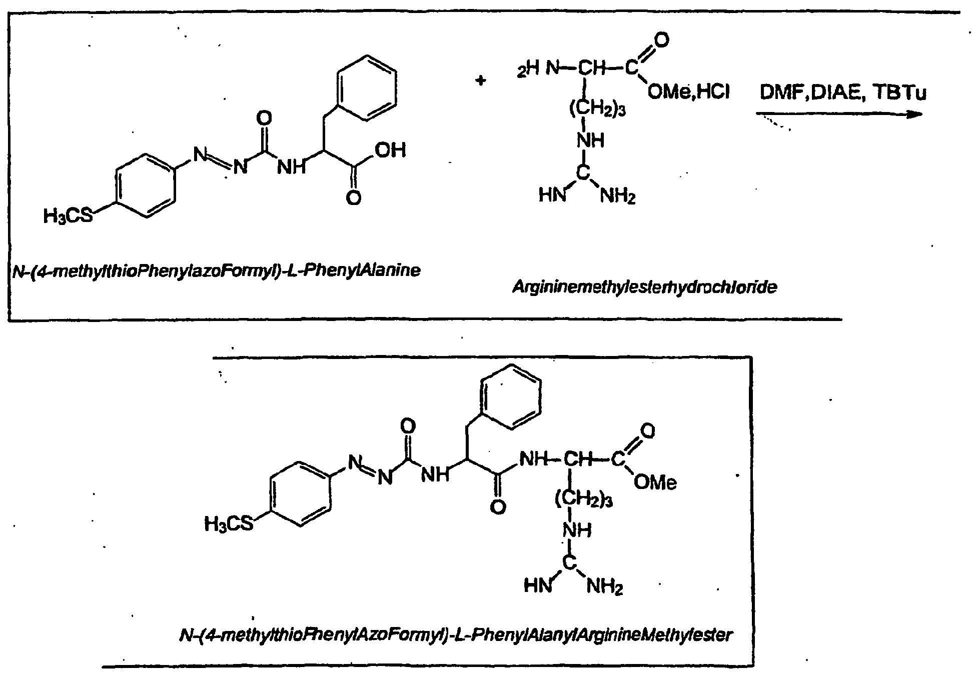

### 8^{ème} étape : Saponification

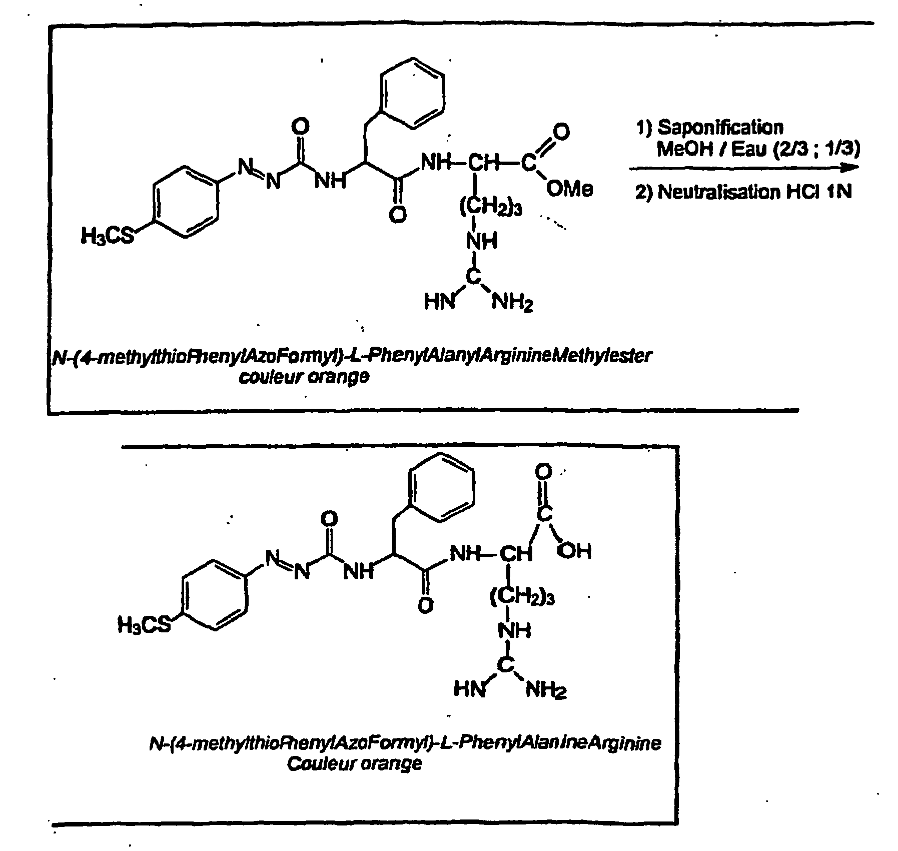

### I/ Synthèse du 4-Methylthiophenylazoformyltyrosylarginine (Composé 9) :

### 1^{ère} étape : Réactif de Grignard

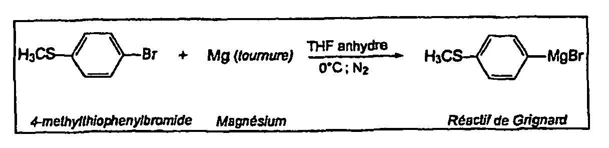

### 2^{ème} étape : Couplage avec le di-t-butylazodicarboxylate

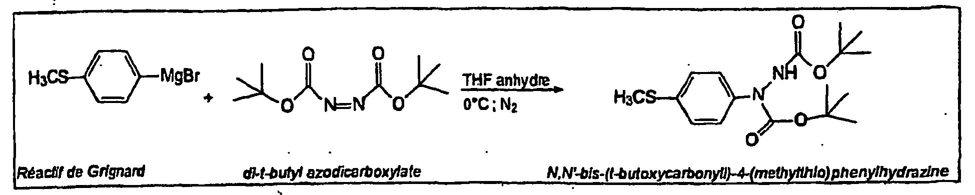

### 3^{ème} étape : Déprotection

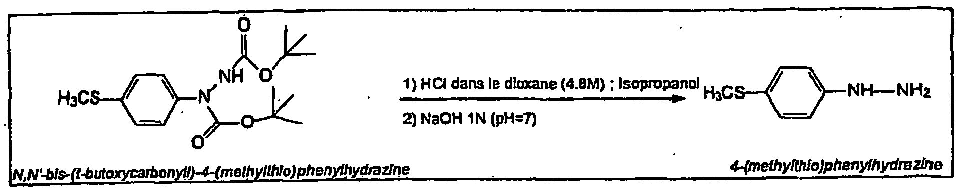

### 4^{ème} étape: Couplage avec le diphenylcarbonate

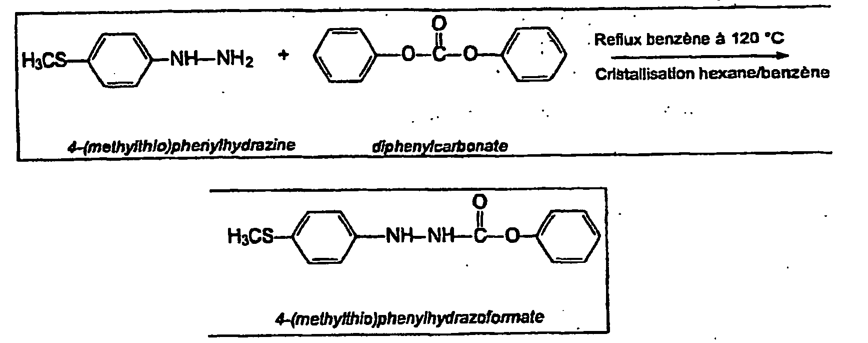

### 5^{ème} étape : Couplage avec la Tyrosine

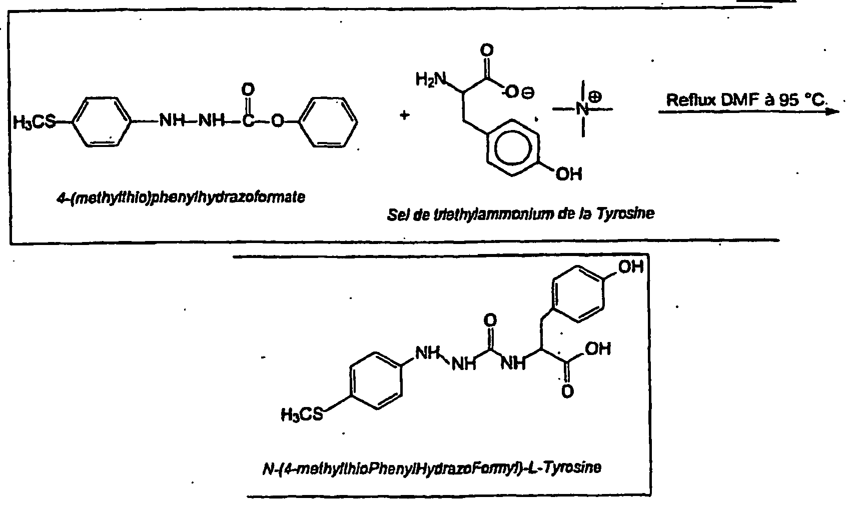

### 6^{ème} étape: Oxydation ; Passage de la forme "hydrazo" à la forme "azo"

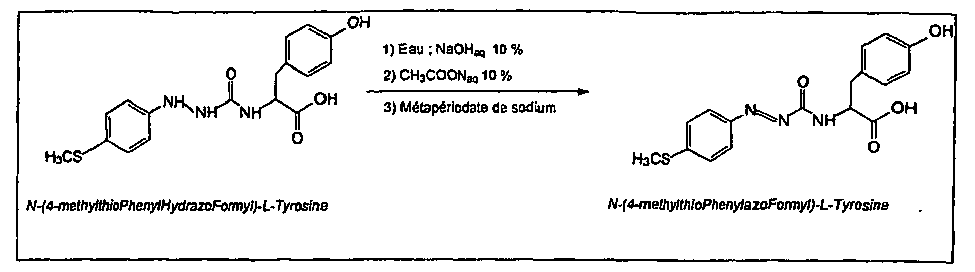

### 7^{ème} étape : Couplage avec l'Arginine

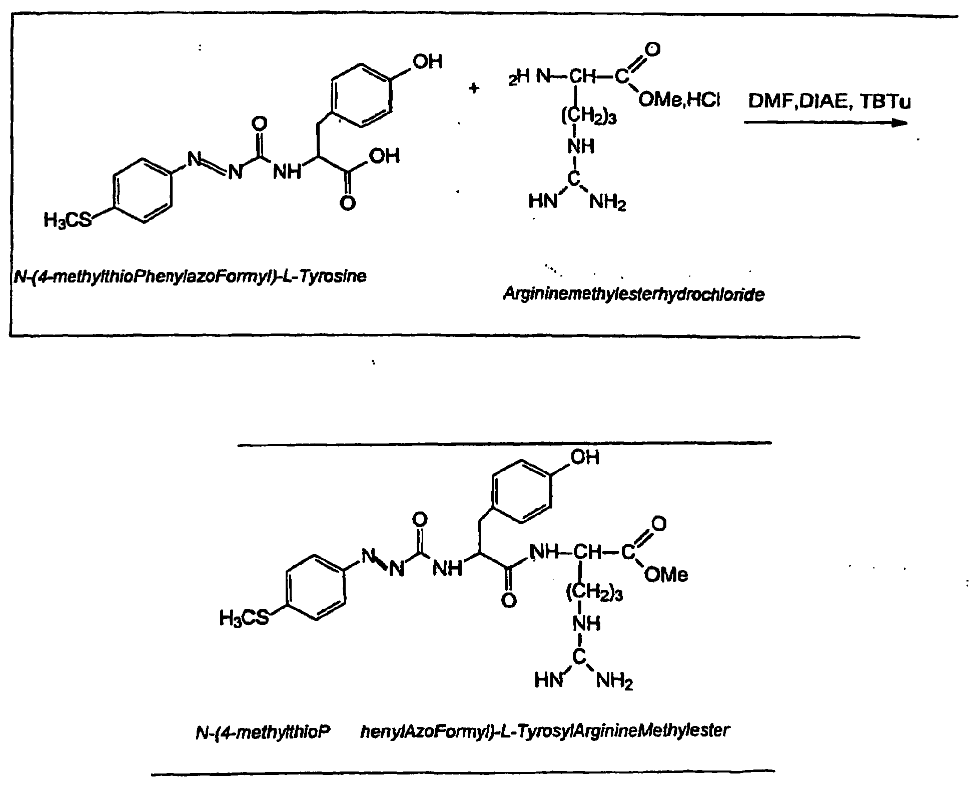

### 8^{ème} étape : Saponification

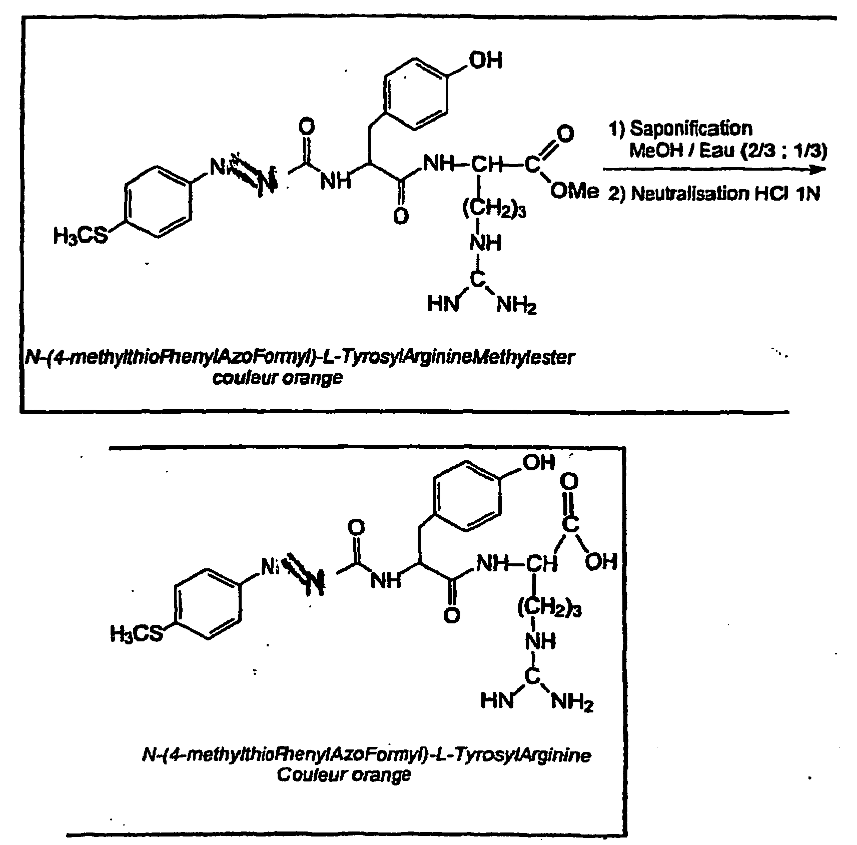

### EXEMPLE N° 2 :

### 1- Principe du dosage et principaux réactifs mis en oeuvre

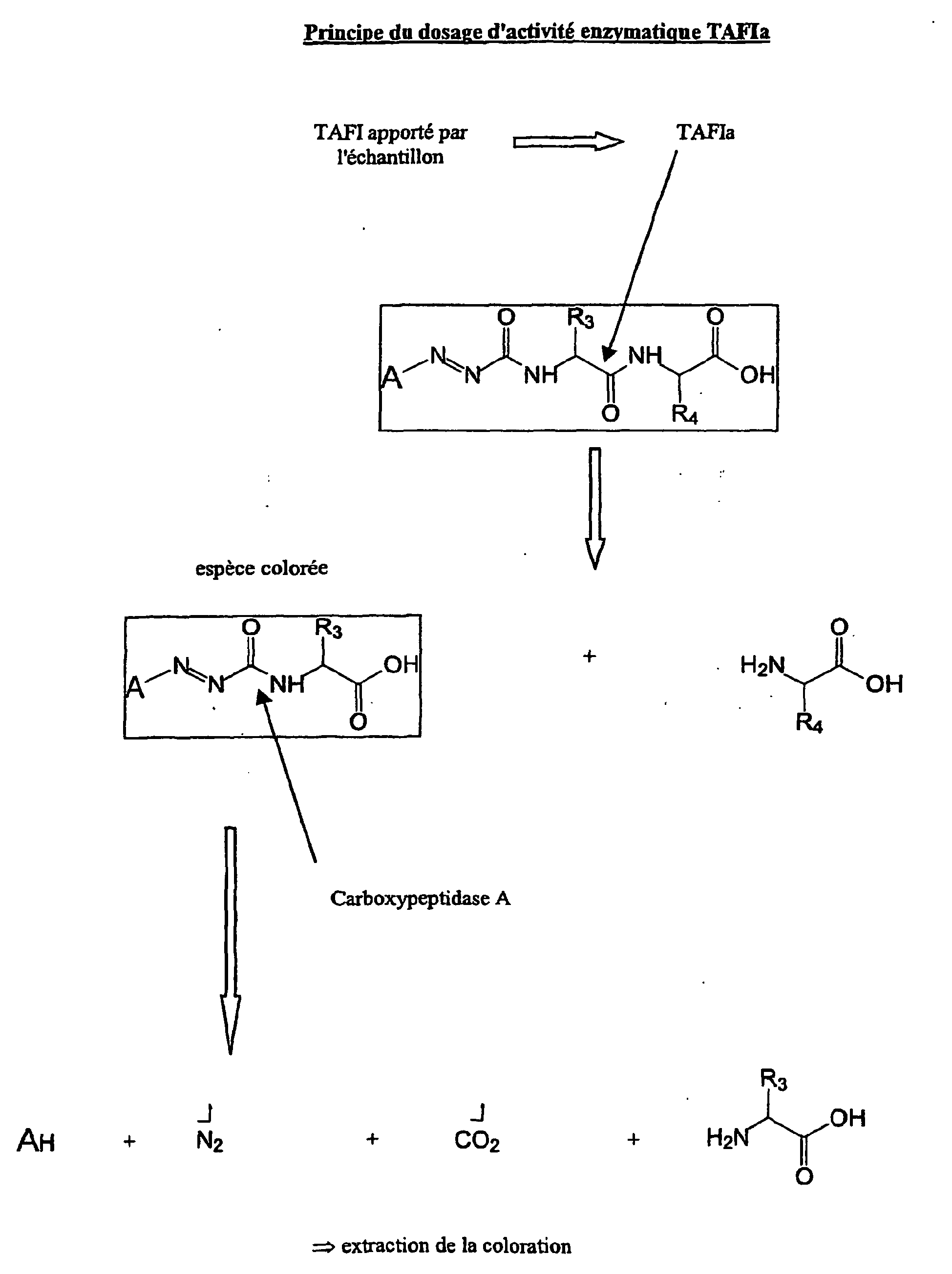

### 2. Exemple de protocole de dosage de l'activité du TAFI selon la méthode de l'invention.

### a) 1er mode opératoire : méthode thrombine - thrombomoduline

L'échantillon de plasma testé est divisé en deux aliquotes, l'un étant additionné de PIC (Calbiochem - Réf. 217359), l'autre de tampon hépès.
Les deux aliquotes sont ensuite traités de manière identique selon le principe suivant :

### Activation du TAFI

150 µl de plasma dilué au 1/20 en tampon hépès (ou TAFI purifié à 13 µg/ml en tampon hépès.
10 µl PIC ou H2O
5 minutes à température ambiante
150 µl tampon activateur de la coagulation
10 minutes à température ambiante
100 µl PPACK + 100 µl MxPAFFR (substrat) (5 mM)

### Test d'activité du TAFI

30 minutes à température ambiante
100 µl HCl 1M + 100 µl NaOH 1M

### Révélation

Dilution au 1/3 en tampon hépès
Lecture de la DO à 382 nm
25 µl Carboxypeptidase A
Lecture de la DO à 382 nm sur 1 minute

### Variantes possibles du premier mode opératoire:

- on peut effectuer le dosage à une température de 37° C
- le substrat peut être incorporé dès le départ, en même temps que le tampon activateur
- la lecture peut être faite par HPLC

### Concentrations finales préférées des différents produits:

- PPACK : H-D-Phe-Pro-Arg-chloromethylketone PM = 451 30 µM (Bachem - Réf. N. 1065).
- Péfabloc : il peut être utilisé à la place du PPACK : 0.1 mM (Pentapharm - Réf. 399.01).
- substrat (MxPAFFR) : 1 mM
- Carboxypeptidase A : origine pancréas bovin (Sigma - Réf. C 0386).
   Flacon de 5.1 ml à 5000 unités, 21 mg prot/ml, 47 unités/mg protéine
   La CPA a été filtrée avec préfiltre, filtre 5 µm et filtre 0.45 µm, pour cela on a ajouté ml H2O.
- PIC : utilisé à 7 µM
   1 mg de PCI peut inhiber environ 8 mg de TAFI (50 U/mg) à 50 % selon indications du fournisseur (Calbiochem).

La concentration de 30 µM de PPACK correspondrait à une concentration initiale de 150 µM. La concentration finale de 1 mM de substrat correspondrait à une concentration initiale de 5 mM. La concentration finale de 7 µM de CPI correspondrait à une concentration initiale de 0,38 mM.

Les inventeurs ont observé que les concentrations indiquées ci-dessus peuvent être modifiées pour utiliser une concentration finale en PPACK diminuée jusqu'à 4µM et une concentration finale en substrat diminuée.

### Tampon activateur :

- La voie d'activation préférée de la coagulation dans le cadre de la présente invention est la voie d'activation par le complexe thrombine/thrombomoduline. Dans ce cas, le tampon activateur utilisé comprend les constituants suivants :
   37 µl thrombomoduline de 0 à 80 nM et préférentiellement à 10 nM (Rabbit lung thrombomodulin - American Diagnostica - Réf. 237).
   6 µl thrombine de 0,2 à 10 NIH/ml et préférentiellement à 0,8 NIH/ml (Diagnostica Stago - Produit de recherche).
   750 µl chlorure de calcium de 0 à 80 nm et préférentiellement à 40 nM
   705 µl tampon hépès 9.4 mM pH = 7.6

   Les concentrations sont données pour un volume final de 1498 µl de tampon activateur.
   N.B. : Le tampon hépès contient : Hépès 20 mM, KCI 4 mM et BSA 1% d'autres essais ont été réalisés avec Hépès 20 mM et NaCl 150 mM.
- Comme cela l'a été indiqué dans la description, d'autres voies d'activation sont possibles dont notamment la voie d'activation par le facteur Xla.
   Dans ce cas un exemple de tampon activateur contient les constituants suivants :
   5 µl thrombomoduline à 30 U/ml
   30 µl Facteur Xla pur (Calbiochem - Réf. 233483).
   88 µl tampon hépès pH = 7.4 (Hépès 20 mM et NaCl 150 mM).

Les concentrations sont données à titre d'exemple et peuvent être réajustées par l'homme du métier.

### b) 2ème mode opératoire: méthode génération de thrombine

Une autre voie d'activation de la coagulation fait intervenir la thrombine et requiert la mise en oeuvre d'un activateur de la phase contact.

| | Génération de thrombine |
|---|---|
| | 100µl échantillon ±10 µl CPI |
| | |
| | 100µl tampon activateur composé de |
| 1) Activation du TAFI | - 30 µl thrombomoduline à 30U/ml, |
| | - 165 µl d'activateur de la phase contact, |
| | - -800 µl de CaCl₂ à 20 mM. |
| | |
| | Incubation 10 min. TA |
| | |
| | 100µl PPACK |
| | 100µl substrat |
| | |
| | incubation 30 min. TA |
| 2) test d'activité TAFIa | |
| | 100µl HCl 1N |
| | 100µl NaOH 1N |
| | dilution de |
| | 250µl du milieu réactionnel par |
| | |
| | 500µl de tampon + 50µl H₂SO₄ |
| | lecture DO₁ |
| | |
| 3) révélation | ou |
| | |
| | 250µl tampon+250µl CPa |
| | incubation 5 minutes TA |
| | 50µl H₂SO₄ |
| | lecture DO₂ 405nm |

### Exemple n° 3 :

### Etablissement d'une gamme d'étalonnage en méthode thrombine/thrombomoduline.

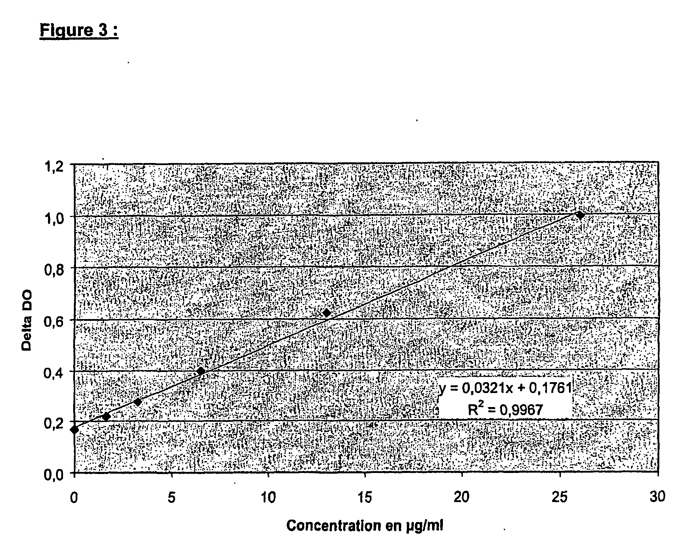

La figure 3 ci-après représente une courbe d'étalonnage établie à partir d'un plasma déficient en TAFI surchargé avec du TAFI purifié pour obtenir une gamme de concentration en TAFI allant de 0 à 26 µg/ml.
Le composé de formule I utilisé est le MxPAAFR à une concentration de 5 mM.
Les Δ DO mesurés selon la méthode de l'invention sont reportés dans le tableau III suivant :

**TABLEAU III :**

| | | | | | | |
|---|---|---|---|---|---|---|
| Concentration en TAFIa (µg/ml) | 0 | 1.63 | 3.25 | 6.5 | 13 | 26 |
| Δ DO mesuré | 0.168 | 0.217 | 0.273 | 0.399 | 0.624 | 0.995 |

Comme cela apparaît sur la figure 3, la courbe d'étalonnage est linéaire pour les gammes de concentrations étudiée. Celle-ci couvre largement la zone de normalité, le TAFI étant présent dans l'organisme à des concentrations allant de 5 à 15 µg/ml.
La méthode de l'invention permet donc de déceller aussi bien des déficits en TAFI que des taux anormalement élevés.

### Exemple n° 4 :

### Résultats obtenus sur différents types de plasmas.

La méthode de l'invention a été appliquée sur différents types de plasmas selon le protocole décrit dans l'exemple n° 2.

### Plasmas frais, plasmas congelés :

On peut effectuer le dosage sur plasma frais ou plasmas congelés mais on observe une diminution de DO entre les deux. Celle-ci résulte d'une légère dégradation du TAFI lors de la décongélation (la protéine se dégrade).

| Plasmas frais | Plasmas congelés |
|---|---|
| 9.4 µg/ml | 6.0 µg/ml |
| 11.2 µg/ml | 6.5 µg/ml |
| 10.7 µg/ml | 5.6 µg/ml |

### Plasma congelé, plasma lyophilisé :

Sur un plasma lyophilisé, le dosage de TAFI est possible et similaire à un plasma congelé.

| | Plasma congelé | Plasma lyophilisé |
|---|---|---|
| Δ DO : | 0.302 | 0.354 |
| | | |

| *Plasma d'origine diverse congelés :* | | |
|---|---|---|
| Thrombolytique | 2.9 µg/ml | |
| CIVD | 2.8 µg/ml | |
| Cirhhose | 1.4 µg/ml | |
| Maternité | 7.1 µg/ml | |
| Hyperfibrinogénémie | 4.6 µg/ml | |
| HNF | 6.1 µg/ml | (héparines non fractionnées) |
| AVK | 5.8 - 8.2 - 6.5 - 7.1 µg/ml | (anti-vitamine K). |

### Exemple n° 5 :

### Dosage réalisés avec différents substrats de l'invention.

La méthode de l'invention a été appliquée sur une solution contenant du TAFI purifié, avec différents composés décrits dans l'Exemple n° 1.

Le test est réalisé sur une solution à 6,5 µg/ml de TAFI purifié dans du tampon hépès. Les résultats sont reportés sur le tableau IV ci-après.

**TABLEAU IV :**

| **Composés** | **Lecture** | **DO de départ** | **DO finale** | **Delta DO** |
|---|---|---|---|---|
| **2,3 DMPAFFR** 5 mM | 350 nm | 0.318 | 0.089 | 0.229 |
| **2,4 DMPAFFR** 5 mM | 330 nm | 1.004 | 0.087 | 0.917 |
| **2,5 DMPAFFR 5** mM | 320 nm | 0.973 | 0.131 | 0.842 |
| **AAFFK** 5 mM | 320 nm | 0.973 | 0.131 | 0.842 |

### EXEMPLE N° 6 :

### Spécificité du TAFIa pour les composés de formule (I) de l'invention :

L'exemple ci-après vise à démontrer la nécessité d'utiliser la méthode et les composés de formule (I) de l'invention pour doser spécifiquement l'activité des CPN ou U, en particulier le TAFI, par rapport à d'autres carboxypeptidases basiques.
1. Le dosage est dans ce cas réalisé avec un substrat de la famille de composés décrites par Mock dans (14). Ce substrat est constitué d'un seul acide aminé porteur du radical chromophore anizylazoformyl (portion CH₃OC₆H₄-N = N-CO-). L'acide aminé choisi est l'arginine, un acide aminé basique normalement hydrolysé par les carboxypeptidases B (1).
Ce substrat est dénommé ci-après AAFR (AnizylAzoFormyArginine) ou MxAFR (4-MethoxyphenylAzoformylarginine).
Ce substrat (0,25 mM) est mis en présence soit d'un échantillon de TAFI plasmatique, soit d'une solution de carboxypeptidase B pancréatique porcine (Sigma - Réf. C9584) à 5,2 mol/l.
La diminution de coloration de chacun des deux échantillons est suivie au spectrophotomètre. Chacun des échantillons est traité selon le protocole suivant :
a) *échantillon de départ :*
   - TAFI plasmatique (présent dans un pool normal de plasmas - dilué au 1/20) ou
   - Carboxypeptidase B pancréatique porcine 5,2 mol/l (dans tampon hépès)
b) activation avec complexe thrombine-thrombomoduline-CaCl2 (voir exemple 2) pour l'échantillon de TAFI plasmatique.
c) addition de AAFR à 5 mM.
d) lecture DO à 382 nm.
**Résultats :**

| à 382 nm | **DO de départ** | **DO finale** | **Delta DO** |
|---|---|---|---|
| CPB porcine | 1.054 | 0.445 | 0.608 |
| TAFIa du plasma | 1.730 | 1.681 | 0.049 |

**Conclusion :**
Le TAFI activé n'hydrolyse pas le AAFR contrairement à la CPB pancréatique.

2. La méthode de l'invention est ensuite appliquée sur une solution de TAFI purifiée, soit avec un substrat de formule (I) (MxPAFFK), soit avec le MxAFR pour vérifier que le TAFIa hydrolyse néanmoins un composé de formule (I). L'activation de la coagulation est déclenchée par le complexe thrombine-thrombomoduline-CaCl2 (voir protocole exemple 2).
La diminution de coloration de chacun des deux échantillons est suivie au spectrophotomètre.
a) échantillons de départ : solution de TAFI purifié à18 µg/ml (dilué dans tampon hépès).
b) activation de la coagulation.
c) addition de MxAFR 27 mM ou MxPAFFK (témoin) concentration du MxAFFK.
d) addition de CPA dans l'échantillon contenant le MxPAFFK.
e) lecture DO à 382 nm.
**Résultats :**

| | **DO de départ** | **DO finale** | **Delta DO** |
|---|---|---|---|
| TAFIa + MxAFR | 1.356 | 1.300 | 0.056 |
| TAFIa + MxPAFFK | 1.397 | 0.849 | 0.548 |

**Conclusion :**
Le TAFI activé n'hydrolyse pas le MxAFR alors qu'il est actif sur le MxPAFFK.

### EXEMPLE N° 7 : METHODOLOGIE GENERATION DE THROMBINE

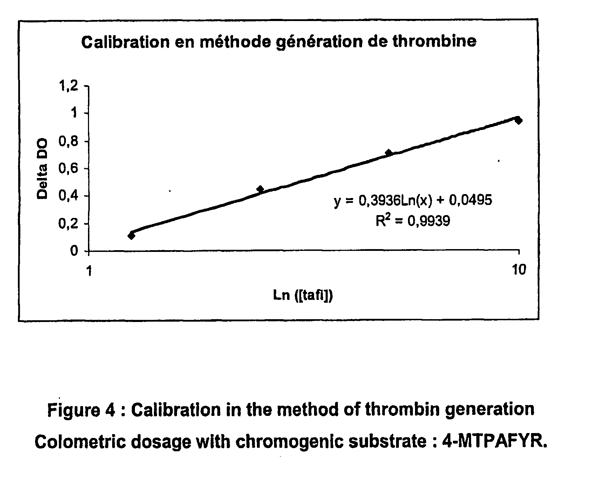

Une gamme est réalisée comme décrit dans l'exemple 2 le mode opératoire b) par dilution d'un plasma pool. (figure 4)

| plasma | delta DO | concentration µg/ml |
|---|---|---|
| Système contrôle N | 0.848 | 7.69 |
| Système contrôle P | 0.670 | 4.90 |
| plasma normal | 0.928 | 9.39 |

Cette méthodologie permet de discriminer les plasmas en fonction de leur capacité à générer la thrombine. Elle est un meilleur reflet de l'état d'hypercoagulabilité du patient.

### BIBLIOGRAPHIE :

1. BOUMA. B.N. et al : "Thrombin-Activatable Fibrinolysis Inhibitor (TAFI, Plasma Procarboxypeptidase B, Procarboxypeptidase R, Procarboxypeptidase U)". Thromb. Research, 101:329-354, 2001.
2. BAJZAR L. : "Purification and Characterization of TAFI, a Thrombin-activable Fibrinolysis Inhibitor". J. of Biol. Chem., 270, 24:14477-14484, 1995.
3. JUHAN-VAGUE 1., ALESSI M.-C. : "TAFI : lien moléculaire entre les processus de coagulation et de fibrinolyse". Sang Thrombose Veineuse, 5, 10:314-6, 1998.
4. SCHATTEMAN K. et al.: "Carboxypeptidase U at the interface between coagulation and fibrinolysis". Clin. Appl. Thrombosis/Hemostasis, 7(2):93-101,2001.
5. WOLF M. et al. : "The kinetics of carboxypeptidase B activity". J. of Biol. Chem., 237, n° 10, Octobre 1962.
6. SUZUKI S. et al. : "Spectrophotometric determination of glycine with 2,4,6-Trichloro-s-Triazine". Analytical Chemistry, Vol. 42, N° 1, January 1970.
7. LORENTZ K. et al. : "Determination of carboxypeptidase B in duodenal contents". Clinica Chimica Acta, 37:515-517, 1972.
8. PLUMMER Th. H. et al. : "An improved spectrophotometric assay for human plasma carboxypeptidase N". Analytical Biochemistry, 108:348-353, 1980.
9. FISCHER G.H. et al. : "Synthetic inhibitors of carboxypeptidase N". Adv. Experimental Med. Biol., 198, Part A, 405-410, 1986.
10. SARUTA H. et al. "Colorimetric determination of carboxypeptidase A activity in serum". Clin. Chem. 32:5, 748-751, 1986.
11. NAM-JOO HONG et al. : "Development of substrate for carboxypeptidase B by employing Thiaarginine peptides". Bull Korean Chem. Soc., Vol. 19, N° 2, 189-93, 1998.
12. MOCK W. L. et al. : "Arazoformyl Dipeptide Substrates for Thermolysin. Confirmation of a Reverse Protonation Catalytic Mechanism". Biochemistry, 35: 7369-7377, 1996.
13. MOCK W. L. et al. : "Arazoformyl Peptide Surrogates as Spectrophotometric Kinetic Assay Substrates for Carboxypeptidase A". Analytical Biochemistry, 239:218-222, 1996.
14. MOCK W. L. et al. : "Catalytic activity of carboxypeptidase B and of carboxypeptidase Y with anisylazoformyl substrates". Bioorganic & Medicinal Chemistry Letters, 9:187-192, 1999.
15. HATTON M.W. : "Studies on the coagulant enzyme from Agkistrodon rhodostoma venom. Isolation and some properties of the enzyme". Biochem. J., 131(4):799-807, 1973.
16. STOCKER K. et al. : "The coagulant enzyme from Bothrops atrox venom (batroxobin)". Methods Enzymol. 45:214-223, 1976.
17. DENSON K.W.E. : "Clot-inducing substances prevent in such venoms with particular reference to Echis carinatus venom". Thromb. Res., 8:351-360, 1976.
18. ROSING J. et al. : "Structural and functional properties of snake venom prothrombin activators". Toxicon, 30(12):1515-1527, 1992.

## Revendications

1. **Composé de formule (I) suivante :** 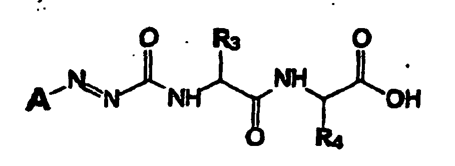 dans laquelle : 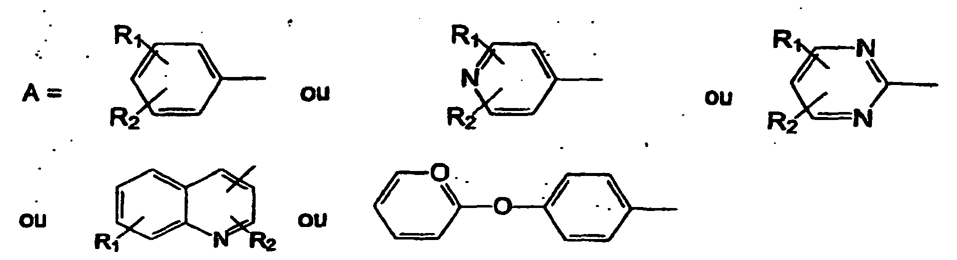
- R1, R2 = H, -CH₃, -CH(CH₃)₂, -OCH₃, -Cl, -CF₃, -OCF₃, -SCH₃
- R₃ = la chaîne latérale d'un acide aminé hydrolysable par une carboxypeptidase A.
- R₄ = la chaîne latérale d'un acide aminé basique.

2. Composé selon la revendication 1 de formule (I) suivante : 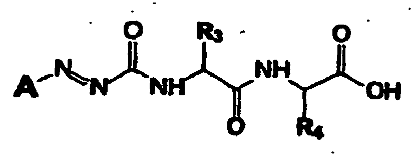 dans laquelle : 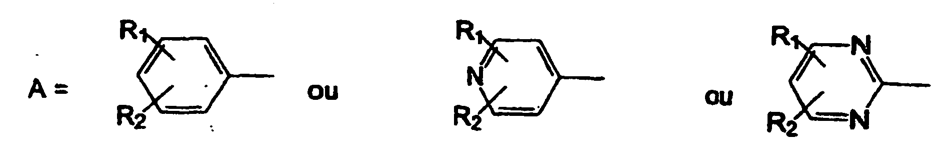 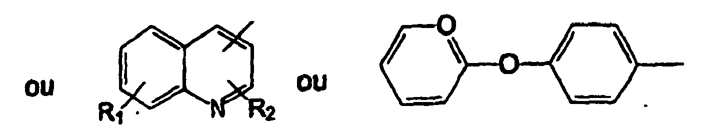
- R1, R2 = H, -CH₃, -CH(CH₃)₂, -OCH₃, -Cl, -CF₃, -OCF₃, -SCH₃
- R₃ = la chaîne latérale d'un acide aminé hydrophobe.
- R₄ = la chaîne latérale de l'arginine ou lysine.

3. Composé selon la revendication 1 ou 2, **caractérisé en ce que** R1=H et R2= -S-CH₃.

4. Composé selon la revendication 1 ou la revendication 3, **caractérisé en ce que** R₃ est choisi parmi les chaînes latérales des acides aminés suivants :
- la tyrosine,
- la phenylalanine,
- l'alanine,
- la valine,
- la leucine,
- l'isoleucine,
- la phénylglycine.

5. Composé selon la revendication 1 ou la revendication 3, **caractérisé en ce que** R₃ représente la chaîne latérale de la phenylalanine.

6. Composé selon la revendication 1 ou la revendication 3, **caractérisé en ce que** R₃ représente la chaîne latérale de la phénylalanine ou de la tyrosine et R₄ celle de l'arginine ou de la lysine.

7. Composé selon la revendication 1 ou la revendication 3, **caractérisé en ce que** R3 représente la chaîne latérale de la tyrosine.

8. Composé selon la revendication 1, **caractérisé en ce que** R₁ est choisi parmi : -H et - CH₃, et, R₂ est choisi parmi CH₃, O-CH₃ et -S-CH₃.

9. Composé selon l'une quelconque des revendications 1 à 8, **caractérisé en ce que** A est

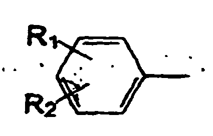

10. Composé selon la revendication 1 de formule (I), dans laquelle 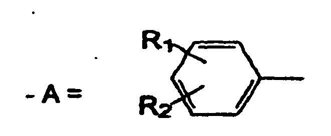 ledit composé étant choisi au sein du groupe constitué par tes composés suivants dans lesquels : 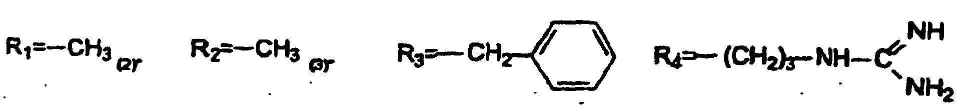 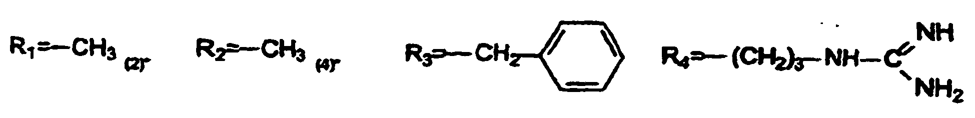 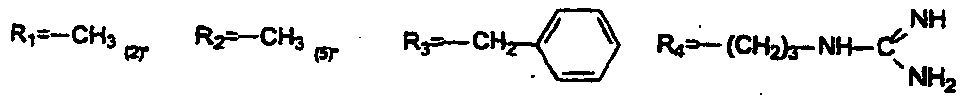 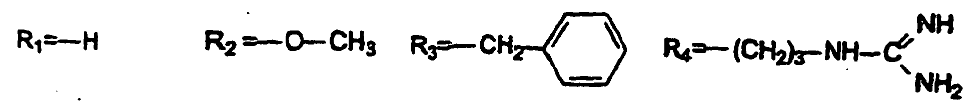 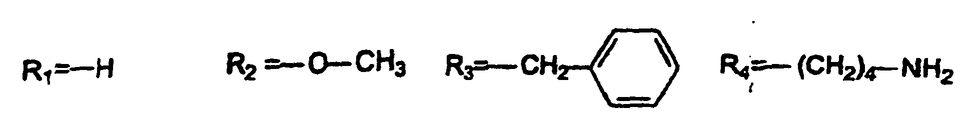 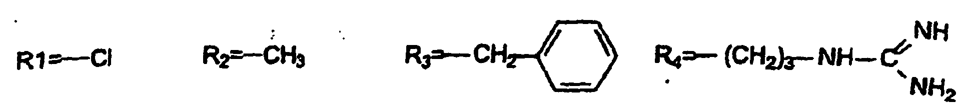 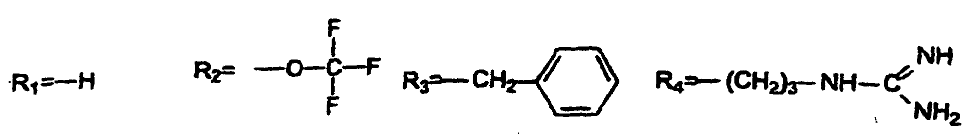 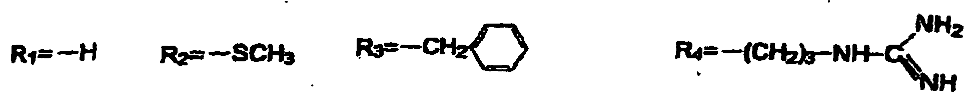 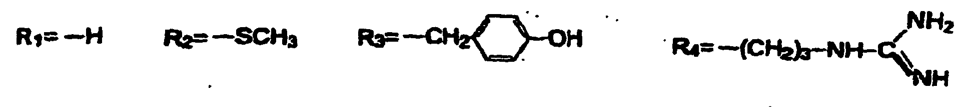 * les chiffres entre parenthèses déterminant la position des groupements methyl sur le radical phényl.

11. Composé selon la revendication 1 **caractérisé en ce qu'**il s'agit du 4-MTPAFYR (4-methylthiophénylazoformyltyrosine arginine).

12. Méthode de dosage de l'activité d'une carboxypeptidase U dans un échantillon biologique, dans laquelle :
- on met en contact ledit échantillon avec un composé de formule (I) selon l'une quelconque des revendications 1 à 11, et une carboxypeptidase A, dans des conditions permettant l'hydrolyse de l'échantillon et,
- on mesure la diminution de coloration de l'échantillon contenant le substrat de formule (I) et la carboxypeptidase A, résultant de la double hydrolyse du substrat de formule (I) par la CPU de l'échantillon et par la CPA.

13. Méthode selon la revendication 12, **caractérisée en ce que** R1 = H et R2=-S-CH3.

14. Méthode selon la revendication 12 ou 13, **caractérisée en ce que** R₄ est la chaîne latérale de l'arginine ou de la lysine.

15. Méthode selon l'une des revendications 12 ou 13, **caractérisée en ce que** le substrat est un composé de formule (I) dans lequel R₃ est choisi parmi les chaînes latérales des acides animés suivants :
- la tyrosine,
- la phénylalanine,
- l'alanine,
- la valine,
- la leucine,
- l'isoleucine,
- la phénylglycine.

16. Méthode selon l'une des revendications 12 à 15, **caractérisée en ce que** R3 est la chaîne laterale de la tyrosine.

17. Méthode selon la revendication 12 à 15, **caractérisée en ce que** le substrat est un composé de formule (I) dans lequel R₃ représente la chaîne latérale de la phénylalanine.

18. Méthode selon les revendications 12 à 15, **caractérisée en ce que** le substrat est un composé de formule (I) dans lequel R₃ représente la chaîne latérale de la phénylalanine et R₄ celle de l'arginine ou de la lysine.

19. Méthode selon l'une quelconque des revendications 12, à 18, **caractérisée en ce que** le substrat est un composé de formule (I) dans lequel R₁ est choisi parmi -H et -CH₃, et, R₂ est choisi parmi CH₃, O-CH₃ et -S-CH₃.

20. Méthode selon la revendication 12, **caractérisée en ce que** le substrat est un composé de formule (1) dans lequel: 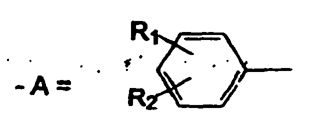 dans lequel
- R1, R2=H, -CH₃ -CH(CH₃)₂, -OCH₃, -Cl, -CF₃, -OCF₃, -SCH₃
- R₃ = la chaîne latérale d'un acide aminé hydrolysable par une carboxypeptidase A.
- R₄ = la chaîne latérale d'un acide aminé basique.

21. Méthode selon la revendication 12, **caractérisée en ce que** le substrat est un composé de formule (I) dans laquelle : 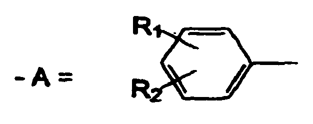 ledit composé étant choisi au sein du groupe constitué par les composés suivants, dans lesquels : 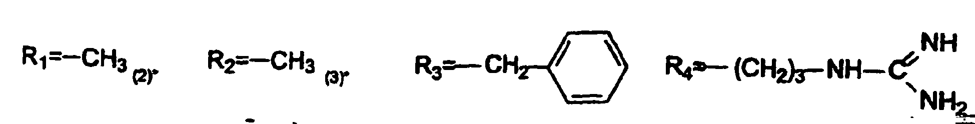 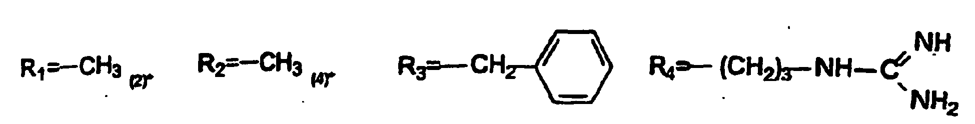 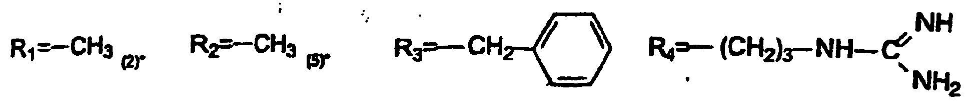 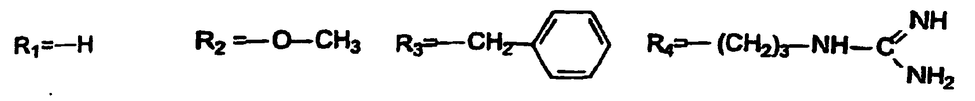 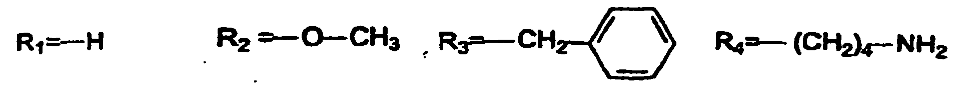 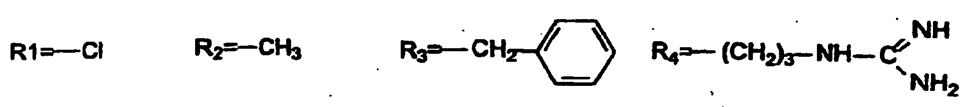 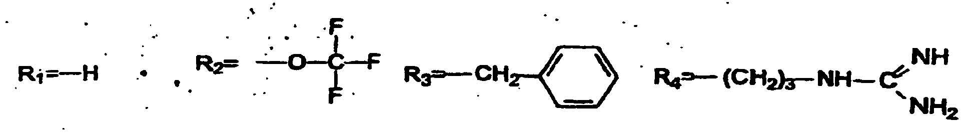 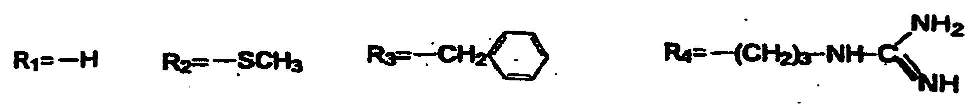 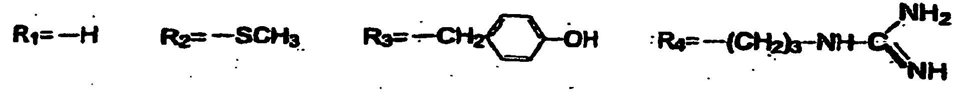 * les chiffres entre parenthèses déterminant la position des groupements methyl sur le radical phényl.

22. Méthode selon l'une quelconque des revendications 12, 13, 15, 20 ou 21, dans laquelle le composé de formule (I) est le 4-MTPAFYR (4-méthylthiophénylazoformyltyrosinearginine).

23. Méthode selon l'une quelconque des revendications 12 à 22, **caractérisée en ce que** la densité optique du mélange est mesurée sans addition de CPA, puis après addition de CPA.

24. Méthode selon l'une quelconque des revendications 12 à 23, **caractérisée en ce que** la diminution de coloration mesurée est comparée avec des valeurs portées sur une courbe d'étalonnage.

25. Méthode selon l'une quelconque des revendications 12 à 24. **caractérisée en ce que** l'échantillon est un échantillon sanguin.

26. Méthode selon ta revendication 25, **caractérisée en ce que** l'échantillon est du plasma

27. Méthode selon l'une quelconque des revendications 12 à 26, **caractérisée en ce que** la CPA est de la CPA pancréatique.

28. Méthode selon l'une quelconque des revendications 12 à 27, **caractérisée en ce que** l'échantillon à tester est mis en présence d'un tampon activateur le temps nécessaire pour obtenir l'activation de la carboxypeptidase U dont on veut mesurer l'activité, puis en présence d'un inhibiteur des serine protéases.

29. Méthode selon la revendication 28, **caractérisée en ce que** le substrat de formule (I) est ajouté en même temps que le tampon activateur, ou simultanément ou immédiatement après l'inhibiteur des sérine protéases.

30. Méthode selon la revendication 28, **caractérisée en ce que** l'activation est réalisée par la voie du complexe thrombine/thrombomoduline.

31. Méthode pour doser l'activité de la CPU constitutionnelle d'un échantillon et celle de la CPU activable du même échantillon,
**caractérisée en ce qu'**on compare l'activité d'hydrolyse de l'échantillon sur un substrat de formule (I) après mise en présence de l'échantillon avec un tampon activateur, le cas échéant le temps nécessaire pour obtenir l'activation de la carboxypeptidase U dont on veut mesurer l'activité, puis en présence d'un inhibiteur des serine protéases, l'activité d'hydrolyse observée étant comparée avec l'activité d'hydrolyse de l'échantillon sur un substrat de formule (I) en l'absence de tampon activateur selon la revendication 21.

32. Méthode selon l'une quelconque des revendications 12 à 28, **caractérisée en ce que** ta carboxypeptidase est le TAFI.

33. Méthode selon l'une quelconque des revendications 28 à 32, **caractérisée en ce que** l'échantillon est traité en présence et en absence d'un inhibiteur spécifique du TAFI.

34. Méthode selon l'une quelconque des revendications 28 à 33, **caractérisée en ce que** l'inhibiteur spécifique du TAFI est le CPI.

35. Méthode de dosage du TAFI activé dans un échantillon sanguin, comprenant les étapes suivantes
a) mise en présence d'un aliquote n° 1 de l'échantillon avec un inhibiteur spécifique du TAFI, et traitement de celui-ci selon la méthode de la revendication 28,
b) traitement d'un aliquote n° 2 de l'échantillon selon la méthode de la revendication 28, en l'absence d'inhibiteur spécifique du TAFI.
c) mesure du Δ DO entre l'aliquote n° 1 et l'aliquote n° 2, représentatif de l'activité du TAFI activé dans l'échantillon.

36. Méthode selon la revendication 35 pour différencier l'activité du TAFI constitutionnel et celle du TAFI activable du même échantillon, **caractérisée en ce que** l'on mesure l'activité d'hydrolyse d'un troisième aliquote de l'échantillon sur un substrat de formule (I) en l'absence de tampon activateur.

37. Utilisation d'un composé de formule (I) selon rune quelconque des revendications 1 à 11 pour doser l'activité enzymatique d'une carboxypeptidase U dans un échantillon.

38. Utilisation selon la revendication 37 **caractérisée en ce que** la carboxypeptidase est le TAFI.

39. Kit pour doser l'activité d'une CPU dans un échantillon comprenant un substrat chromogène constitué par un composé selon l'une quelconque des revendications 1 à 11.

40. Kit pour doser l'activité du TAFI dans un échantillon biologique, comprenant
- un tampon activateur du TAFI,
- de ta carboxypeptidase A,
- un substrat de formule (1), selon l'une quelconque des revendications 1 à 11,
- un inhibiteur du TAFI.

## Claims

1. A compound with the following formula (I): 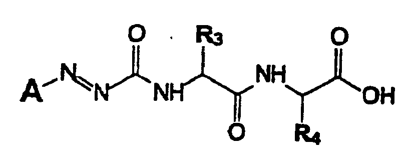 in which: 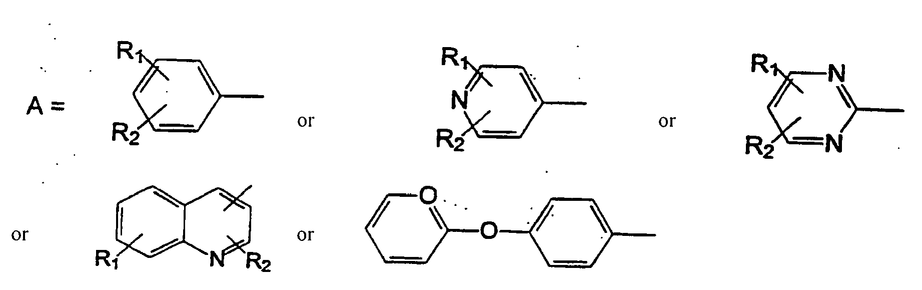
• R₁, R₂ = H, -CH₃, -CH(CH₃)₂, -OCH₃, -Cl, -CF₃, -OCF₃, -SCH₃;
• R₃ = the lateral chain of an amino acid hydrolysable by a carboxypeptidase A;
• R₄ = the lateral chain of a basic amino acid.

2. A compound according to claim 1 with the following formula (I): 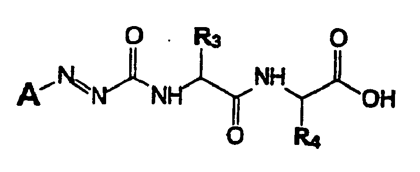 in which: 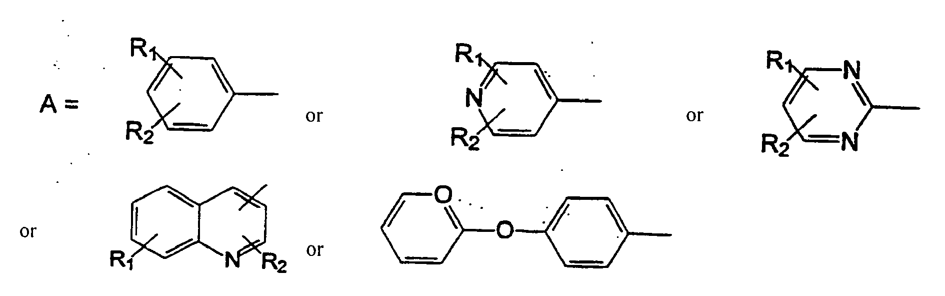
• R₁, R₂ = H, -CH₃, -CH(CH₃)₂, -OCH₃, -Cl, -CF₃, -OCF₃, -SCH₃;
• R₃ = the lateral chain of a hydrophobic amino acid;
• R₄ = the lateral chain of an arginine or of a lysine.

3. A compound according to claim 1 or claim 2, **characterized in that** R₁ = H and R₂ = -S-CH₃.

4. A compound according to claim 1 or claim 3, **characterized in that** R₃ is selected from the the lateral chain of the following amino acids:
• tyrosine:
• phenylalanine;
• alanine;
• valine;
• leucine;
• isoleucine;
• phenylglycine.

5. A compound according to claim 1 or claim 3, **characterized in that** R₃ represents the lateral chain of the phenylalanine.

6. A compound according to claim 1 or claim 3, **characterized in that** R₃ represents the lateral chain of the phenylalanine or of the tyrosine and R₄ represents the lateral chain of the arginine or the lateral chain of the lysine.

7. A compound according to claim 1 or claim 3, **characterized in that** R₃ represents the lateral chain of the tyrosine.

8. A compound according to claim 1, **characterized in that** R₁ is selected from: -H and -CH₃, and R₂ is selected from CH₃, O-CH₃ and -S-CH₃.

9. A compound according to any one of claims 1 to 8, **characterized in that** A is:

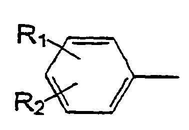

10. A compound according to claim 1 with formula (I), in which: 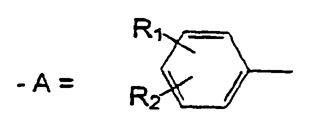 said compound being selected from the group constituted by the following compounds in which: 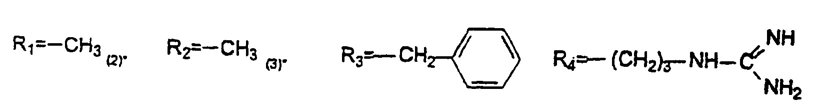 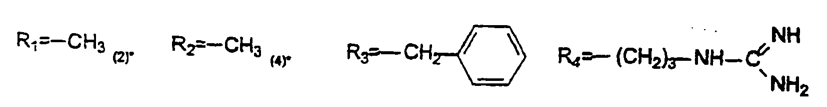 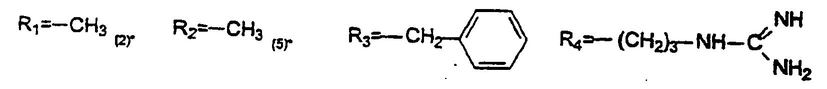 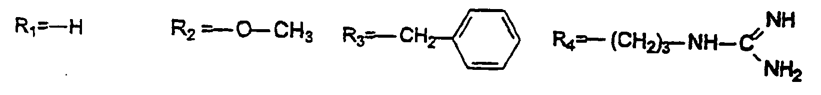 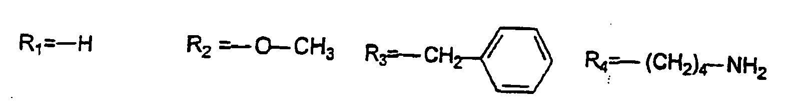 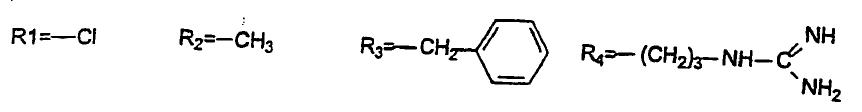 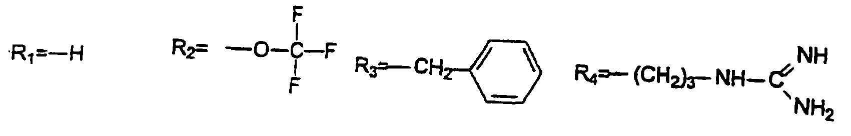 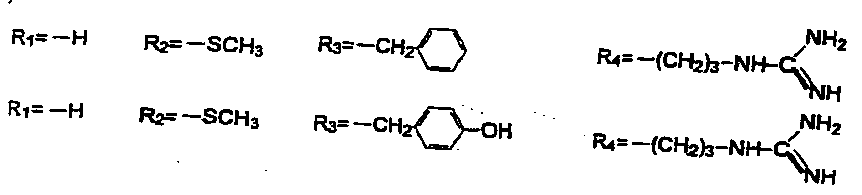 *the numbers in brackets determining the position of the methyl groups on the phenyl radical.

11. A compound according to claim 1, **characterized in that** it is 4-MTPAFYR (4-methylthiophenylazoformyltyrosine arginine).

12. A method for assaying the activity of a carboxypeptidase U in a biological sample, in which:
• said sample is brought into contact with a compound with formula (I) according to any one of claims 1 to 11, and with a carboxypeptidase A, under conditions that allow hydrolysis of the sample; and
• the reduction in coloration of the sample containing the substrate with formula (I) and carboxypeptidase A is measured, resulting from double hydrolysis of the substrate with formula (I) by the CPU of the sample and by CPA.

13. A method according to claim 12, **characterized in that** R₁ = H and R₂ = -S-CH₃.

14. A method according to claim 12 or claim 13, **characterized in that** R₄ is the lateral chain of an arginine or of a lysine.

15. A method according to claim 12 or claim 13, **characterized in that** the substrate is a compound with formula (I) in which R₃ is selected from the lateral chain of the following amino acids:
• tyrosine:
• phenylalanine;
• alanine;
• valine;
• leucine;
• isoleucine;
• phenylglycine.

16. A method according to one of claims 12 to 15, **characterized in that** R₃ is the lateral chain of the tyrosine.

17. A method according to claims 12 to 15, **characterized in that** the substrate is a compound with formula (I), in which R₃ represents the lateral chain of the phenylalanine.

18. A method according to claims 12 to 15, **characterized in that** the substrate is a compound with formula (I) in which R₃ represents phenylalanine and R₄ represents the lateral chain of an arginine or of a lysine.

19. A method according to any one of claims 12 to 18, **characterized in that** the substrate is a compound with formula (I) in which R₁ is selected from -H and -CH₃, and R₂ is selected from CH₃, O-CH₃ and -S-CH₃.

20. A method according to claim 12, **characterized in that** the substrate is a compound with formula (I) in which: 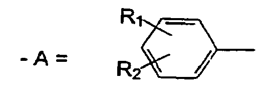 in which:
• R₁, R₂ = H, -CH₃, -CH(CH₃)₂, -OCH₃, -Cl, -CF₃, -OCF₃, -SCH₃;
• R₃ = the lateral chain of an amino acid hydrolysable by a carboxypeptidase A;
• R₄ = the lateral chain of a basic amino acid.

21. A method according to claim 12, **characterized in that** the substrate is a compound with formula (I) in which: 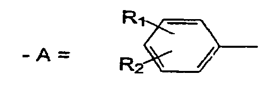 said compound being selected from the group constituted by the following compounds: 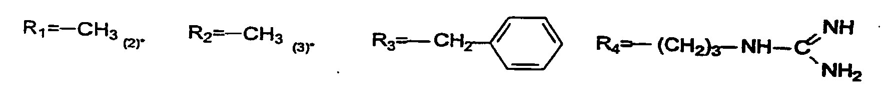 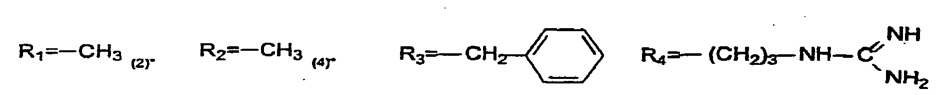 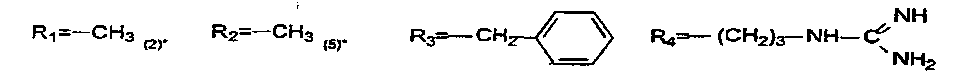 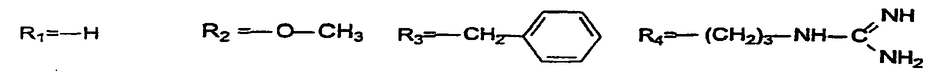 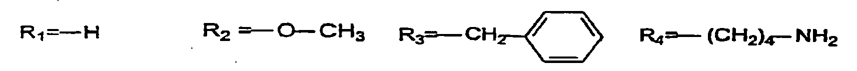 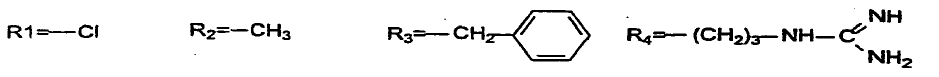 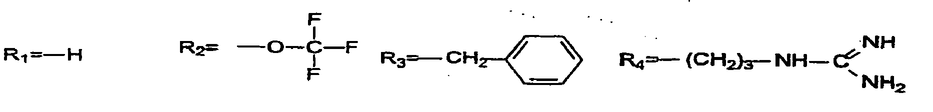 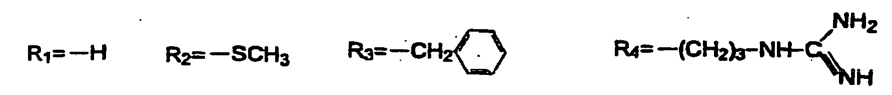 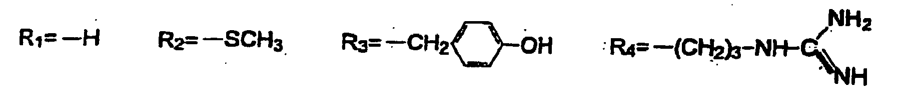 *the numbers in brackets determining the position of the methyl groups on the phenyl radical.

22. A method according to any one of claims 12, 13, 15, 20 or 21, in which the compound with formula (I) is 4-MTPAFYR (4-methylthiophenylazoformyltyrosine arginine).

23. A method according to any one of claims 12 to 22, **characterized in that** the optical density of the mixture is measured without adding CPA, then after adding CPA.

24. A method according to any one of claims 12 to 23, **characterized in that** the measured decrease in coloration is compared with values on a calibration curve.

25. A method according to any one of claims 12 to 24, **characterized in that** the sample is a blood sample.

26. A method according to claim 25, **characterized in that** the sample is plasma.

27. A method according to any one of claims 12 to 26, **characterized in that** the CPA is pancreatic CPA.

28. A method according to any one of claims 12 to 27, **characterized in that** the test sample is brought into the presence of an activator buffer for the time necessary to obtain activation of the carboxypeptidase U the activity of which is to be measured, then into the presence of a protease serine inhibitor.

29. A method according to claim 28, **characterized in that** the substrate with formula (I) is added at the same time as the activator buffer, or simultaneously or immediately after the serine protease inhibitor.

30. A method according to claim 28, **characterized in that** activation is carried out using the thrombin/thrombomodulin complex route.

31. A method for assaying the activity of the constitutional CPU of a sample and that of the activatable CPU of the same sample, **characterized in that** the hydrolysis activity of the sample on a sample with formula (I) is compared after bringing the sample into the presence of an activator buffer, if necessary for the time necessary to obtain activation of the carboxypeptidase U the activity of which is to be measured, then into the presence of a protease serine inhibitor, the observed hydrolysis activity being compared with the hydrolysis activity of the sample on a substrate with formula (I) in the absence of an activator buffer in accordance with claim 21.

32. A method according to any one of claims 12 to 28, **characterized in that** the carboxypeptidase is TAFI.

33. A method according to any one of claims 28 to 32, **characterized in that** the sample is treated in the presence and in the absence of a specific TAFI inhibitor.

34. A method according to any one of claims 28 to 33, **characterized in that** the specific TAFI inhibitor is CPI.

35. A method for assaying activated TAFI in a blood sample, comprising the following steps:
a) bringing a first aliquot of the sample into contact with a specific TAFI inhibitor and treating it using the method defined in claim 28;
b) treating a second aliquot of the sample using the method of claim 28, in the absence of specific TAFI inhibitor;
c) measuring the Δ OD between the first and second aliquot, representative of the activity of the activated TAFI in the sample.

36. A method according to claim 35 for differentiating between the activity of constitutional TAFI and that of activatable TAFI in the same sample, **characterized in that** the hydrolysis activity of a third aliquot of the sample is measured on a substrate with formula (I) in the absence of a buffer activator.

37. Use of a compound with formula (I) according to any one of claims 1 to 11, to assay the enzymatic activity of a carboxypeptidase U in a sample.

38. Use according to claim 37, **characterized in that** the carboxypeptidase is TAFI.

39. A kit for assaying the activity of a CPU in a sample comprising a chromogenic substrate constituted by a compound according to any one of claims 1 to 11.

40. A kit for assaying the activity of TAFI in a biological sample, comprising:
• a TAFI activator buffer;
• carboxypeptidase A;
• a substrate with formula (I) according to any one of claims 1 to 11;
• a TAFI inhibitor.

## Patentansprüche

1. Verbindung der folgenden Formel (I): 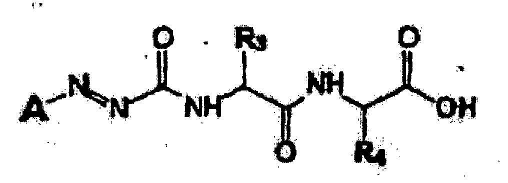 in welcher: 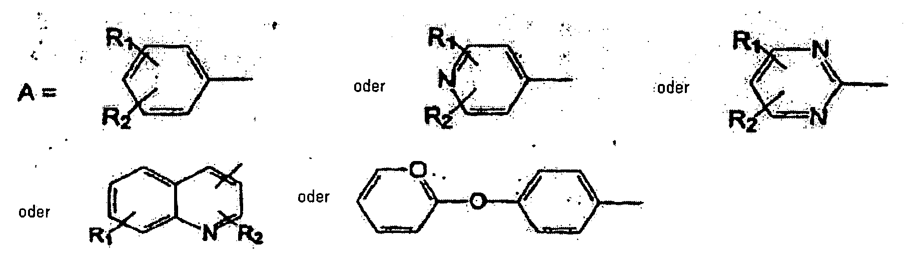
- R₁, R₂ = H, -CH₃, -CH(CH₃)₂, -OCH₃, -Cl, -CF₃, -OCF₃, -SCH₃
- R₃ = die Seitenkette einer durch eine Carboxypeptidase A hydrolysierbaren Aminosäure.
- R₄ = die Seitenkette einer basischen Aminosäure.

2. Verbindung gemäß Anspruch 1 der folgenden Formel (I): 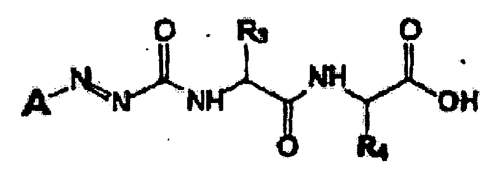 in welcher: 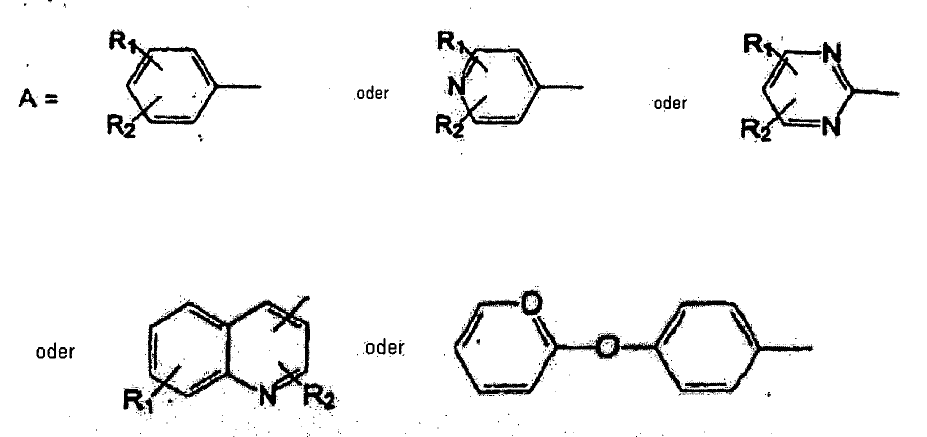
- R1, R2 = H, -CH₃, -CH(CH₃)₂, -OCH₃, -Cl, -CF₃, -OCF₃, -SCH₃
- R₃ = die Seitenkette einer hydrophoben Aminosäure.
- R₄ = die Seitenkette von Arginin oder Lysin.

3. Verbindung gemäß Anspruch 1 oder 2, **dadurch gekennzeichnet, dass** R1 = H und R2 = -S- CH₃.

4. Verbindung gemäß Anspruch 1 oder 3, **dadurch gekennzeichnet, dass** R₃ aus den Seitenketten der folgenden Aminosäuren gewählt ist:
- Tyrosin,
- Phenylalanin,
- Alanin,
- Valin,
- Leucin,
- Isoleucin,
- Phenylglycin.

5. Verbindung gemäß Anspruch 1 oder Anspruch 3, **dadurch gekennzeichnet, dass** R₃ die Seitenkette von Phenylalanin darstellt.

6. Verbindung gemäß Anspruch 1 oder Anspruch 3, **dadurch gekennzeichnet, dass** R₃ die Seitenkette von Phenylalanin oder von Tyrosin und R₄ die von Arginin oder von Lysin darstellt.

7. Verbindung gemäß Anspruch 1 oder Anspruch 3, **dadurch gekennzeichnet, dass** R3 die Seitenkette von Tyrosin darstellt.

8. Verbindung gemäß Anspruch 1, **dadurch gekennzeichnet, dass** R₁ gewählt ist aus: -H und -CH₃ und R₂ gewählt ist aus CH₃, O-CH₃ und -S-CH₃.

9. Verbindung gemäß einem der Ansprüche 1 bis 8, **dadurch gekennzeichnet, dass** A ist:

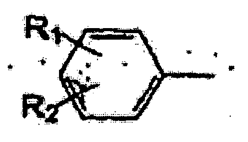

10. Verbindung gemäß Anspruch 1 der Formel (I), in welcher 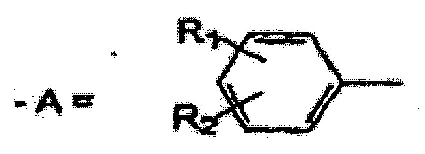 wobei die Verbindung aus der Gruppe gewählt ist, die aus den folgenden Verbindungen gebildet ist: 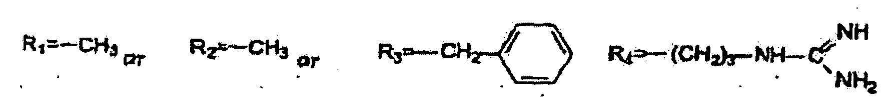 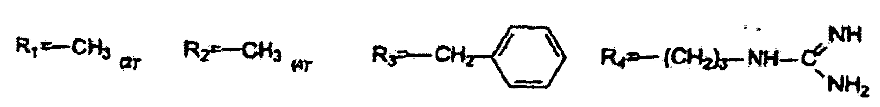 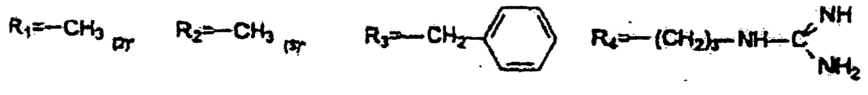 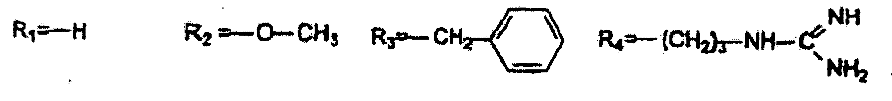 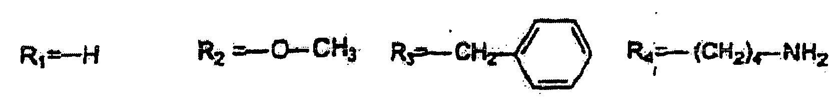 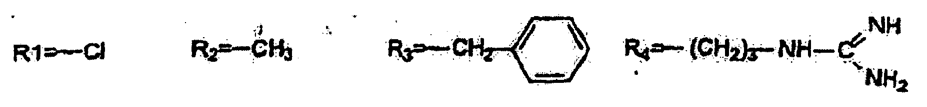 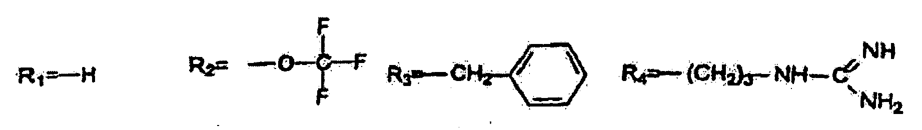 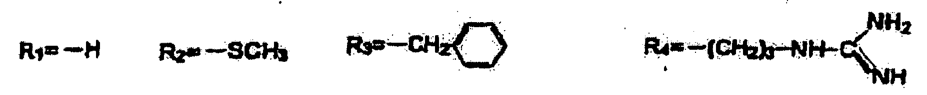 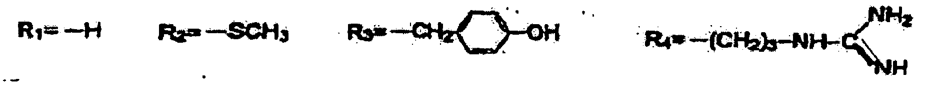 * wobei die Zahlen in Klammern die Position der Methylgruppen an dem Phenylradikal bestimmen.

11. Verbindung gemäß Anspruch 1, **dadurch gekennzeichnet, dass** es sich um 4-MTPAFYR (4-Methylthiophenylazoformyltyrosinarginin) handelt.

12. Verfahren zur Dosierung der Aktivität einer Carboxypeptidase U in einer biologischen Probe, bei welchem:
- die Probe mit einer Verbindung der Formel (I) gemäß einem der Ansprüche 1 bis 11 und einer Carboxypeptidase A unter Bedingungen, die die Hydrolyse der Probe ermöglichen, in Kontakt gebracht werden, und
- die Minderung der Färbung der Probe, welche das Substrat der Formel (I) und die Carboxypeptidase A enthält, gemessen wird, die sich aus der zweifachen Hydrolyse des Substrats der Formel (I) durch CPU der Probe und durch CPA ergibt.

13. Verfahren gemäß Anspruch 12, **dadurch gekennzeichnet, dass** R1 = H und R2 = -S-CH₃.

14. Verfahren gemäß Anspruch 12 oder 13, **dadurch gekennzeichnet, dass** R₄ die Seitenkette von Arginin oder von Lysin ist.

15. Verfahren gemäß Anspruch 12 oder 13, **dadurch gekennzeichnet, dass** das Substrat eine Verbindung der Formel (I) ist, in welcher R₃ aus den Seitenketten der folgenden Aminosäuren gewählt ist:
- Tyrosin,
- Phenylalanin,
- Alanin,
- Valin,
- Leucin,
- Isoleucin,
- Phenylglycin.

16. Verfahren gemäß einem der Ansprüche 12 bis 15, **dadurch gekennzeichnet, dass** R3 die Seitenkette von Tyrosin ist.

17. Verfahren gemäß einem der Ansprüche 12 bis 15, **dadurch gekennzeichnet, dass** das Substrat eine Verbindung der Formel (I) ist, in welcher R₃ die Seitenkette von Phenylalanin darstellt.

18. Verfahren gemäß einem der Ansprüche 12 bis 15, **dadurch gekennzeichnet, dass** das Substrat eine Verbindung der Formel (I) ist, in welcher R₃ die Seitenkette von Phenylalanin und R₄ die von Arginin oder von Lysin darstellt.

19. Verfahren gemäß einem der Ansprüche 12 bis 18, **dadurch gekennzeichnet, dass** das Substrat eine Verbindung der Formel (I) ist, in welcher R₁ aus -H und -CH₃ gewählt ist und R₂ gewählt ist aus CH₃, O-CH₃ und -S-CH₃.

20. Verfahren gemäß Anspruch 12, **dadurch gekennzeichnet, dass** das Substrat eine Verbindung der Formel (I) ist, in welcher 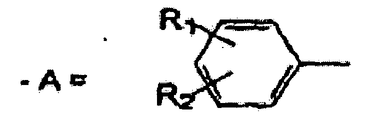 in welcher
R1, R2 = H, -CH₃, -CH(CH₃)₂, -OCH₃, -Cl, -CF₃, -OCF₃, -SCH₃
R₃ = die Seitenkette einer durch eine Carboxypeptidase A hydrolysierbaren Aminosäure.
R₄ = die Seitenkette einer basischen Aminosäure.

21. Verfahren gemäß Anspruch 12, **dadurch gekennzeichnet, dass** das Substrat eine Verbindung der Formel (I) ist, in welcher 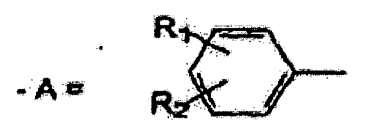 wobei die Verbindung aus der Gruppe gewählt ist, die aus den folgenden Verbindungen gebildet ist: 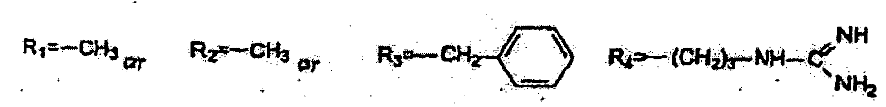 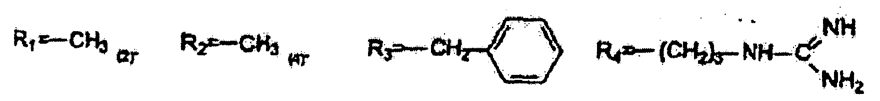 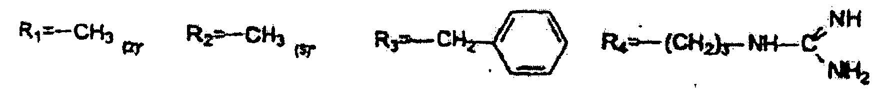 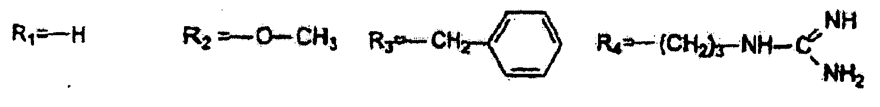 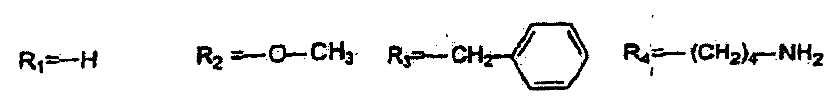 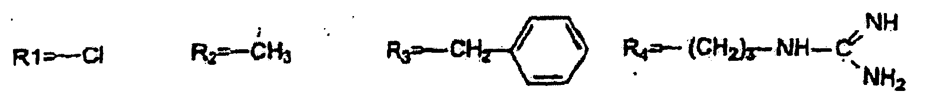 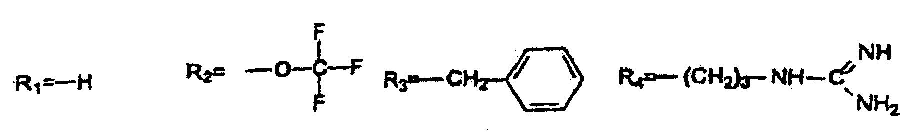 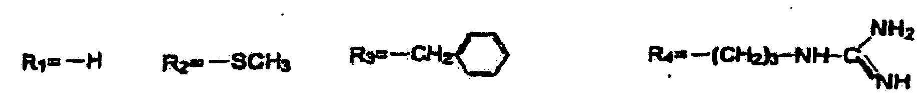 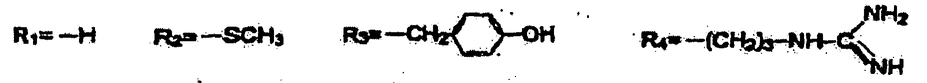 * wobei die Zahlen in Klammern die Position der Methylgruppen an dem Phenylradikal bestimmen.

22. Verfahren gemäß einem der Ansprüche 12, 13, 15, 20 oder 21, **dadurch gekennzeichnet, dass** die Verbindung der Formel (I) 4-MTPAFYR (4-Methylthiophenylazoformyltyrosinarginin) ist.

23. Verfahren gemäß einem der Ansprüche 12 bis 22, **dadurch gekennzeichnet, dass** die optische Dichte der Mischung ohne Zusatz von CPA gemessen wird, dann nach Zusatz von CPA

24. Verfahren gemäß einem der Ansprüche 12 bis 23, **dadurch gekennzeichnet, dass** die gemessene Färbungsminderung mit den Wertespannen auf einer Eichkurve verglichen wird.

25. Verfahren gemäß einem der Ansprüche 12 bis 24, **dadurch gekennzeichnet, dass** die Probe eine Blutprobe ist.

26. Verfahren gemäß Anspruch 25, **dadurch gekennzeichnet, dass** die Probe Plasma ist.

27. Verfahren gemäß einem der Ansprüche 12 bis 26, **dadurch gekennzeichnet, dass** das CPA Bauchspeicheldrüsen-CPA ist.

28. Verfahren gemäß einem der Ansprüche 12 bis 27, **dadurch gekennzeichnet, dass** die zu testende Probe für die Zeit, die zum Erhalten der Aktivierung der Carboxypeptidase U, deren Aktivität gemessen werden soll, erforderlich ist, in Gegenwart eines Aktivatorpuffers, dann in Gegenwart eines Inhibitors für Serin-Proteasen gebracht wird.

29. Verfahren gemäß Anspruch 28, **dadurch gekennzeichnet, dass** das Substrat der Formel (I) zur gleichen Zeit hinzugefügt wird wie der Aktivatorpuffer oder gleichzeitig mit oder unmittelbar nach dem Inhibitor für Serin-Proteasen.

30. Verfahren gemäß Anspruch 28, **dadurch gekennzeichnet, dass** die Aktivierung auf dem Weg des Thrombin-/ Thrombomodulinkomplexes durchgeführt wird.

31. Verfahren zum Dosieren der Aktivität von CPU, welche die Probe bildet, und jener von aktivierbarer CPU der gleichen Probe, **dadurch gekennzeichnet, dass** die Hydrolyseaktivität der Probe auf einem Substrat der Formel (I) nach In-Gegenwart-Bringen der Probe mit einem Aktivatorpuffer verglichen wird, gegebenenfalls für die Zeit, die erforderlich ist, um die Aktivierung der Carboxypeptidase U, deren Aktivität gemessen werden soll, zu erhalten, dann in Gegenwart eines Inhibitors für Serin-Proteasen, wobei die beobachtete Hydrolyseaktivität mit der Hydrolyseaktivität der Probe auf einem Substrat der Formel (I) bei Fehlen eines Aktivatorpuffers gemäß Anspruch 21 verglichen wird.

32. Verfahren gemäß einem der Ansprüche 12 bis 28, **dadurch gekennzeichnet, dass** die Carboxypeptidase TAFI ist.

33. Verfahren gemäß einem der Ansprüche 28 bis 32, **dadurch gekennzeichnet, dass** die Probe in Gegenwart und bei Fehlen eines für TAFI spezifischen Inhibitors behandelt wird.

34. Verfahren gemäß einem der Ansprüche 28 bis 33, **dadurch gekennzeichnet, dass** der für TAFI spezifische Inhibitor CPI ist.

35. Verfahren zur Dosierung aktivierten TAFIs in einer Blutprobe, welches die folgenden Schritte aufweist:
a) In-Gegenwart-Bringen eines Aliquots Nr. 1 der Probe mit einem für TAFI spezifischen Inhibitor und dessen Behandlung entsprechend dem Verfahren gemäß Anspruch 28,
b) Behandeln eines Aliquots Nr. 2 der Probe entsprechend dem Verfahren gemäß Anspruch 28 bei Fehlen des für TAFI spezifischen Inhibitors,
c) Messung des Δ DO zwischen Aliquot Nr. 1 du Aliquot Nr. 2, welches für die Aktivität des aktivierten TAFIs in der Probe repräsentativ ist.

36. Verfahren gemäß Anspruch 35 zur Unterscheidung des konstitutionellen TAFIs und des aktivierbaren TAFIs der gleichen Probe, **dadurch gekennzeichnet, dass** die Hydrolyseaktivität eines dritten Aliquots der Probe auf einem Substrat der Formel (I) bei Fehlen des Aktivatorpuffers gemessen wird.

37. Verwendung der Verbindung der Formel (I) gemäß einem der Ansprüche 1 bis 11, um die enzymatische Aktivität einer Carboxypeptidase U in einer Probe zu dosieren.

38. Verwendung gemäß Anspruch 37, **dadurch gekennzeichnet, dass** die Carboxypeptidase TAFI ist.

39. Kit zum Dosieren der Aktivität einer CPU in einer Probe, welches ein Chromogensubstrat aufweist, das aus einer Verbindung gemäß einem der Ansprüche 1 bis 11 gebildet ist.

40. Kit zum Dosieren der Aktivität von TAFI in einer biologischen Probe, welches aufweist:
- einen Aktivatorpuffer für TAFI,
- Carboxypeptidase A
- ein Substrat der Formel (I) gemäß einem der Ansprüche 1 bis 11,
- einen TAFI-Inhibitor.
